(19) Europäisches Patentamt
European Patent Office
Office européen des brevets

(11) Publication number: 0 192 489 B1

(12) EUROPEAN PATENT SPECIFICATION

(45) Date of publication of patent specification: 10.04.91

(51) Int. Cl.5: C07D 239/52, C07D 239/46, C07D 239/42, C07D 251/16, C07D 251/46, C07D 401/12, C07D 405/12, A01N 47/36

(21) Application number: 86301249.8

(22) Date of filing: 21.02.86

The file contains technical information submitted after the application was filed and not included in this specification

(54) **Herbicidal sulfonamides.**

(30) Priority: 21.02.85 US 703669
23.12.85 US 810336
26.02.85 US 705913
27.11.85 US 802269

(43) Date of publication of application: 27.08.86 Bulletin 86/35

(45) Publication of the grant of the patent: 10.04.91 Bulletin 91/15

(84) Designated Contracting States: BE CH DE FR GB IT LI LU NL SE

(56) References cited:
EP-A- 0 023 141
EP-A- 0 046 677
EP-A- 0 084 020
EP-A- 0 098 569
EP-A- 0 103 537
EP-A- 0 135 332
EP-A- 0 153 601
US-A- 4 339 267
US-A- 4 496 392

(73) Proprietor: E.I. DU PONT DE NEMOURS AND COMPANY
1007 Market Street
Wilmington Delaware 19898(US)

(72) Inventor: Dumas, Donald Joseph
407 Baynard Boulevard
Wilmington Delaware 19803(US)

(74) Representative: Hildyard, Edward Martin et al
Frank B. Dehn & Co. Imperial House 15-19 Kingsway
London WC2B 6UZ(GB)

EP 0 192 489 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Background of the Invention

The following publications describe herbicidal sulfonamides of various complexity. None of them, however, suggests the particular sulfonamides of this invention. U.S. 4,310,346 discloses, in part, herbicidal sulfonamides of formula

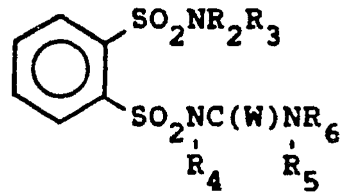

wherein:
$R_2$ is H, $C_1$ to $C_6$ alkyl, $C_3$ to $C_4$ alkenyl, $C_3$ to $C_6$ cycloalkyl, $C_4$ to $C_7$ cycloalkylalkyl. $C_5$ to $C_6$ cycloalkenyl. $c_3$ to $C_5$ alkynyl, $C_3$ to $C_6$ cycloalkyl substituted with 1 to 2 $CH_3$ groups, $CF_2CF_2H$, $CF_2CHFCl$, $CF_2CHFBr$, $CF_2CHFCF_3$, $C(CH_3)_2CN$, $(CH_2)_mCN$, where m is 1 or 2, $CH_2CH_2OCH_3$, $CH_2CH(CH_3)OCH_3$, $(CH_2)_3OCH_3$, $CHR_7CO_2R_8$ or $CHR_7CON(R_8)_2$; and
$R_3$ is $C_1$ to $C_4$ alkyl, $C_3$ to $C_4$ alkenyl, $CH_2CH_2OCH_3$, $CH_2CH(CH)_3OCH_3$, $CH_2CF_3$ or $(CH_2)_mCN$, where m is 1 or 2 or $CHR_7CO_2R_8$.

U.S. 4,478,635 discloses, in part, herbicidal sulfonamides of formula

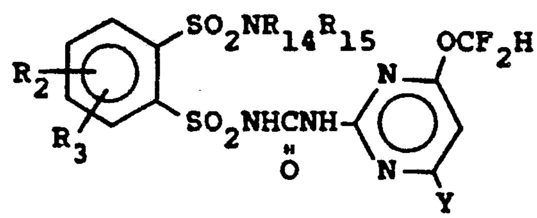

wherein:
$R_{14}$ is H, $OCH_3$, $OC_2H_5$, $C_1$ to $C_4$ alkyl or $CO_2R_{12}$;
and
$R_{15}$ is H or $C_1$ to $C_4$ alkyl.

EP-A-135,332 discloses, in part, herbicidal sulfonylureas of formula

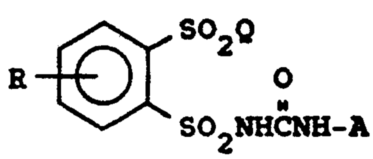

wherein:

Q is $NR_1R_2$, 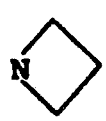 , 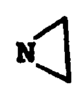 or 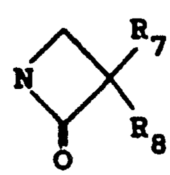 ;

$R_1$ is H, $C(O)R_3$, $C(O)NR_4R_5$, $CO_2R_6$, $C(O)NHR_9$ or $CF_2H$; and
$R_2$ is H or $C_1$ to $C_3$ alkyl.

U.S. 4,339,267 discloses, in part, herbicidal sulfonamides of formula

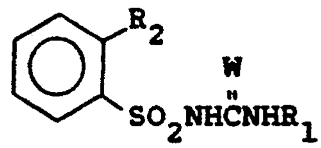

wherein:

$R_1$ is 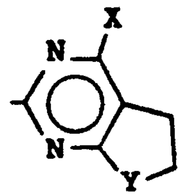 or 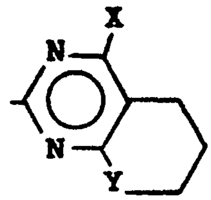 ;

$R_2$ is, among others, $SO_2NR_{10}R_{11}$ or $SO_2N(OCH_3)CH_3$;
and
$R_{10}$ and $R_{11}$ are independently $C_1$ to $C_6$ alkyl or $C_3$ to $C_4$ alkenyl or $R_{10}$ and $R_{11}$ can be taken together to be $(CH_2)_4$, $(CH_2)_5$ or $O(CH_2CH_2)_2$.

U.S. 4,496,392 discloses, in part, herbicidal sulfonamides of formula

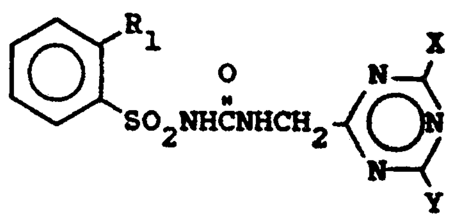

wherein:
$R_1$ is, among others, $SO_2NR_8R_9$ or $SO_2N(CH_3)OCH_3$;
$R_8$ is $CH_3$: and
$R_9$ is $C_1$ to $C_3$ alkyl.

U.S. 4,487,626 discloses, in part, herbicidal sulfonamides of formula

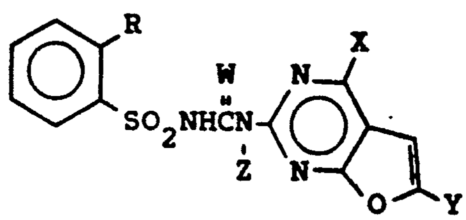

wherein:
R is, among others, $SO_2NR^6R^7$ or $SO_2N(OCH_3)CH_3$; and
$R^6$ and $R^7$ are independently $C_1$ to $C_4$ alkyl, provided that the total number of carbon atoms of $R^6$ and $R^7$ is less than or equal to 5.

Japanese Patent Application 60-166668 (laid open 8/29/85) and EP-A-153,601 disclose herbicidal sulfonamides of the formula

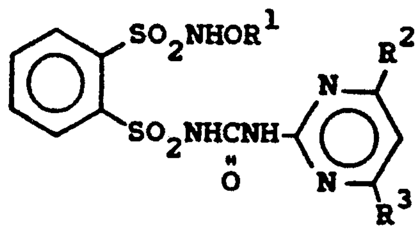

wherein:
$R^1$, $R^2$ and $R^3$ are lower alkyl.

Summary of the Invention

This invention pertains to compounds of Formula I and their agriculturally suitable salts, agriculturally suitable compositions containing them and their use as general and as selective preemergent and postemergent herbicides and plant growth regulants. The compounds of Formula I are:

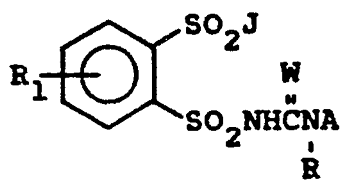

I

characterized in that:
J is $NHR_2$, $NR_3R_4$ or $NR'_2NR'_3R'_4$;
W is O or S;
R is H or $CH_3$;
$R_1$ is H, $C_1$ to $C_3$ alkyl, $C_1$ to $C_3$ haloalkyl, halogen, nitro. $C_1$ to $C_3$ alkoxy, $SO_2NR_aR_b$, $C_1$ to $C_3$ alkylthio, $C_1$ to $C_3$ alkylsulfinyl, $C_1$ to $C_3$ alkylsulfonyl, CN, $CO_2R_c$, $C_1$ to $C_3$ haloalkoxy, $C_1$ to $C_3$ haloalkylthio, $C_2$ to $C_3$ alkoxyalkyl, $C_2$ to $C_3$ haloalkoxyalkyl, $C_2$ to $C_3$ alkylthioalkyl, $C_2$ to $C_3$ haloalkylthioalkyl, $C_2$ to $C_3$ cyanoalkyl or $NR_dR_e$;
$R_a$ is H, $C_1$ to $C_4$ alkyl, $C_2$ to $C_3$ cyanoalkyl, methoxy or ethoxy:
$R_b$ is H, $C_1$ to $C_4$ alkyl or $C_3$ to $C_4$ alkenyl: or
$R_a$ and $R_b$ can be taken together as $-(CH_2)_3-$, $-(CH_2)_4-$, $-(CH_2)_5-$ or $-CH_2CH_2OCH_2CH_2-$;
$R_c$ is $C_1$ to $C_4$ alkyl, $C_3$ to $C_4$ alkenyl, $C_3$ to $C_4$ alkynyl, $C_2$ to $C_4$ haloalkyl, $C_2$ to $C_3$ cyanoalkyl, $C_5$ to $C_6$ cycloalkyl, $C_4$ to $C_7$ cycloalkylalkyl or $C_2$ to $C_4$ alkoxyalkyl:
$R_d$ and $R_e$ are independently H or $C_1$ to $C_2$ alkyl;
$R_2$ is $C_5$ to $C_6$ alkyl, $C_5$ to $C_6$ alkenyl, $C_2$ to $C_6$ haloalkenyl, $C_3$ to $C_6$ alkynyl, $C_3$ to $C_6$ cycloalkyl, $C_3$ to $C_6$ cycloalkyl optionally substituted with 1 or 2 $CH_3$ groups, $C_4$ to $C_7$ cycloalkylalkyl, $C_5$ to $C_6$ cycloalkenyl, $C_3$ to $C_6$ epoxyalkyl, $C_2$ to $C_6$ haloalkyl, $CH_2CH_2(OR_5)_2$,

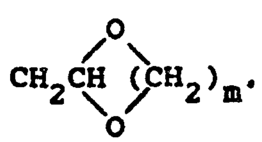

$(CH_2)_3OCH_3$, phenyl which can be optionally substituted with $R_7$,

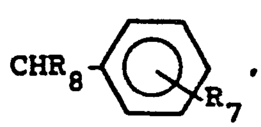

$CH_2C(O)CH_3$, CN, $OR_6$, $C_1$ to $C_6$ alkyl substituted with $OR_9$, $S(O)_nR_{10}$ or $NR_{11}R_{12}$, Q, $CHR_8Q$ or $CH_2CH_2Q$;

$R_3$ is $C_1$ to $C_6$ alkyl, $C_3$ to $C_6$ alkenyl, $C_3$ to $C_6$ alkynyl, $C_3$ to $C_6$ cycloalkyl which can be optionally substituted with 1 or 2 $CH_3$ groups, $C_4$ to $C_7$ cycloalkylalkyl. $C_5$ to $C_6$ cycloalkenyl, $C_1$ to $C_6$ haloalkyl, $C_1$ to $C_5$ alkoxy, $C_3$ to $C_6$ epoxyalkyl or $C_1$ to $C_6$ alkyl substituted with $OR_9$, $S(O)_nR_{10}$, $NR_{11}R_{12}$ or $P(O)(OR_5)_2$;

$R_4$ is $C_1$ to $C_3$ alkyl substituted with 1 to 3 atoms of F, Cl or Br, $C_3$ to $C_4$ alkynyl. $CH_2CH_2(OR_5)_2$,

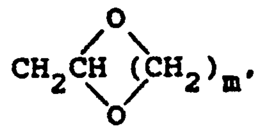

$C_2$ to $C_6$ haloalkenyl, $C_3$ to $C_6$ epoxyalkyl, $CH_2C(O)CH_3$, CN. $C_1$ to $C_6$ alkyl substituted with $OR_9$, $S(O)_nR_{10}$ or $NR_{11}R_{12}$, Q, $CHR_8Q$ or $CH_2CH_2Q$:

$R_3$ and $R_4$ can be taken together with the sulfonamide nitrogen to form a saturated 5- or 6-membered ring substituted by one or more groups selected from L or a partially saturated 5- or 6-membered ring optionally substituted by one or more groups selected from L:

$R_5$ is $C_1$ to $C_3$ alkyl;

$R_6$ is $C_1$ to $C_5$ alkyl:

$R_7$ is H, $C_1$ to $C_3$ alkyl, halogen, $NO_2$, $CF_3$, CN or $C_1$ to $C_3$ alkoxy:

$R_8$ is H or $CH_3$;

$R_9$ is H, $SO_2R_5$, $C(O)R_5$, $CO_2R_5$, $C(O)NR_{11}R_{12}$ or $P(O)(OR_5)_2$:

$R_{10}$ is $C_1$ to $C_3$ alkyl:

$R_{11}$ is H or $C_1$ to $C_3$ alkyl:

$R_{12}$ is H or $C_1$ to $C_3$ alkyl:

m is 2 or 3:

n is 0, 1 or 2:

$R'_2$, $R'_3$ and $R'_4$ are independently H, $C_1$ to $C_4$ alkyl, $C_3$ to $C_4$ alkenyl, $C_3$ to $C_4$ alkynyl, $C_1$ to $C_4$ haloalkyl, $C(O)R'_5$, $CO_2R'_6$, $C(O)NR'_7R'_8$, $C(S)NR'_7R'_8$, Q, CHRQ, $CH_2CH_2Q$, $C_2$ to $C_3$ alkyl substituted with $OR'_9$, phenyl which can be optionally substituted with $R'_{10}$ and $R'_{11}$ or

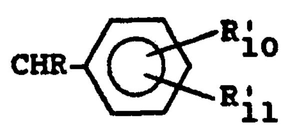 ;

$R'_3$ and $R'_4$ can be taken together to form $-(CH_2)_4-$, $-(CH_2)_5-$, $CH_2CH_2OCH_2CH_2$, $CH=CHCH=CH$, $CH=N-N=CH$

or 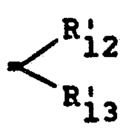 ;

$R'_5$ is $C_1$ to $C_3$ alkyl or phenyl which can be optionally substituted with $R'_{10}$ and $R'_{11}$ ;

$R'_6$ is $C_1$ to $C_3$ alkyl;

$R'_7$ and $R'_8$ are independently H or $C_1$ to $C_3$ alkyl:

$R'_9$ is H, $SO_2R'_6$, $C(O)R'_6$, $CO_2R'_6$, $C(Q)NR'_7R'_8$, $C_1$ to $C_3$ alkyl, $C_1$ to $C_3$ haloalkyl or $P(O)(OR'_6)_2$;

$R'_{10}$ and $R'_{11}$ are independently H, $C_1$ to $C_3$ alkyl, Cl, F, Br, $NO_2$ $CF_3$, CN or $C_1$ to $C_3$ alkoxy:

$R'_{12}$ and $R'_{13}$ are independently H, $C_1$ to $C_3$ alkyl, phenyl which can optionally substituted with $R'_{10}$ and $R'_{11}$ or

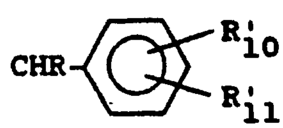 ;

$R'_{12}$ and $R'_{13}$ can be taken together to form $-(CH_2)_4-$or $-(CH_2)_5-$:

Q is a saturated 5- or 6-membered ring which is bonded through a carbon atom and contains 1 heteroatom

selected from oxygen, sulfur, or nitrogen or an unsaturated or partially unsaturated 5- or 6-membered ring which is bonded through a carbon atom and contains 1 to 3 heteroatoms selected from 1 sulfur, 1 oxygen or 1 to 3 nitrogen: and Q can be optionally substituted by one or more groups selected from L:
L is $C_1$ to $C_4$ alkyl, $C_1$ to $C_3$ haloalkyl, halogen, $C_1$ to $C_3$ alkoxy, $C_1$ to $C_3$ alkylthio, $C_3$ to $C_4$ alkenyloxy, $C_3$ to $C_4$ alkenylthio, $C_1$ to $C_2$ haloalkoxy or $C_1$ to $C_2$ haloalkylthio:

A is 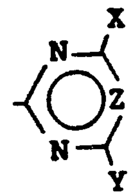

X is H, $C_1$ to $C_4$ alkyl, $C_1$ to $C_4$ alkoxy, $C_1$ to $C_4$ haloalkoxy, $C_1$ to $C_4$ haloalkyl, $C_1$ to $C_4$ haloalkylthio, $C_1$ to $C_4$ alkylthio, halogen. $C_2$ to $C_5$ alkoxyalkyl, $C_2$ to $C_5$ alkoxyalkoxy, amino, $c_1$ to $C_3$ alkylamino or di($C_1$ to $C_3$ alkyl)amino:
Y is H, $C_1$ to $C_4$ alkyl, $C_1$ to $C_4$ alkoxy, $C_1$ to $C_4$ haloalkoxy, $C_1$ to $C_4$ haloalkylthio, $C_1$ to $C_4$ alkylthio, $C_2$ to $C_5$ alkoxyalkyl, $C_2$ to $C_5$ alkoxyalkoxy, amino, $C_1$ to $C_3$ alkylamino, di($C_1$ to $C_3$ alkyl)amino, $C_3$ to $C_4$ alkenyloxy, $C_3$ to $C_4$ alkynyloxy, $C_2$ to $C_5$ alkylthioalkyl, $C_1$ to $C_4$ haloalkyl, $C_3$ to $C_5$ cycloalkyl, $C_2$ to $C_4$ alkynyl, or $N(OCH_3)CH_3$;
Z is CH or N;
characterized further in that:

a) when X is Cl, F, Br or I, then Z is CH and Y is $OCH_3$, $OC_2H_5$, $N(OCH_3)CH_3$, $NHCH_3$ or $N(CH_3)_2$;
b) the total number of carbon atoms in $R_2'$, $R_3'$ and $R_4'$ does not exceed 10;
c) $R_2$ is not $CH_2CF_3$;
d) when $R_3$ or $R_4$ is $CH_2CF_3$, then the other is $CHF_2$, $CH_2CH_2F$, $CH_2CH_2Cl$, $CH_2CH_2Br$ or $CH_2CF_3$;
e) when $R_4$ is $CF_2H$, then $R_3$ is other than $C_1$ to $C_3$ alkyl;
f) when $R_4$ is $C_3$ to $C_4$ alkynyl, then $R_3$ is $CHF_2$, $CH_2CH_2F$, $CH_2CH_2Cl$, $CH_2CH_2Br$ or $C_3$ to $C_4$ alkynyl;
g) when $R_2$ is $OR_6$ and Z is CH, then one or both of X and Y is other than $C_1$ to $C_4$ alkyl; and
h) neither of X and Y is halomethoxy.

In the above definitions, the term "alkyl", used either alone or in compound words such as "alkylthio" or "haloalkyl", means straight chain or branched alkyl such as methyl, ethyl, n -propyl, isopropyl or the different butyl, pentyl and hexyl isomers.

"Alkoxy" means methoxy, ethoxy, n -propoxy, isopropoxy and the different butoxy isomers.

"Alkenyl" means straight chain or branched alkenes such as vinyl, 1-propenyl, 2-propenyl, 3-propenyl and the different butenyl, pentyl and hexenyl isomers.

"Alkynyl" means straight chain or branched alkynes such as ethynyl, 1-propynyl, 2-propynyl and the different butynyl, pentyl and hexynyl isomers.

"Cycloalkyl" means cyclopropyl, cyclobutyl, cyclopentyl and cyclohexyl.

$C_4$ to $C_7$ cycloalkylalkyl means cyclopropylmethyl through cyclopropylbutyl or cyclohexylmethyl.

The term "halogen", either alone or in compound words such as "haloalkyl", means fluorine, chlorine, bromine or iodine. Further, when used in compound words such as "haloalkyl" said alkyl can be partially halogenated or fully substituted with halogen atoms, which can be the same or different. Examples of haloalkyl include $CH_2cH_2F$, $CF_2CF_3$ and $CH_2CHFCl$.

The total number of carbon atoms in a substituent group is indicated by the $C_i$ to $C_j$ prefix where i and j are numbers from 1 to 7. For example, $C_1$ to $C_3$ alkylsulfonyl designates methylsulfonyl through propylsulfonyl, $C_2$ alkoxyalkoxy designates $OCH_2OCH_3$; $C_4$ alkoxyalkoxy designates the various isomers of an alkoxy group substituted With a second alkoxy group containing a total of 4 carbon atoms including $OCH_2OCH_2CH_2CH_3$ and $OCH_2CH_2OCH_2CH_3$; $C_2$ cyanoalkyl designates $CH_2CN$ and $C_3$ cyanoalkyl designates $CH_2CH_2CN$ and $CH(CN)CH_3$: and so on.

Preferred Subgenera (II to XIII)

Preferred for reasons of increased ease of synthesis and/or greater herbicidal efficacy are compounds of Formula I where:
II. W is O;

J is $NHR_2$; and

$R_2$ is $C_5$ to $C_6$ alkyl, $C_5$ to $C_6$ alkenyl, $C_2$ to $C_6$ haloalkenyl, $C_3$ to $C_6$ alkynyl, $C_3$ to $C_6$ cycloalkyl, $C_3$ to $C_6$ cycloalkyl optionally substituted with 1 or 2 $CH_3$ groups, $C_4$ to $C_7$ cycloalkylalkyl, $C_5$ to $C_6$ cycloalkenyl, $C_3$ to $C_6$ epoxyalkyl, $C_2$ to $C_6$ haloalkyl, $CH_2CH_2(OR_5)_2$, $(CH_2)_3OCH_3$, $OR_6$, phenyl which can be optionally substituted with

$R_7$, 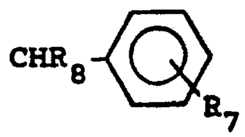 ,

$CH_2C(O)CH_3$,CN or $C_1$ to $C_6$ alkyl substituted with $OR_9$, $S(O)_nR_{10}$ or $NR_{11}R_{12}$.

Also preferred are compounds of Formula I where:

III. W is O;

J is $NHR_2$; and

$R_2$ is Q, $CHR_8Q$ or $CH_2CH_2Q$.

Also preferred are compounds of Formula I where:

IV. W is O; and

J is $NR_3R_4$.

Also preferred are compounds of Formula I where:

V. W is O;

J is $NR_2'NR_3'R_4'$;

R is H;

$R_1$ is H, $C_1$ to $C_2$ alkyl, F, Cl, Br, $NO_2$, $C_1$ to $C_2$ alkoxy, $C_1$ to $C_2$ alkylthio, $C_1$ to $C_2$ haloalkoxy, $CH_2OCH_3$, $CH_2SCH_3$ or $CH_2CN$ and is not para to the sulfonylurea bridge;

X is $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ or $CH_2Br$;

Y is H, $C_1$ to $C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C{\equiv}CH$, $CH_2OCH_2CH_3$, $OCH_2CH_2OCH_3$, $CH_2SCH_3$, $SCF_2H$, cyclopropyl, $C{\equiv}CH$ or $C{\equiv}CCH_3$,

Also preferred are compounds of subgenera II where:

VI. R is H;

$R_1$ is H, $C_1$ to $C_2$ alkyl, F, Cl, Br, $NO_2$, $C_1$ to $C_2$ alkoxy, $C_1$ to $C_2$ alkylthio, $C_1$ to $C_2$ haloalkoxy, $CH_2OCH_3$, $CH_2SCH_3$ or $CH_2CN$ and is not para to the sulfonylureas bridge;

X is $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ or $CH_2Br$; and

Y is H, $C_1$ to $C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C{\equiv}CH$, $CH_2OCH_2CH_3$, $OCH_2CH_2OCH_3$, $CH_2SCH_3$, $SCF_2H$, cyclopropyl, $C{\equiv}CH$ or $C{\equiv}CCH_3$.

Also preferred are compounds of subgenera III where:

VII. R is H;

$R_1$ is H, $C_1$ to $C_2$ alkyl, F, Cl, Br, $NO_2$, $C_1$ to $C_2$ alkoxy, $C_1$ to $C_2$ alkylthio, $C_1$ to $C_2$ haloalkoxy $CH_2OCH_3$, $CH_2SCH_3$ or $CH_2CN$ and is not para to the sulfonylurea bridge;

X is $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ or $CH_2Br$; and

Y is H, $C_1$ to $C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C{\equiv}CH$, $CH_2OCH_2CH_3$, $OCH_2CH_2OCH_3$, $CH_2SCH_3$, $SCF_2H$, cyclopropyl, $C{\equiv}CH$ or $C{\equiv}CCH_3$,

Also preferred are compounds of Subgenera IV where:

VIII. R is H;

$R_1$ is H $C_1$ to $C_2$ alkyl, F, Cl, Br, $NO_2$, $C_1$ to $C_2$ alkoxy, $C_1$ to $C_2$ alkylthio, $C_1$ to $C_2$ haloalkoxy, $CH_2OCH_3$, $CH_2SCH_3$ or $CH_2CN$ and is not para to the sulfonylurea bridge;

X is $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ or $CH_2Br$: and

Y is H, $C_1$ to $C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C{\equiv}CH$, $CH_2OCH_2CH_3$, $OCH_2CH_2OCH_3$, $CH_2SCH_3$, $SCF_2H$, cyclopropyl, $C{\equiv}CH$ or $C{\equiv}CCH_3$.

Also preferred are compounds of Subgenera V where:

IX. $R_2'$, $R_3'$ and $R_4'$ are independently H, $C_1$ to $C_2$ alkyl or phenyl provided that one of $R_2'$, $R_3'$ and $R_4'$ is H.

Also preferred are compounds of subgenera VI where:
X. $R_1$ is H, Cl, $OCH_3$, $OCF_2H$, $CH_2OCH_3$ or $CH_2CN$;
$R_2$ is $C_3$ to $C_4$ alkynyl, $C_3$ to $C_6$ cycloalkyl, $C_2$ to $C_4$ haloalkenyl, $CH_2CH_2F$, $CH_2CH_2Cl$ or $CH_2CH_2Br$;
X is $CH_3$, $OCH_3$, $OCH_2CH_3$, Cl, or $OCH_2CF_3$;
and
Y is $CH_3$, $OCH_3$, $CH_2CH_3$, $CH_2OCH_3$, or $NHCH_3$.
Also preferred are compounds of subgenera VII where:
XI. $R_1$ is H, Cl, $OCH_3$, $OCF_2H$, $CH_2OCH_3$ or $CH_2CN$;
$R_2$ is Q or $CH_2Q$;
X is $CH_3$, $OCH_3$, $CH_2CH_3$, Cl, or $OCH_2CF_3$;
and
Y is $CH_3$, $OCH_3$, $CH_2CH_3$, $CH_2OCH_3$ or $NHCH_3$,
Also preferred are compounds of Subgenera VIII where:
XII. $R_1$ is H, Cl, $OCH_3$, $OCF_2H$, $CH_2OCH_3$ or $CH_2CN$;
$R_3$ is $C_1$ to $C_3$ alkyl, $C_3$ to $C_4$ alkenyl, $C_3$ to $C_4$ alkynyl, $C_3$ to $C_6$ cycloalkyl or $C_1$ to $C_2$ haloalkyl;
$R_4$ is $C_2$ to $C_4$ haloalkenyl or $C_3$ to $C_4$ epoxyalkyl;
X is $CH_3$, $OCH_3$, $OCH_2CH_3$, Cl, or $OCH_2CF_3$;
and
Y is $CH_3$, $OCH_3$, $CH_2CH_3$, $CH_2OCH_3$, or $NHCH_3$.
Also preferred are compounds of Subgenera IX where:
XIII. $R_1$ is H, Cl, $OCH_3$, $OCF_2H$, $CH_2OCH_3$ or $CH_2CN$;
X is $Ch_3$, $OCH_3$, $OCH_2CH_3$, Cl, or $OCH_2CF_3$;
and
Y is $CH_3$, $OCH_3$, $CH_2CH_3$, $CH_2OCH_3$ or $NHCH_3$.

Specifically preferred for reasons of greatest ease of synthesis and/or greatest herbicidal efficacy are:
XIV. N-Cyclopropyl-N'-[(4,6-dimethoxypyrimidin-2-yl)aminocarbonyl]-1,2-benzenedisulfonamide, m.p. 197.5 to 199° C(d);
XV N-Cyclopropyl-N'-[(4-methoxy-6-methyl-1,3,5-triazin-2-yl)aminocarbonyl]-1,2-benzenedisulfonamide, m.p. 184.5 to 185.5° C(d):
XVI 2-(2,2-dimethylhydrazinosulfonyl)-N-[(4,6-dimethoxypyrimidin-2-yl)aminocarbonyl]-benzenesulfonamide, m.p. 127 to 128° C(d); and
XVII 2-(2,2-dimethylhydrazinsulfonyl)-N-[(4-methoxy-6-methyl-1,3,5-triazin-2-yl)aminocarbonyl]-benzenesulfonamide, m.p. 123 to 124° C(d).

## DETAILS OF THE INVENTION

### Synthesis

Compounds of Formula I can be prepared by one or more of the procedures shown in Equations 1, 4, and 5. R, $R_1$, J and A are as previously defined. Reaction conditions are given for guidance and illustration only.

**Equation 1**

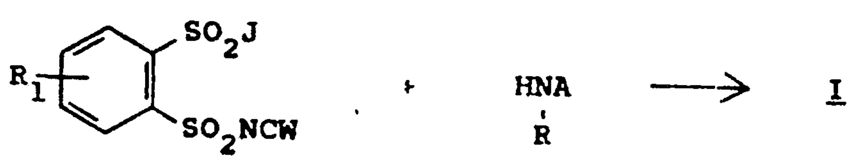

II

III

The reaction of Equation 1 is best carried out in an inert aprotic organic solvent such as dichloromethane, 1,2-dichloroethane, tetrahydrofuran, or acetonitrile, at a temperature between 20° and 85° C. The order of addition is not critical; however, it is often convenient to add the sulfonyl isocyanate, or a

solution of it in the reaction solvent, to a stirred suspension of the amine.

In some cases, the desired product is insoluble in the reaction solvent at ambient temperature and crystallizes from it in pure form. Products soluble in the reaction solvent are isolated by evaporation of the solvent. Compounds of Formula I then may be purified by trituration of the evaporation residue with solvents such as 1-chlorobutane or ethyl ether and filtration, by recrystallization from mixtures of solvents such as 1,2-dichloroethane, 1-chlorobutane and heptane or by chromatography on silica gel.

Sulfonyl isocyanates (II, W is O) are known in the art and are prepared from the corresponding sulfonamides (IV) by one of the following two general methods.

**Equation 2**

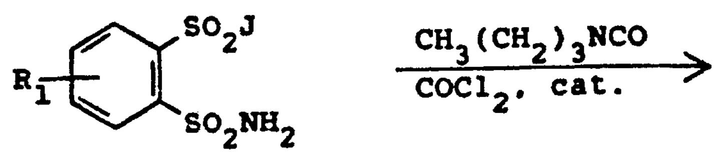


II.
W is O

IV

The sulfonamide (IV) is reacted with an alkyl isocyanate (e.g., n -butyl isocyanate) in a solvent whose boiling point is above 135 °C, such as xylene. The reaction can optimally be carried out in the presence of a catalytic amount of 1,4-diaza[2.2.2]-bicyclooctane (DABCO). The reaction mixture is heated to 135-140 °C and held at that temperature for 5-60 minutes, after which phosgene is slowly added at such a rate that the temperature remains between 133 and 135 °C. When the consumption of phosgene has ceased, the mixture is cooled and filtered to remove insoluble material. Finally, the solvent, alkyl isocyanate, and excess phosgene are evaporated, leaving the sulfonyl isocyanate (II).

If desired, the alkyl isocyanate-sulfonamide adduct can be made and isolated before reaction with the phosgene. In this case the sulfonamide (IV), alkyl isocyanate, and anhydrous base (e.g, $K_2CO_3$) in a polar, aprotic solvent (e.g. acetone, butanone, or acetonitrile) are mixed and heated under reflux for 1 to 6 hours. The reaction mixture is then diluted with water, and the pH is adjusted to about 3 with acid (e.g. HCl, $H_2SO_4$). The adduct is filtered out and dried, and then reacted with phosgene as described above. This procedure modification is especially useful when the sulfonamide (IV) is high melting and has low solubility in the phosgenation solvent.

Sulfonyl isocyanates (II, W is O) can also be prepared by the following method.

**Equation 3**

(a) IV

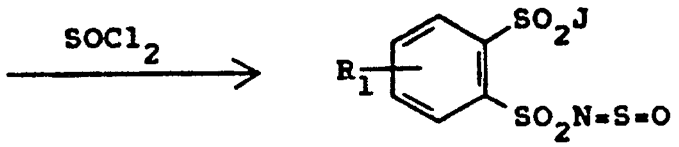


V

$COCl_2$.
pyridine cat.
(b) V → II. W is O

The sulfonamide (IV) is heated at reflux in an excess of thionyl chloride. The reaction is continued until the sulfonamide protons are no longer detectable in the proton magnetic resonance spectrum. From 16 hours to 5 days is typically sufficient for complete conversion to the thionylamide (V) (Equation 3a).

The thionyl chloride is evaporated and the residue is treated with an inert solvent (e.g. toluene) containing at least one equivalent (typically 2-3 equivalents) of phosgene. A catalytic amount of pyridine (typically 0.1 equivalent) is added, and the mixture is heated to about 60-140 °C, with 80-100 ° preferred. Conversion to the isocyanate (II. W is O) is usually substantially complete within 15 minutes to 3 hours (Equation 3b). The mixture is then cooled and filtered, and the solvent is evaporated, leaving the sulfonyl

isocyanate (II, W is O).

Sulfonyl isothiocyanates (II. W is S) are known in the art and are prepared from the corresponding sulfonamides (IV) by reaction with carbon disulfide and potassium hydroxide followed by treatment of the resulting dipotassium salt with phosgene. Such a procedure is described in Arch. Pharm. 299 , 174 (1966).

Many of the compounds of Formula I can be prepared by the procedure shown in Equation 4.

**Equation 4**

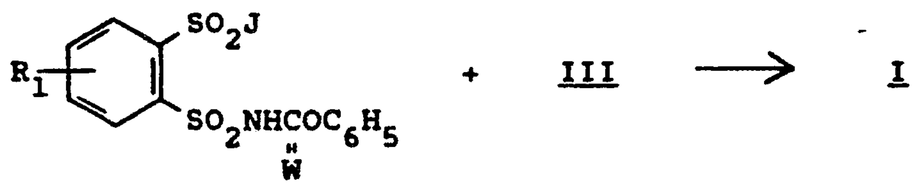
III
I

VI

The reaction of Equation 4 is carried out by contacting phenylcarbamates or phenylthiocarbamates of Formula VI with aminoheterocycles of Formula III in an inert organic solvent such as dioxane or tetrahydrofuran at temperatures of about 20-100 °C for a period of about one-half to twenty-four hours. The product can be isolated by evaporation of the reaction solvent and purified by methods previously described.

Phenylcarbamates and phenylthiocarbamates of Formula VI can be prepared by the methods described, or modifications thereof known to those skilled in the art. One such method is taught in U.S. 4,443,243.

Alternatively, many of the compounds of Formula I can be prepared by the method described in Equation 5.

**Equation 5**

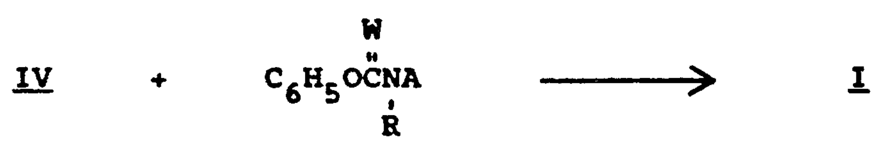
IV
I

VII

The reaction of Equation 5 can be carried out by contacting equimolar amounts of a sulfonamide of Formula IV with a heterocyclic phenylcarbamate or phenylthiocarbamate of Formula VII in the presence of a base such as 1,8-diazabicyclo[5.4.0]undec-7-ene (DBU), by methods analogous to those described in South African Patent Application 83/0441. The phenylcarbamates and phenylthiocarbamates of Formula VII can be prepared by methods, or modifications thereof known to those skilled in the art, described in South African Patent Application 82/5671 and South African Patent Application 82/5045.

Sulfonamides of formula IV can be prepared by one or both of the procedures shown in Equations 6a and 6b. Equation 6a illustrates the reaction of sulfonyl chlorides of Formula VIII with the appropriate amine or hydrazine derivative of Formula IX to give sulfonamides of Formula IV . Equation 6b illustrates the reaction of sulfonyl chlorides of Formula X with ammonia to give compounds of Formula IV wherein $R_1$ and J are as previously defined.

Equation 6

(a)

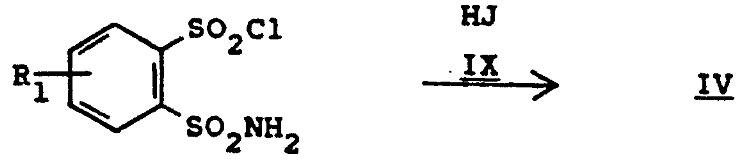


VIII

IV

(b)

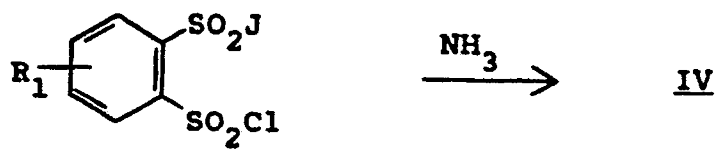


X

IV

The reactions of Equations 6a and 6b are preferably carried out in an inert, aprotic solvent such as methylene chloride, tetrahydrofuran, or acetonitrile at a temperature between -78° and 40° C. These reactions require the presence of a scavenger for the by-product hydrochloric acid. This can be accomplished by the use of a two-fold excess of the amine or the hydrazine derivative or by the addition of an equivalent of a base such as triethylamine, pyridine, or aqueous sodium hydroxide. In cases in which the products are insoluble in the reaction solvent, they may be isolated by simple filtration followed by washing with water to remove hydrochloride salts. When the products are soluble, they can be isolated by filtration, to remove any insoluble salts, followed by evaporation of the solvent and trituration of the residue with solvents such as 1-chlorobutane, diethyl ether or ethyl acetate, and filtration. In some cases it is helpful to wash the product with water to remove residual salts.

Sulfonyl chlorides of Formula VIII where $R_1$ is as previously defined can be prepared by the methods shown in Equations 7 and 8.

Diazotization of appropriately substituted aniline derivatives of Formula XI as shown in Equation 7, and subsequent coupling with sulfur dioxide in the presence of either cupric or cuprous chloride gives the desired products of Formula VIII. This reaction can be accomplished by methods described, or modifications thereof known to those skilled in the art, in the Journal of Pharmacy and Pharmacology, Vol. 12, pages 648 to 655 (1960).

Equation 7

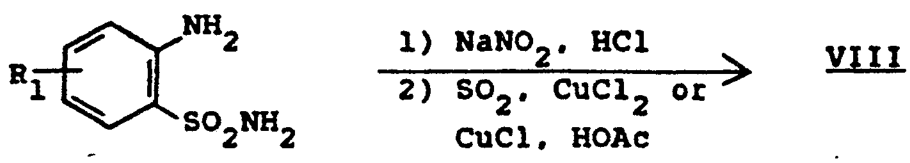


XI

VIII

The reaction of Equation 7 can be affected by analogous methods described in U.S. Patent No, 4,310,346. In Equation 7, a substituted aniline XI , wherein $R_1$ is not $NR_dR_e$ in concentrated hydrochloric acid is treated with a solution of sodium nitrite in water at -5° to 5° C. After being stirred for 10-30 minutes at about 0° C, the solution is added to a mixture of excess sulfur dioxide and a catalytic amount of cupric or cuprous chloride in acetic acid at about 10° C. After stirring for 0.25 to 20 hours at temperatures between 10° to 25° C, the solution is poured into a large excess of ice water. The sulfonyl chlorides VIII can be isolated by filtration, or by extraction into a solvent such as methylene chloride or diethyl ether, followed by drying and evaporation of the solvent.

Oxidative chlorination of appropriately substituted arylthioethers of Formula XII give the desired products wherein $R_{13}$ is $C_2$ to $C_4$ alkyl or benzyl and $R_1$ is not $C_1$ to $C_3$ alkylthio, $C_1$ to $C_3$ alkylsulfinyl, $C_1$ to $C_3$ haloalkylthio, $C_2$ to $C_3$ alkylthioalkyl or $C_2$ to $C_3$ haloalkylthioalkyl.

**Equation 8**

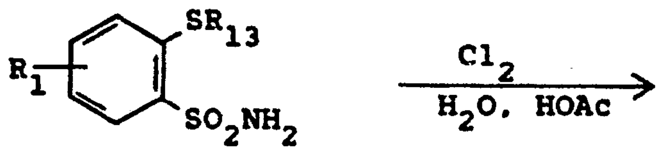


VIII

XII

The reaction of Equation 8 can be carried out by treating a solution of the thioether XII in a solvent such as acetic acid in the presence of at least 2.5 equivalents of water and at least 3.0 equivalents of chlorine at 0-30 °C for .25 to 5 hours. The reaction is poured into ice-water and the product is isolated by extraction with a suitable solvent such as methylene chloride, dried, and the solvent evaporated to yield a product sufficiently pure to be carried directly on to the next step.

Sulfonyl chlorides of Formula X in Equation 6b wherein J is $NHR_2$ or $NR_3R_4$ are known in the art and can be prepared by analogous methods described in U.S. Patent No. 4,310,346.

Sulfonyl chlorides of Formula X in Equation 6b wherein J is $NR_2'$ $NR_3'$ $R_4'$ can be prepared by the procedure shown in Equation 9 wherein $R_1'$ , $R_2'$ , $R_3'$ , and $R_4'$ are as previously defined. This procedure is best suited to cases in which both $R_3'$ and $R_4'$ are other than H.

Diazotization of appropriately substituted aniline derivatives of Formula XIII , as shown in Equation 9, and subsequent coupling with sulfur dioxide in the presence of either cupric or cuprous chloride give the desired products of Formula X .

**Equation 9**

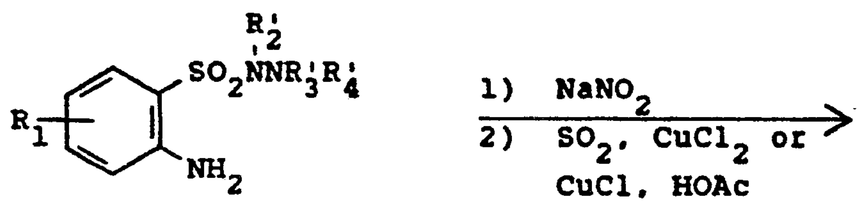


X

XIII

The reactions of Equation 9, wherein $R_1$ is not $NR_dR_e$, can be carried out under the conditions described for the reactions of Equation 7.

Aniline derivatives of Formula XI in Equation 7 can be prepared as shown in Equation 10 by reduction of the corresponding nitro compounds of Formula XIV wherein $R_1$ is other than $NO_2$.

**Equation 10**

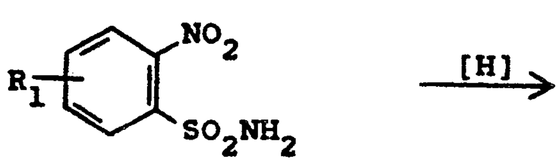


XI

XIV

The reduction reactions of Equation 10 can be accomplished by methods known in the literature by those skilled in the art. For details see, for example, U.S. 4,511,392 and references cited therein.

The nitro compounds of Formula XIV are well known in the art and can be prepared by methods such as those described in U.S. Patent No. 4,120,691.

The arylthioethers of Formula XII in Equation 8 are known in the art and can be prepared by methods

described in U.S. Patent No. 4,371,391.

The aniline derivatives of Formula XIII in Equation 9 can be prepared as shown in Equation 11 by reduction of the corresponding nitro compounds of Formula XV wherein $R_1$ is other than $NO_2$.

**Equation 11**

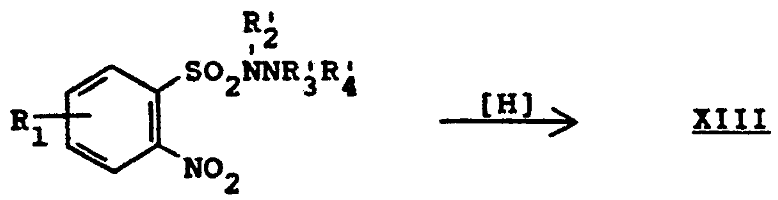

XV

The reaction of Equation 11 can be carried out under the conditions described for Equation 10.

Compounds of Formula XV can be prepared as shown in Equation 12 by the reaction of ortho - nitrobenzenesulfonyl chlorides of Formula XVI with the appropriate hydrazine derivatives of Formula IX to give ortho nitrobenzenesulfonyl hydrazides of Formula XV.

**Equation 12**

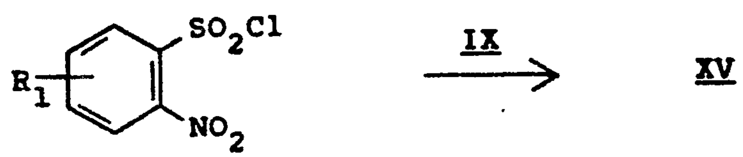

XVI

The reaction of Equation 12 can be carried out under the conditions described for Equation 6.

Sulfonyl chlorides of Formula XVI are known in the art and can be prepared by methods described in U.S. Patent No. 4,120,691.

The primary and secondary amines of Formula IX wherein J is $NHR_2$ or $NR_3R_4$ required for the reactions shown in Equation 6a are known in the art or can be prepared by one skilled in the art using methods known in the literature.

In cases where the required secondary amine is not readily available, and $R_a$ and $R_b$ are other than H, it is more convenient to prepare sulfonamides of Formula IV using the method of Equation 13.

Equation 13a illustrates the reaction of orthonitrobenzenesulfonyl chlorides of Formula XVI with primary amines to give secondary sulfonamides of Formula XVII which can then be alkylated as shown in Equation 13b to give tertiary sulfonamides of Formula XVIII. $R_1$, $R_3$, and $R_4$ are as previously defined and B is an appropriate displaceable substituent such as Cl, Br, I, $OSO_2CH_3$, $OSO_2CF_3$, $OSO_2C_6H_5$ OR $OSO_2$-p - $CH_3C_6H_4$.

**Equation 13**

(a)

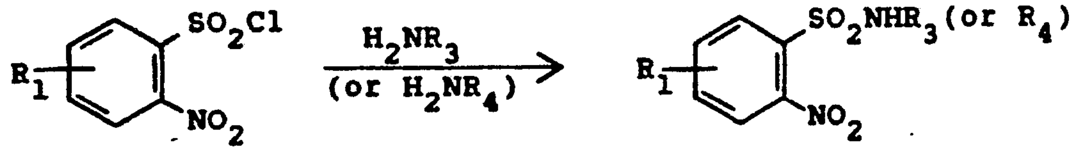


XVI XVII

(b)

XVII

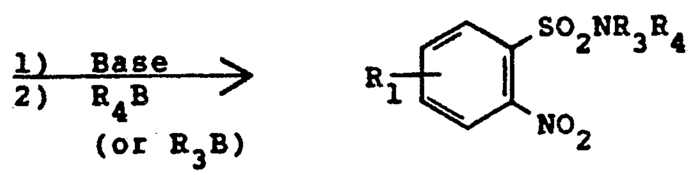


XVIII

The reactions of Equation 13a can be carried out under the conditions described for the reactions of Equation 6. The reactions of Equation 13b are best carried out by the addition of a base such as triethylamine, DBU, or potassium carbonate to a solution of the secondary sulfonamide of Formula XVII in an aprotic polar solvent such as acetonitrile or N,N-dimethylformamide at about -78 to 40° C. The alkylating agent is then added and after an additional one-half to 24 hours at about 0-100° C the reaction mixture is added to water to precipitate the product. Solid products can be isolated by simple filtration. If necessary, the products can be extracted with a solvent such as ethyl acetate or methylene chloride. The product is then isolated by drying and evaporation of the solvent and, when necessary, further purified by chromatography on silica gel.

The nitro compounds of Formula XVIII can be converted to sulfonamides of Formula IV by the methods described in Equations 7 and 10.

When $R_1$ is other than $C_1$ to $C_3$ alkylthio, $C_1$ to $C_3$ alkylsulfinyl, $C_1$ to $C_3$ haloalkylthio, $C_2$ to $C_3$ alkylthioalkyl or $C_2$ to $C_3$ haloalkylthioalkyl, sulfonamides of Formula IVa wherein $R_2$ is $C_3$ to $C_6$ epoxyalkyl and sulfonamides of Formula IVb wherein either or both $R_3$ and $R_4$ are $C_3$ to $C_6$ epoxyalkyl can be conveniently prepared by contacting the corresponding alkenyl derivatives with an oxidizing agent such as hydrogen peroxide or <u>meta</u> -chloroperbenzoic acid using procedures which are well known in the art.

The hydrazine, benzoyl hydrazine, acyl hydrazine, hydrazinocarboxylate, semicarbazide, thiosemicarbazide, and aminoguanidine derivatives of Formula IX in Equations 6a and 12 are known in the art or can be prepared by one skilled in the art using methods such as those reviewed by E. W. Schmidt in Hydrazine and its Derivatives: Preparation, Properties, Applications, J. Wiley, New York, 1984 and by Smith in Derivatives of Hydrazine and Other Hydronitrogens Having N-N Bonds , Benjamin/Cummings, Reading, Massachusetts, 1983.

The Preparation of compounds of Formula I wherein J is $NR_2' NR_3' R_4'$ with certain combinations of the substitutents $R_2'$ , $R_3'$ , and $R_4'$ may be more conveniently achieved using a sulfonylurea of Formula I or a sulfonamide of Formula IV as the intermediate. For example, sulfonylureas of Formula I in which both $R_3'$ and $R_4'$ are H may be prepared by acid catalyzed hydrolysis of compounds of Formula I in which $R_3'$ and $R_4'$ are taken together to form $=CR_{12}' R_{13}'$ . This and other transformations may be accomplished by procedures which are known in the literature by those skilled in the art.

In cases where $R_2$, $R_3$, or $R_4$ is $C_1$ to $C_6$ alkyl substituted with $OSO_2R_5$, $OC(O)R_5$, $OCO_2R_5$, $OC(O)NR_{11}R_{12}$, or $OP(O)(OR_5)_2$ or $R_2'$ , $R_3'$ or $R_4'$ is $C_2$ to $C_3$ alkyl substituted with $OSO_2R_6'$ , $C(O)R_6'$ , $OCO_2R_6'$ , $C(O)NR_7' R_8'$ or $P(O)(OR_6'$ ) it is often more convenient to first prepare the corresponding sulfonamides of Formula IV or sulfonylureas of Formula I in which $R_2$, $R_3$, or $R_4$ is $C_1$ to $C_6$ alkyl substituted with OH or $R_2'$ , $R_3'$ or $R_4'$ is $C_2$ to $C_3$ alkyl substituted with OH. The hydroxy group can then be functionalized to give the desired derivatives using methods which are well know in the art.

When $R_1$ is other than $C_1$ to $C_3$ alkylthio, $C_1$ to $C_3$ alkylsulfinyl, $C_1$ to $C_3$ haloalkylthio, $C_2$ to $C_3$ alkylthioalkyl or $C_2$ to $C_3$ haloalkylthioalkyl and $R_2$, $R_3$, or $R_4$ is $C_1$ to $C_6$ alkyl substituted with $S(O)_nR_7$ and n is equal to 1 or 2, the sulfonamides (of Formulas IVa and IVb) or sulfonylureas (of Formula I) may also be prepared from the corresponding compounds in which n is equal to 0 by contact with an oxidizing agent

such as hydrogen peroxide or meta -chloroperbenzoic acid. The level of oxidation may be controlled by the amount of oxidant used and by the reaction temperature according to procedures which are known in the art.

The heterocyclic amines of Formula III in Equation 3 above can be prepared by methods known in the literature, or simple modifications thereof, by those skilled in the art. For instance, EP-A No. 84,224 (published July 27, 1983) and W. Braker et al., J. Chem Soc. , 69 , 3072 (1947) describes methods for preparing substituted aminopyridines and triazines. Also, for example, South African patent application Nos. 82/5045 and 82/5671 describe methods for preparing aminopyrimidines and triazines substituted by haloalkyl or haloalkythio groups such as $OCH_2CH_2F$, $OCH_2CF_3$ or $SCF_2H$, among other groups. South African Patent Application No. 83/7434 (published October 5, 1983) describes methods for the synthesis of cyclopropylpyrimidines and triazines substituted by such groups as alkyl, haloalkyl, alkoxy, haloalkoxy, alkylamino, dialkylamino or alkoxyalkyl.

In addition, general methods for preparing aminopyrimidines and triazines have been reviewed in the following publications:
"The Chemistry of Heterocyclic Compounds", a series published by Interscience Publishers, Inc., New York and London;
"Pyrimidines", Vol. 16 of the same series by D. J. Brown;
"s -Triazines and Derivatives": Vol. 12 of the same series by E. M. Smolin and L. Rappoport; and
F. C. Schaefer, U.S. Patent 3,154,547 and K. R. Huffman and F. C., Schaefer, J. Org. Chem. , 28 , 1812 (1963), which describes the synthesis of triazines.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydroxide). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g., alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium sodium or calcium salt.

Acid addition salts, useful in this invention, can be obtained by reacting a compound of Formula I with a suitable acid, e.g., p -toluenesulfonic acid or trichloroacetic acid.

The preparation of the compounds of this invention is further illustrated by the following specific examples. Unless otherwise indicated, temperatures are in degrees centigrade.

Example 1
N-Cyclopropyl-1,2-benzenedisulfonamide

A solution of 1.28 g of 2-(aminosulfonyl)benzenesulfonyl chloride in 12 ml of dry THF was cooled to -65°C and a solution of 0.80 ml of cyclopropylamine in 12 ml of dry THF added dropwise. The resulting mixture was allowed to warm to room temperature, filtered, and the filtrate concentrated in vacuo . The solid residue was partitioned between water and ethyl acetate, the organic phase was separated. washed with water, dried ($MgSO_4$), concentrated in vacuo , and the residue triturated with 1-chlorobutane to provide 1.15 g of N-cyclopropyl-1,2-benzenedisulfonamide as a white solid, m.p. 202-203°C.

NMR($CDCl_3$/DMSO-$d_6$)δ: 0.7 (br d, 4H, $-CH_2CH_2-$);
2.2 (m, 1H, CH of cyclopropane);
6.5 (br s, 1H, $SO_2N\underline{H}$ cyclopropyl);
7.0 (br s, 2H, $SO_2NH_2$);
7.7-7.9 (m, 2H, aromatics); and
8.2-8.4 (m, 2H, aromatics).

Example 2

N-Cyclopropyl-N'-[(4,6-dimethoxypyrimidin-2-yl)-aminocarbonyl]-1,2-benzenedisulfonamide

To a solution of 0.14 g of the product of Example 1 and 0.15 g of phenyl(4,6-dimethoxypyrimidin-2-yl)-carbamate in 2 ml of dry acetonitrile was added 0.1 ml of 1,5-diazabicyclo[5.4.0]undec-5-ene (DBU). The solution was stirred for 15 minutes at room temperature, diluted with 2 ml of water and acidified with 5% hydrochloric acid. The precipitated product was collected by filtration, washed successively with water, diethyl ether, and 1-chlorobutane, and dried in vacuo at 40°C to afford 0.19 g of the subject compound. m.p. 197.5-199°C (dec.).

NMR($CDCl_3$)δ: 0.6 (m, 4H, $-CH_2CH_2-$);
2.15 (m, 1H, CH of cyclopropane);
3.98 (s, 6H, $OCH_3$'s);
5.76 (s, 1H, pyr. $C_5$-H);
6.16 (br s, 1H, $SO_2N\underline{H}$ cyclopropyl);
7.19 (br s, 1H, $-SO_2NHCON\underline{H}-$);
7.8 (m, 2H, aromatics);
8.26 (m, 1H, aromatic);
8.58 (m, 1H, aromatic); and
12.7 (br s, 1H, $-SO_2NHCO-$).
IR(nujol) 1705 $cm^{-1}$ (C=O).

Example 3

2-(2,2-Dimethylhydrazinosulfonyl)-benzenesulfonamide

A solution of 1.28 g of 2-(aminosulfonyl)benzenesulfonyl chloride in 12 mL of dry tetrahydrofuran (THF) was cooled to -65°C and a solution of 0.88 mL of 1,1-dimethylhydrazine in 12 mL of dry THF added dropwise. The resulting mixture was allowed to warm to room temperature, filtered, and the filtrate concentrated in vacuo . The solid residue was partitioned between water and ethyl acetate, the organic phase was separated, washed with water, washed with brine, dried ($MgSO_4$), concentrated in vacuo , and the residue triturated with 1-chlorobutane to provide 1.07 g of the subject compound, m.p. 122-124°C(dec).

NMR ($CDCl_3$/DMSO-$d_6$)δ 2.37 (s,6H,$N(CH_3)_2$);
6.9 (br s, 2H, $SO_2NH_2$);
7.1 (br s, 1H, $SO_2N\underline{H}NMe_2$);
7.75-7.9 (m, 2H, aromatics); and
8.25-8.4 (m, 2H, aromatics).

Example 4
2-(2,2-Dimethylhydrazinosulfonyl)-N-[(4,6-dimethoxypyrimidin-2-yl)aminocarbonyl]benzenesulfonamide

To a solution of 0.14 g of the product of Example 1 and 0.15 g of phenyl(4,6-dimethoxypyrimidin-2-yl) carbamate in 2 mL of dry acetonitrile was added 0.1 mL of 1,5-diazabicyclo[5.4.0]undec-5-ene. The solution was stirred for 15 minutes at room temperature, diluted with 2 mL of water and acidified with 5% hydrochloric acid to a pH of 4. The precipitated product was collected by filtration, washed thoroughly with water, washed with 1-chlorbutane, and dried in vacuo at 40°C to afford 0.02 g of the subject compound, m.p. 127-128°C (dec).

NMR($CDCl_3$)δ 2.37 (s, 6H, $N(CH_3)_2$);
3.99 (s, 6H, $OCH_3$'s);
5.77 (s, 1H, pyr. C5-H);
6.72 (br s, 1H, $SO_2N\underline{H}NMe_2$);
7.18 (br s, 1H, C(=O)N$\underline{H}$ pyr.);
7.82 (m, 2H, aromatics);
8.31 (m, 1H, aromatic);
8.56 (m, 1H, aromatic); and
12.68 (br s, 1H, $SO_2NHCO$)
IR (nujol) 1730, 1710 $cm^{-1}$ (C=O).

Using the methods described in Equations 1 to 13 and Examples 1 to 4, or simple modifications thereof, the compounds of Tables I, Ia, II, IIa, III, IIIa, can be prepared.

## General Structures for Tables I-IIIa

Structure I

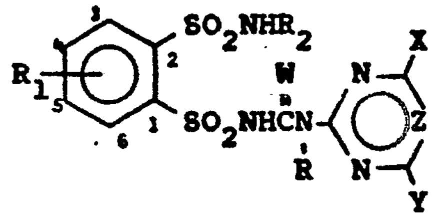

Structure III

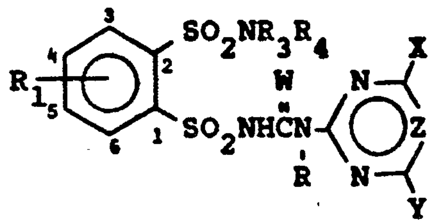

Structure V

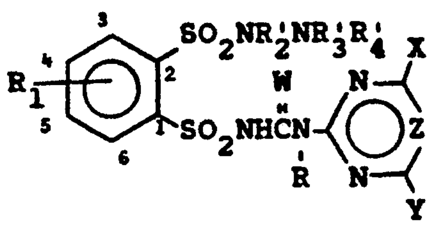

**Table I**

**Structure I, W = O**

| R | $R_1$ | $R_2$ | X | Y | Z | m.p.(°C) |
|---|---|---|---|---|---|---|
| H | H | n-$C_5H_{11}$ | $OCH_3$ | $CH_3$ | N | |
| H | H | n-$C_5H_{11}$ | $OCH_3$ | $OCH_3$ | CH | |
| H | H | n-$C_5H_{11}$ | Cl | $OCH_3$ | CH | |
| H | H | $CH_2CH_2CH(CH_3)_2$ | $CH_3$ | $OCH_3$ | CH | |
| H | H | $CH_2CH_2CH(CH_3)_2$ | $CH_3$ | $OC_2H_5$ | CH | |
| H | H | $CH_2CH_2CH(CH_3)_2$ | $OCH_3$ | $OCH_3$ | N | |
| H | H | $CH_2CH_2CH_2CH(CH_3)_2$ | $OCH_3$ | $CH_3$ | N | |
| H | H | $CH_2CH_2CH_2CH(CH_3)_2$ | $OCH_3$ | $CH_3$ | CH | |
| H | H | $CH_2CH_2CH_2CH(CH_3)_2$ | $OCH_3$ | $OCH_3$ | CH | |
| H | H | $CH(CH_3)CH_2CH_2CH_2CH_3$ | $OCH_3$ | $OCH_3$ | CH | |
| H | H | $CH(CH_3)CH_2CH_2CH_2CH_3$ | $OCH_3$ | $OCH_3$ | N | |
| H | H | $CH(CH_3)CH_2CH_2CH_2CH_3$ | $CH_3$ | $OCH_3$ | N | |
| H | H | $CH_2CH_2CH_2CH=CH_2$ | Cl | $OC_2H_5$ | CH | |
| H | H | $CH_2CH_2CH_2CH=CH_2$ | $CH_3$ | $OCH_3$ | N | |
| H | H | $CH_2CH_2CH_2CH=CH_2$ | $CH_3$ | $OCH_3$ | CH | |
| H | H | $CH=CF_2$ | $OCH_3$ | $OCH_3$ | CH | |
| H | H | $CH=CF_2$ | $CH_3$ | $OCH_3$ | N | |
| H | H | $CH_2CCl=CHCl$ | $CH_3$ | $OCH_3$ | N | |
| H | H | $CH_2CCl=CHCl$ | $OCH_3$ | $OCH_3$ | N | |
| H | H | $CH_2CCl=CHCl$ | $OCH_3$ | $OCH_3$ | CH | |
| H | H | $CH_2CCl=CHCl$ | Cl | $OC_2H_5$ | CH | |
| H | H | $CH_2CH=CHCF_3$ | $CH_3$ | $CH_3$ | CH | |
| H | H | $CH_2CH=CHCF_3$ | $CH_3$ | $OCH_3$ | N | |
| H | H | $CH_2CH_2CH=CHCH_2CH_2Cl$ | $CH_3$ | $OCH_3$ | N | |
| H | H | $CH_2CH_2CH=CHCH_2CH_2Cl$ | $OCH_3$ | $OCH_3$ | CH | |
| H | H | $CH_2C\equiv CH$ | $OCH_3$ | $OCH_3$ | CH | 206-207(d) |
| H | H | $CH_2C\equiv CH$ | Cl | $OCH_3$ | CH | 207.5-209(d) |
| H | H | $CH_2C\equiv CH$ | $CH_3$ | $OCH_3$ | CH | 199-200(d) |
| H | H | $CH_2C\equiv CH$ | $CH_3$ | $CH_3$ | CH | 201-203(d) |
| H | H | $CH_2C\equiv CH$ | $CH_3$ | $OCH_3$ | N | 198-201(d) |
| H | H | $CH_2C\equiv CH$ | $OCH_3$ | $OCH_3$ | N | 187-189(d) |

**Table I (continued)**

| R | $R_1$ | $R_2$ | X | Y | Z | m.p.(°C) |
|---|---|---|---|---|---|---|
| H | H | $CH_2C≡CH$ | $OCH_3$ | $C≡CH$ | N | |
| H | H | $CH_2C≡CH$ | Br | $OCH_3$ | CH | |
| H | H | $CH_2C≡CH$ | Br | $NHCH_3$ | CH | |
| H | H | $CH_2C≡CH$ | $CH_3$ | $N(CH_3)_2$ | CH | |
| H | H | $CH_2C≡CH$ | $OC_2H_5$ | $OCH_3$ | CH | |
| H | H | $CH_2C≡CH$ | $OCH_3$ | $C_2H_5$ | CH | |
| H | H | $CH_2C≡CCH_3$ | $OCH_3$ | $CH_3$ | N | |
| H | H | $CH_2C≡CCH_3$ | $OCH_3$ | $OCH_3$ | N | |
| H | H | $CH_2C≡CCH_3$ | $OCH_3$ | $OCH_3$ | CH | |
| H | H | $CH_2C≡CCH_3$ | $CH_3$ | H | CH | |
| H | H | $CH_2CH_2CH_2C≡CH$ | $OCH_3$ | $CH_3$ | N | |
| H | H | $CH_2CH_2CH_2C≡CH$ | $OCH_3$ | $OCH_3$ | CH | |
| H | H | $CH_2CH_2C≡CCH_2CH_3$ | $OCH_3$ | $OCH_3$ | CH | |
| H | H | $CH_2CH_2C≡CCH_2CH_3$ | $CH_3$ | $OCH_3$ | N | |
| H | H | cyclopropyl | H | H | CH | |
| H | H | cyclopropyl | $CH_3$ | H | CH | |
| H | H | cyclopropyl | $CH_2CH_2CH_2CH_3$ | $CH_3$ | CH | |
| H | H | cyclopropyl | $CH_2CH_2CF_3$ | $CH_3$ | CH | |
| H | H | cyclopropyl | $CH_2CF_3$ | $CH_3$ | CH | |
| H | H | cyclopropyl | $CH_2CH_2Cl$ | $CH_3$ | CH | |
| H | H | cyclopropyl | $CF_3$ | $CH_3$ | CH | |
| H | H | cyclopropyl | $CH_2Cl$ | $CH_3$ | CH | |
| H | H | cyclopropyl | $CH_2Br$ | $CH_3$ | CH | |
| H | H | cyclopropyl | $CH_2F$ | $CH_3$ | CH | |
| H | H | cyclopropyl | $CH_3$ | $CH_3$ | CH | 190-192(d) |
| H | H | cyclopropyl | $OCH_3$ | $CH_3$ | CH | 192-193.5(d) |
| H | H | cyclopropyl | $OCH_3$ | $OCH_3$ | CH | 197.5-199(d) |
| H | H | cyclopropyl | F | $OCH_3$ | CH | |
| H | H | cyclopropyl | Cl | $OCH_3$ | CH | 210-213(d) |
| H | H | cyclopropyl | Br | $OCH_3$ | CH | |
| H | H | cyclopropyl | I | $OCH_3$ | CH | |
| H | H | cyclopropyl | Cl | $OC_2H_5$ | CH | |

Table I (continued)

| R | $R_1$ | $R_2$ | X | Y | Z | m.p.(°C) |
|---|---|---|---|---|---|---|
| H | H | cyclopropyl | Cl | $N(OCH_3)CH_3$ | CH | |
| H | H | cyclopropyl | Cl | $NHCH_3$ | CH | |
| H | H | cyclopropyl | Cl | $N(CH_3)_2$ | CH | |
| H | H | cyclopropyl | $N(CH_3)_2$ | $N(CH_3)_2$ | CH | |
| H | H | cyclopropyl | $OC_2H_5$ | $CH_3$ | CH | |
| H | H | cyclopropyl | O-n-$C_4H_9$ | $CH_3$ | CH | |
| H | H | cyclopropyl | $OCH_2CF_3$ | $CH_3$ | CH | |
| H | H | cyclopropyl | $OCH_2CH_2CH_2Cl$ | $CH_3$ | CH | |
| H | H | cyclopropyl | $SCH_3$ | $CH_3$ | CH | |
| H | H | cyclopropyl | $SC_2H_5$ | $CH_3$ | CH | |
| H | H | cyclopropyl | $SCH_3$ | $OC_2H_5$ | CH | |
| H | H | cyclopropyl | $CH_3$ | H | N | |
| H | H | cyclopropyl | $CH_3$ | $CH_3$ | N | |
| H | H | cyclopropyl | $CH_3$ | C≡CH | N | |
| H | H | cyclopropyl | $CH_3$ | $C≡CCH_3$ | N | |
| H | H | cyclopropyl | $CH_3$ | Cl | N | |
| H | H | cyclopropyl | $CH_3$ | $CH_2CH_2Cl$ | N | |
| H | H | cyclopropyl | $CH_3$ | $CH_2OCH_3$ | N | |
| H | H | cyclopropyl | $CH_3$ | $CH_2OC_2H_5$ | N | |
| H | H | cyclopropyl | $CH_3$ | $SCH_3$ | N | |
| H | H | cyclopropyl | $CH_3$ | O-n-$C_4H_9$ | N | |
| H | H | cyclopropyl | $CH_3$ | $OCH_2CH=CH_2$ | N | |
| H | H | cyclopropyl | $CH_3$ | $OCH_2C≡CCH_3$ | N | |
| H | H | cyclopropyl | $CH_3$ | $CH_2SCH_3$ | N | |
| H | H | cyclopropyl | $CH_3$ | $CF_3$ | CH | |
| H | H | cyclopropyl | $CH_3$ | $OCH_3$ | N | 184.5-185.5(d) |
| H | H | cyclopropyl | $OCH_3$ | $OCH_3$ | N | 190-192(d) |
| H | H | cyclopropyl | $OCH_3$ | cyclopropyl | CH | |
| H | H | cyclopropyl | $OCH_3$ | $OCF_3$ | CH | |
| H | H | cyclobutyl | $OCH_3$ | $OCH_3$ | CH | 195.5-197(d) |
| H | H | cyclobutyl | Cl | $OCH_3$ | CH | 214.5-217(d) |
| H | H | cyclobutyl | $CH_3$ | $OCH_3$ | CH | 191-192.5(d) |

Table I (continued)

| R | $R_1$ | $R_2$ | X | Y | Z | m.p.(°C) |
|---|---|---|---|---|---|---|
| H | H | cyclobutyl | $CH_3$ | $CH_3$ | CH | 180-184(d) |
| H | H | cyclobutyl | $CH_3$ | $N(C_2H_5)_2$ | CH | |
| H | H | cyclobutyl | $CH_3$ | $NHCH_2CH_2CH_3$ | CH | |
| H | H | cyclobutyl | $NH_2$ | $CH_3$ | N | |
| H | H | cyclobutyl | $SCH_2CH_2Cl$ | $CH_3$ | N | |
| H | H | cyclobutyl | $CH_2OC_2H_7$ | $CH_3$ | N | |
| H | H | cyclobutyl | $OC_2H_5OCH_3$ | $CH_3$ | CH | |
| H | H | cyclobutyl | $OCH_3$ | $CH_3$ | N | 190-192(d) |
| H | H | cyclobutyl | $OCH_3$ | $OCH_3$ | N | 191-192.5(d) |
| H | H | cyclobutyl | $CH_3$ | $CH_3$ | N | |
| H | H | cyclobutyl | $OCH_3$ | $C{\equiv}CH$ | N | |
| H | H | cyclobutyl | $CH_3$ | $SCH_3$ | N | |
| H | H | cyclopentyl | $OCH_3$ | $CH_3$ | CH | 182.5-183.5(d) |
| H | H | cyclopentyl | $OCH_2OCH_3$ | $CH_3$ | CH | |
| H | H | cyclopentyl | $OCH_2CH_2OCH_3$ | $CH_3$ | CH | |
| H | H | cyclopentyl | $NH_2$ | $CH_3$ | CH | |
| H | H | cyclopentyl | $CH_3$ | $CH_3$ | CH | 190-192(d) |
| H | H | cyclopentyl | $OCH_3$ | $OCH_3$ | CH | 187-188.5(d) |
| H | H | cyclopentyl | Cl | $OCH_3$ | CH | 214-215.5(d) |
| H | H | cyclopentyl | $SCH_3$ | $OCH_3$ | CH | |
| H | H | cyclopentyl | $OCH_3$ | $OCH_3$ | N | 193-195(d) |
| H | H | cyclopentyl | $CH_3$ | $OCH_3$ | N | 180-182(d) |
| H | H | cyclopentyl | $CH_3$ | $CH_3$ | N | |
| H | H | cyclopentyl | $CH_3$ | n-$C_4H_9$ | N | |
| H | H | cyclohexyl | $CH_3$ | $CH_3$ | N | |
| H | H | cyclohexyl | $CH_3$ | $OCH_3$ | N | |

Table I (continued)

| R | $R_1$ | $R_2$ | X | Y | Z | m.p.(°C) |
|---|---|---|---|---|---|---|
| H | H | cyclohexyl | $OCH_3$ | $OCH_3$ | N | |
| H | H | cyclohexyl | $OCH_3$ | $OCH_3$ | CH | |
| H | H | cyclohexyl | Cl | $OCH_3$ | CH | |
| H | H | cyclohexyl | $CH_3$ | $OCH_3$ | CH | |
| H | H | cyclohexyl | $CH_3$ | $CH_3$ | CH | |
| H | H | 3-$CH_3$-cyclopentyl | $OCH_3$ | $OCH_3$ | CH | |
| H | H | 3-$CH_3$-cyclopentyl | $OCH_3$ | $CH_3$ | N | |
| H | H | 4-$CH_3$-cyclohexyl | $OCH_3$ | $OCH_3$ | CH | |
| H | H | 4-$CH_3$-cyclohexyl | $CH_3$ | $OCH_3$ | N | |
| H | H | $CH_2$-cyclopropyl | $CH_3$ | $CH_3$ | N | |
| H | H | $CH_2$-cyclopropyl | $OCH_3$ | $CH_3$ | N | 182-184 |
| H | H | $CH_2$-cyclopropyl | $OCH_3$ | $OCH_3$ | N | 184-186 |
| H | H | $CH_2$-cyclopropyl | $OCH_3$ | $OCH_3$ | CH | 171-173 |
| H | H | $CH_2$-cyclopropyl | $CH_3$ | $OCH_3$ | CH | 173-176 |
| H | H | $CH_2$-cyclopropyl | Cl | $OCH_3$ | CH | 207-209 |
| H | H | $CH_2$-cyclopropyl | $CH_3$ | $CH_3$ | CH | 186-188 |
| H | H | cyclohexen-1-yl | $CH_3$ | $OCH_3$ | N | |
| H | H | cyclohexen-1-yl | $OCH_3$ | $OCH_3$ | CH | |
| H | H | $CH_2$-oxirane | $OCH_3$ | $OCH_3$ | CH | 184-185(d) |
| H | H | $CH_2$-oxirane | Cl | $OCH_3$ | CH | |
| H | H | $CH_2$-oxirane | $CH_3$ | $OCH_3$ | CH | |
| H | H | $CH_2$-oxirane | $CH_3$ | $CH_3$ | N | |
| H | H | $CH_2$-oxirane | $CH_3$ | $OCH_3$ | N | |
| H | H | $CH_2$-oxirane | $OCH_3$ | $OCH_3$ | N | |
| H | H | $CH_2$-oxirane | $OCH_3$ | $C\equiv CH$ | N | |
| H | H | $CH_2CH_2F$ | $OCH_3$ | $OCH_3$ | N | 178-179.5(d) |
| H | H | $CH_2CH_2F$ | $CH_3$ | $OCH_3$ | N | 192.5-194(d) |
| H | H | $CH_2CH_2F$ | Cl | $OCH_3$ | CH | 205-208(d) |
| H | H | $CH_2CH_2F$ | $OCH_3$ | $OCH_3$ | CH | 199.5-201(d) |
| H | H | $CH_2CH_2Cl$ | $OCH_3$ | $OCH_3$ | CH | |
| H | H | $CH_2CH_2Cl$ | $OCH_3$ | $CH_3$ | CH | |
| H | H | $CH_2CH_2Cl$ | $OCH_3$ | $CH_3$ | N | |

Table I (continued)

| R | $R_1$ | $R_2$ | X | Y | Z | m.p.(°C) |
|---|---|---|---|---|---|---|
| H | H | $CH_2CH_2Br$ | $OCH_3$ | $CH_3$ | N | |
| H | H | $CH_2CH_2Br$ | $OCH_3$ | $OCH_3$ | CH | |
| H | H | $CH_2CH(OCH_3)_2$ | $CH_3$ | $OCH_3$ | N | 174-175(d) |
| H | H | $CH_2CH(OCH_3)_2$ | $OCH_3$ | $OCH_3$ | CH | 193.5-195(d) |
| H | H | $CH_2CH(OC_2H_5)_2$ | $OCH_3$ | $CH_3$ | N | |
| H | H | $CH_2CH(OC_2H_5)_2$ | $OCH_3$ | $OCH_3$ | CH | |
| H | H | $CH_2$-1,3-dioxan-2-yl | $CH_3$ | $OCH_3$ | N | |
| H | H | $CH_2$-1,3-dioxan-2-yl | $OCH_3$ | $OCH_3$ | CH | |
| H | H | $CH_2CH_2CH_2OCH_3$ | $OCH_3$ | $CH_3$ | N | |
| H | H | $CH_2CH_2CH_2OCH_3$ | $OCH_3$ | $OCH_3$ | CH | |
| H | H | $OCH_3$ | $CH_3$ | $OCH_3$ | N | 205-206(d) |
| H | H | $OCH_3$ | $OCH_3$ | $OCH_3$ | CH | 209-211(d) |
| H | H | $C_6H_5$ | $OCH_3$ | $OCH_3$ | CH | |
| H | H | $C_6H_5$ | $CH_3$ | $OCH_3$ | N | |
| H | H | 2-$CH_3$-$C_6H_4$ | $OCH_3$ | $OCH_3$ | CH | |
| H | H | 2-$CH_3$-$C_6H_4$ | $OCH_3$ | $CH_3$ | N | |
| H | H | 2-Cl-$C_6H_4$ | $OCH_3$ | $CH_3$ | N | |
| H | H | 2-Cl-$C_6H_4$ | $OCH_3$ | $OCH_3$ | CH | |
| H | H | 4-Br-$C_6H_4$ | $CH_3$ | $OCH_3$ | N | |
| H | H | 4-Br-$C_6H_4$ | $OCH_3$ | $OCH_3$ | CH | |
| H | H | 3-$CF_3$-$C_6H_4$ | $OCH_3$ | $CH_3$ | N | |
| H | H | 3-$CF_3$-$C_6H_4$ | $OCH_3$ | $OCH_3$ | CH | |
| H | H | 4-$CH_3O$-$C_6H_4$ | $OCH_3$ | $CH_3$ | N | |
| H | H | 4-$CH_3O$-$C_6H_4$ | $OCH_3$ | $OCH_3$ | CH | |
| H | H | $CH_2C_6H_5$ | $OCH_3$ | $OCH_3$ | CH | 198-199(d) |
| H | H | $CH_2C_6H_5$ | $CH_3$ | $OCH_3$ | CH | 180-182(d) |
| H | H | $CH_2C_6H_5$ | Cl | $OCH_3$ | CH | 168-170.5(d) |
| H | H | $CH_2C_6H_5$ | $CH_3$ | $CH_3$ | CH | 199-201(d) |
| H | H | $CH_2C_6H_5$ | $CH_3$ | $CH_3$ | N | |
| H | H | $CH_2C_6H_5$ | $OCH_3$ | $CH_3$ | N | 160.5-162.5(d) |
| H | H | $CH_2C_6H_5$ | $OCH_3$ | $OCH_3$ | N | 173-175(d) |
| H | H | $CH_2COCH_3$ | $OCH_3$ | $CH_3$ | N | |

Table I (continued)

| R | $R_1$ | $R_2$ | X | Y | Z | m.p.(°C) |
|---|---|---|---|---|---|---|
| H | H | $CH_2COCH_3$ | $OCH_3$ | $OCH_3$ | CH | |
| H | H | CN | $OCH_3$ | $CH_3$ | N | |
| H | H | CN | $OCH_3$ | $OCH_3$ | CH | |
| H | H | $CH_2CH_2OH$ | $OCH_3$ | $CH_3$ | N | 175-178(d) |
| H | H | $CH_2CH_2OH$ | $OCH_3$ | $OCH_3$ | CH | 148-151(d) |
| H | H | $CH_2CH_2OC(O)CH_3$ | $CH_3$ | $OCH_3$ | N | |
| H | H | $CH_2CH_2OC(O)CH_3$ | $OCH_3$ | $OCH_3$ | CH | |
| H | H | $CH_2CH_2SO_2CH_3$ | $CH_3$ | $OCH_3$ | N | |
| H | H | $CH_2CH_2SO_2CH_3$ | $OCH_3$ | $OCH_3$ | CH | |
| H | H | $CH_2CH_2CO_2C_2H_5$ | $CH_3$ | $OCH_3$ | N | |
| H | H | $CH_2CH_2CO_2C_2H_5$ | $OCH_3$ | $OCH_3$ | CH | |
| H | H | $CH_2CH_2CH_2C(O)N(CH_3)_2$ | $CH_3$ | $OCH_3$ | N | |
| H | H | $CH_2CH_2CH_2C(O)N(CH_3)_2$ | $OCH_3$ | $OCH_3$ | CH | |
| H | H | $CH_2CH_2CH_2CH_2OP(O)(OCH_3)_2$ | $OCH_3$ | $CH_3$ | N | |
| H | H | $CH_2CH_2CH_2CH_2OP(O)(OCH_3)_2$ | $OCH_3$ | $OCH_3$ | CH | |
| H | H | $CH_2CH_2CH_2SCH_3$ | $OCH_3$ | $CH_3$ | N | |
| H | H | $CH_2CH_2CH_2SCH_3$ | $OCH_3$ | $OCH_3$ | CH | |
| H | H | $CH_2CH_2CH_2SO_2CH_3$ | $OCH_3$ | $CH_3$ | N | |
| H | H | $CH_2CH_2CH_2SO_2CH_3$ | $OCH_3$ | $OCH_3$ | CH | |
| H | H | 1,3-dithiolan-2-yl | $CH_3$ | $OCH_3$ | N | |
| H | H | 1,3-dithiolan-2-yl | $OCH_3$ | $OCH_3$ | CH | |
| H | H | $CH_2$-tetrahydrofuran-2-yl | $OCH_3$ | $OCH_3$ | CH | |
| H | H | $CH_2$-tetrahydrofuran-2-yl | $CH_3$ | $OCH_3$ | N | |
| H | H | 4,6-dimethoxy-pyrimidin-2-yl | $CH_3$ | $OCH_3$ | CH | |
| H | H | 4-picolyl | $OCH_3$ | $OCH_3$ | CH | |
| H | 3-$CH_3$ | $\underline{n}$-$C_5H_{11}$ | $OCH_3$ | $OCH_3$ | CH | |
| H | 4-$CH_3$ | $\underline{n}$-$C_5H_{11}$ | $OCH_3$ | $OCH_3$ | CH | |
| H | 5-$C_2H_5$ | $\underline{n}$-$C_5H_{11}$ | $OCH_3$ | $OCH_3$ | CH | |
| H | 5-$CF_3$ | $CH_2$-cyclopropyl | $OCH_3$ | $OCH_3$ | CH | |
| H | 5-$CH_2CH_2Cl$ | $CH_2$-cyclopropyl | $OCH_3$ | $OCH_3$ | CH | |
| H | 5-Cl | $CH_2$-cyclopropyl | $OCH_3$ | $OCH_3$ | CH | |

Table I (continued)

| R | $R_1$ | $R_2$ | X | Y | Z | m.p.(°C) |
|---|---|---|---|---|---|---|
| H | 5-Br | $CH_2$-cyclopropyl | $OCH_3$ | $OCH_3$ | CH | |
| H | 5-F | $CH_2$-cyclopropyl | $OCH_3$ | $OCH_3$ | CH | |
| H | 5-$NO_2$ | cyclobutyl | $OCH_3$ | $OCH_3$ | CH | |
| H | 5-$OCH_3$ | cyclobutyl | $OCH_3$ | $OCH_3$ | CH | |
| H | 5-$SO_2NHCH_3$ | cyclobutyl | $OCH_3$ | $OCH_3$ | CH | |
| H | 5-$SCH_3$ | cyclobutyl | $OCH_3$ | $OCH_3$ | CH | |
| H | 5-$SOC_2H_5$ | cyclobutyl | $OCH_3$ | $OCH_3$ | CH | |
| H | 5-$SO_2C_2H_5$ | $CH_2C_6H_5$ | $OCH_3$ | $OCH_3$ | CH | |
| H | 5-CN | $CH_2C_6H_5$ | $OCH_3$ | $OCH_3$ | CH | |
| H | 6-$CH_2CH_2CH_3$ | $CH_2C_6H_5$ | $OCH_3$ | $OCH_3$ | CH | |
| H | 6-$CH_2CF_3$ | $CH_2C_6H_5$ | $OCH_3$ | $OCH_3$ | CH | |
| H | 6-$CF_3$ | $CH_2C_6H_5$ | $OCH_3$ | $OCH_3$ | CH | |
| H | 6-$SCH_2F$ | $CH_2C_6H_5$ | $OCH_3$ | $OCH_3$ | CH | |
| H | 6-$OCH_2CF_3$ | $CH_2C_6H_5$ | $OCH_3$ | $OCH_3$ | CH | |
| H | 6-$CO_2CH_3$ | cyclopropyl | $OCH_3$ | $OCH_3$ | CH | |
| H | 6-$CO_2C_2H_5$ | cyclopropyl | $OCH_3$ | $OCH_3$ | CH | |
| H | 6-$CO_2CH_2CH{=}CH_2$ | cyclopropyl | $OCH_3$ | $OCH_3$ | CH | |
| H | 6-$CO_2CH_2CH_2CN$ | cyclopropyl | $OCH_3$ | $OCH_3$ | CH | |
| H | 6-$CO_2$-cyclohexyl | cyclopropyl | $OCH_3$ | $OCH_3$ | CH | |
| H | 6-$CH_2OCH_3$ | cyclopropyl | $OCH_3$ | $OCH_3$ | CH | |
| H | 6-Cl | cyclopropyl | $OCH_3$ | $OCH_3$ | CH | |
| H | 6-$SCH_3$ | cyclopropyl | $OCH_3$ | $OCH_3$ | CH | |
| H | 6-$SO_2CH_2CH_2CH_3$ | cyclopropyl | $OCH_3$ | $OCH_3$ | CH | |
| H | 6-$OCH_3$ | cyclopropyl | $OCH_3$ | $OCH_3$ | CH | |
| $CH_3$ | 6-$OCH_3$ | cyclopropyl | $OCH_3$ | $OCH_3$ | CH | |
| $CH_3$ | H | cyclopropyl | $OCH_3$ | $OCH_3$ | CH | |
| $CH_3$ | H | cyclopropyl | $CH_3$ | $OCH_3$ | N | |
| $CH_3$ | H | cyclopropyl | $OCH_3$ | $OCH_3$ | N | |
| $CH_3$ | H | $CH_2C{\equiv}CH$ | $OCH_3$ | $OCH_3$ | CH | |
| $CH_3$ | H | $CH_2C{\equiv}CH$ | $CH_3$ | $OCH_3$ | N | |
| $CH_3$ | H | $CH_2C{\equiv}CH$ | Cl | $OCH_3$ | CH | |
| $CH_3$ | H | $CH_2$-cyclobutyl | $OCH_3$ | $OCH_3$ | CH | |

Table I (continued)

| R | $R_1$ | $R_2$ | X | Y | Z | m.p.(°C) |
|---|---|---|---|---|---|---|
| $CH_3$ | H | $CH_2$-cyclobutyl | $OCH_3$ | $CH_3$ | N | |
| $CH_3$ | H | $CH_2C_6H_5$ | $OCH_3$ | $CH_3$ | N | |
| $CH_3$ | H | $CH_2C_6H_5$ | $OCH_3$ | $OCH_3$ | CH | |
| $CH_3$ | H | $CH_2$-oxirane | $OCH_3$ | $CH_3$ | N | |
| $CH_3$ | H | $CH_2$-oxirane | $OCH_3$ | $OCH_3$ | CH | |
| $CH_3$ | H | 4-Cl-$C_6H_4$ | $CH_3$ | $OCH_3$ | N | |
| $CH_3$ | H | 4-Cl-$C_6H_4$ | $OCH_3$ | $OCH_3$ | CH | |
| H | H | $CH_2CH_2F$ | $CH_3$ | $CH_3$ | CH | 188-190(d) |
| H | H | $CH_2CH_2F$ | $CH_3$ | $OCH_3$ | CH | 192-193.5(d) |
| H | H | $CH_2CH_2Cl$ | $CH_3$ | $CH_3$ | CH | |
| H | H | $CH_2CH_2Cl$ | Cl | $OCH_3$ | CH | |
| H | H | $CH_2CH_2Cl$ | $OCH_3$ | $OCH_3$ | N | |
| H | H | $CH_2CH_2Br$ | $CH_3$ | $CH_3$ | CH | |
| H | H | $CH_2CH_2Br$ | $CH_3$ | $OCH_3$ | CH | |
| H | H | $CH_2CH_2Br$ | Cl | $OCH_3$ | CH | |
| H | H | $CH_2CH_2Br$ | $OCH_3$ | $OCH_3$ | N | |
| H | H | $CH_2CH(OCH_3)_2$ | $CH_3$ | $CH_3$ | CH | 185-186.5(d) |
| H | H | $CH_2CH(OCH_3)_2$ | $CH_3$ | $OCH_3$ | CH | 187-188.5(d) |
| H | H | $CH_2CH(OCH_3)_2$ | Cl | $OCH_3$ | CH | 180-182(d) |
| H | H | $CH_2CH(OCH_3)_2$ | $OCH_3$ | $OCH_3$ | N | 176-178(d) |
| H | H | $CH_2COCH_3$ | $CH_3$ | $CH_3$ | CH | |
| H | H | $CH_2COCH_3$ | $CH_3$ | $OCH_3$ | CH | |
| H | H | $CH_2COCH_3$ | Cl | $OCH_3$ | CH | |
| H | H | $CH_2COCH_3$ | $OCH_3$ | $OCH_3$ | N | |
| H | H | CN | $CH_3$ | $CH_3$ | CH | |
| H | H | CN | $CH_3$ | $OCH_3$ | CH | |
| H | H | CN | Cl | $OCH_3$ | CH | |
| H | H | CN | $OCH_3$ | $OCH_3$ | N | |
| H | H | $CH_2CH_2OH$ | $CH_3$ | $CH_3$ | CH | 177-179(d) |
| H | H | $CH_2CH_2OH$ | $CH_3$ | $OCH_3$ | CH | 163-165(d) |
| H | H | $CH_2CH_2OH$ | Cl | $OCH_3$ | CH | 151-153(d) |
| H | H | $CH_2CH_2OH$ | $OCH_3$ | $OCH_3$ | N | >250 |

Table I (continued)

| R | $R_1$ | $R_2$ | X | Y | Z | m.p.(°C) |
|---|---|---|---|---|---|---|
| H | H | $OCH_3$ | $CH_3$ | $OCH_3$ | CH | 206-208(d) |
| H | H | $OCH_3$ | Cl | $OCH_3$ | CH | 225-227(d) |
| H | H | $OCH_3$ | $OCH_3$ | $OCH_3$ | N | 197-199(d) |
| H | H | Thiazol-2-yl | $OCH_3$ | $OCH_3$ | CH | |
| H | H | Thiazol-2-yl | $CH_3$ | $OCH_3$ | N | |
| H | H | 1,3,4-Thiadiazol-2-yl | $OCH_3$ | $OCH_3$ | CH | |
| H | H | 1,3,4-Thiadiazol-2-yl | $CH_3$ | $OCH_3$ | N | |
| H | H | 2-Picolyl | $OCH_3$ | $OCH_3$ | CH | |
| H | H | 2-Picolyl | $CH_3$ | $OCH_3$ | N | |
| H | H | 3-Picolyl | $OCH_3$ | $OCH_3$ | CH | |
| H | H | 3-Picolyl | $CH_3$ | $OCH_3$ | N | |
| H | H | 2-Pyridyl | $OCH_3$ | $OCH_3$ | CH | |
| H | H | 2-Pyridyl | $CH_3$ | $OCH_3$ | N | |
| H | H | 3-Pyridyl | $OCH_3$ | $OCH_3$ | CH | |
| H | H | 3-Pyridyl | $CH_3$ | $OCH_3$ | N | |
| H | H | 4-Pyridyl | $OCH_3$ | $OCH_3$ | CH | |
| H | H | 4-Pyridyl | $CH_3$ | $OCH_3$ | N | |
| H | H | $CH_2CH_2$-Pyridin-2-yl | $OCH_3$ | $OCH_3$ | CH | |
| H | H | $CH_2CH_2$-Pyridin-2-yl | $CH_3$ | $OCH_3$ | N | |
| H | H | $CH_2$-1-Methyl-pyrrolidin-2-yl | $OCH_3$ | $OCH_3$ | CH | |
| H | H | $CH_2$-1-Methyl-pyrrolidin-2-yl | $CH_3$ | $OCH_3$ | N | |
| H | H | $CH_2CH_2$-1-Methyl-pyrrol-2-yl | $OCH_3$ | $OCH_3$ | CH | |
| H | H | $CH_2CH_2$-1-Methyl-pyrrol-2-yl | $CH_3$ | $OCH_3$ | N | |
| H | 5-$OCH_2CF_3$ | cyclopropyl | $CH_3$ | $CH_3$ | CH | |
| H | 5-$OCH_2CF_3$ | cyclopropyl | $CH_3$ | $OCH_3$ | CH | |
| H | 5-$OCH_2CF_3$ | cyclopropyl | $OCH_3$ | $OCH_3$ | CH | |
| H | 5-$OCH_2CF_3$ | cyclopropyl | $CH_3$ | $OCH_3$ | N | |
| H | 5-$OCH_2CF_3$ | cyclopropyl | Cl | $OCH_3$ | CH | |

Table I (continued)

| R | $R_1$ | $R_2$ | X | Y | Z | m.p.(°C) |
|---|---|---|---|---|---|---|
| H | 5-$CH_2OCH_2CF_3$ | cyclopropyl | $OCH_3$ | $OCH_3$ | CH | |
| H | 5-$CH_2CN$ | cyclopropyl | $OCH_3$ | $OCH_3$ | CH | |
| H | 5-$N(CH_3)_2$ | cyclopropyl | $OCH_3$ | $OCH_3$ | CH | |
| H | H | $CH_2$-Pyridin-4-yl | $CH_3$ | $CH_3$ | CH | 177-179 |
| H | H | $CH_2$-Pyridin-4-yl | $CH_3$ | $OCH_3$ | CH | 172-174 |
| H | H | $CH_2$-Pyridin-4-yl | $OCH_3$ | $OCH_3$ | CH | 164-165 |
| H | H | $CH_2$-Pyridin-4-yl | $CH_3$ | $OCH_3$ | N | 177-178 |
| H | H | $CH_2$-Pyridin-4-yl | $OCH_3$ | $OCH_3$ | N | 174-177 |
| H | H | $CH_2$-Pyridin-4-yl | Cl | $OCH_3$ | CH | 180-181 |
| H | H | $CH_2CH_2CH_2Br$ | $CH_3$ | $CH_3$ | CH | 160-162 |
| H | H | $CH_2CH_2CH_2Br$ | $CH_3$ | $OCH_3$ | CH | 180-181 |
| H | H | $CH_2CH_2CH_2Br$ | $OCH_3$ | $OCH_3$ | CH | 163-165 |
| H | H | $CH_2CH_2CH_2Br$ | $CH_3$ | $OCH_3$ | N | 159-160 |
| H | H | $CH_2CH_2CH_2Br$ | $OCH_3$ | $OCH_3$ | N | 170-172 |
| H | H | $CH_2CH_2CH_2Br$ | Cl | $OCH_3$ | CH | 193-195 |
| H | H | $CH_2CH_2N(CH_3)_2$ | $CH_3$ | $OCH_3$ | CH | 132-134(d) |

**Table Ia**

**Structure I, W = S**

| R | $R_1$ | $R_2$ | X | Y | Z | m.p.(°C) |
|---|---|---|---|---|---|---|
| H | H | $CH_2C{\equiv}CH$ | $CH_3$ | $OCH_3$ | N | |
| H | H | $CH_2C{\equiv}CH$ | $OCH_3$ | $OCH_3$ | CH | |
| H | H | cyclopropyl | $CH_3$ | $OCH_3$ | CH | |
| H | H | cyclopropyl | $OCH_3$ | $OCH_3$ | CH | |
| H | H | $CH_2CH_2F$ | $OCH_3$ | $OCH_3$ | CH | |
| H | H | $CH_2CH_2F$ | $CH_3$ | $OCH_3$ | N | |
| H | H | $CH_2CH_2Cl$ | Cl | $OCH_3$ | CH | |
| H | H | $CH_2CH_2Cl$ | $OCH_3$ | $OCH_3$ | CH | |
| H | H | $OCH_3$ | $CH_3$ | $OCH_3$ | CH | |
| H | H | $OCH_3$ | $OCH_3$ | $OCH_3$ | CH | |
| H | H | $OC_2H_5$ | $CH_3$ | $OCH_3$ | N | |
| H | H | $OC_2H_5$ | $OCH_3$ | $OCH_3$ | CH | |
| H | H | $CH_2$-cyclopropyl | $CH_3$ | $OCH_3$ | CH | |
| H | H | $CH_2$-cyclopropyl | $OCH_3$ | $OCH_3$ | CH | |
| H | $OCH_3$ | $CH_2CH_2F$ | $OCH_3$ | $OCH_3$ | CH | |

**Table II**

**Structure III, W = O**

| R | $R_1$ | $R_3$ | $R_4$ | X | Y | Z | m.p.(°C) |
|---|---|---|---|---|---|---|---|
| H | H | $CH_3$ | $CH_2CH_2F$ | $OCH_3$ | $OCH_3$ | CH | |
| H | H | $CH_3$ | $CH_2CH_2F$ | $OCH_3$ | $CH_3$ | CH | |
| H | H | $CH_3$ | $CH_2CH_2F$ | $CH_3$ | $CH_3$ | CH | |
| H | H | $CH_3$ | $CH_2CH_2F$ | $CF_3$ | $CH_3$ | CH | |
| H | H | $CH_3$ | $CH_2CH_2F$ | Cl | $OCH_3$ | CH | |
| H | H | $CH_3$ | $CH_2CH_2F$ | $OCH_3$ | $OCH_3$ | N | |
| H | H | $CH_3$ | $CH_2CH_2F$ | $OCH_3$ | $CH_3$ | N | |
| H | H | $CH_3$ | $CH_2CH_2F$ | $CH_3$ | $CH_3$ | N | |
| H | H | $CH_3$ | $CH_2CH_2Cl$ | $OCH_3$ | $CH_3$ | N | |
| H | H | $CH_3$ | $CH_2CH_2Cl$ | $OCH_3$ | $OCH_3$ | N | |
| H | H | $CH_3$ | $CH_2CH_2Cl$ | $OCH_3$ | $OCH_3$ | CH | |
| H | H | $CH_3$ | $CH_2CH_2Cl$ | Cl | $OCH_3$ | CH | |
| H | H | $CH_3$ | $CH_2CH_2Cl$ | $CH_3$ | $OCH_3$ | CH | |
| H | H | $CH_3$ | $CH_2CH_2Cl$ | $CH_3$ | $CH_3$ | CH | |
| H | H | $CH_3$ | $CH_2CH_2Br$ | $OCH_3$ | $CH_3$ | N | |
| H | H | $CH_3$ | $CH_2CH_2Br$ | $OCH_3$ | $OCH_3$ | N | |
| H | H | $CH_3$ | $CH_2CH_2Br$ | Cl | $OCH_3$ | CH | |
| H | H | $CH_3$ | $CH_2CH_2Br$ | $OCH_3$ | $OCH_3$ | CH | |
| H | H | $CH_3$ | $CH_2CH_2Br$ | $CH_3$ | $CH_3$ | CH | |
| H | H | $CH_3$ | $CH_2CH_2Br$ | $OCH_3$ | $CH_3$ | CH | |
| H | H | $CH_3$ | $CH_2CH_2CH_2F$ | $OCH_3$ | $OCH_3$ | CH | |
| H | H | $CH_3$ | $CH_2CH_2CH_2F$ | $CH_3$ | $OCH_3$ | N | |
| H | H | $CH_3$ | $CH_2CH(OC_2H_5)_2$ | $CH_3$ | $CH_3$ | CH | |
| H | H | $CH_3$ | $CH_2CH(OC_2H_5)_2$ | $CH_3$ | $OCH_3$ | N | |
| H | H | $CH_3$ | $CH_2$-1,3-dioxan-2-yl | $CH_3$ | $OCH_3$ | N | |
| H | H | $CH_3$ | $CH_2$-1,3-dioxan-2-yl | $OCH_3$ | $OCH_3$ | CH | |
| H | H | $CH_3$ | $CH_2CH=CH-CF_3$ | $OCH_3$ | $CH_3$ | CH | |
| H | H | $CH_3$ | $CH_2CH=CH-CF_3$ | $OCH_3$ | $CH_3$ | N | |
| H | H | $CH_3$ | $CH_2$-oxirane | $OCH_3$ | $OCH_3$ | N | |
| H | H | $CH_3$ | $CH_2$-oxirane | $CH_3$ | $OCH_3$ | CH | |
| H | H | $CH_3$ | $CH_2$-oxirane | $OCH_3$ | $OCH_3$ | CH | |

Table II (continued)

| R | R1 | R3 | R4 | X | Y | Z | m.p. (°C) |
|---|---|---|---|---|---|---|---|
| H | H | $CH_3$ | $CH_2$-oxirane | Cl | $OCH_3$ | CH | |
| H | H | $CH_3$ | $CH_2$-oxirane | $CH_3$ | $CH_3$ | CH | |
| H | H | $CH_3$ | $CH_2$-oxirane | $OCH_3$ | $CH_3$ | N | |
| H | H | $CH_3$ | $CH_2C(O)CH_3$ | $OCH_3$ | $OCH_3$ | CH | |
| H | H | $CH_3$ | $CH_2CH_2CH_2OC(O)CH_2CH_3$ | $OCH_3$ | $OCH_3$ | CH | |
| H | H | $CH_3$ | $CH_2CH_2OSO_2CH_3$ | $OCH_3$ | $OCH_3$ | CH | |
| H | H | $CH_3$ | $CH_2CH_2SCH_3$ | $OCH_3$ | $OCH_3$ | CH | |
| H | H | $CH_3$ | $CH_2CH_2CH_2N(CH_3)_2$ | $OCH_3$ | $OCH_3$ | CH | |
| H | H | $CH_3$ | 4-picolyl | $OCH_3$ | $OCH_3$ | CH | |
| H | H | $C_2H_5$ | $CH_2CH_2CH_2Cl$ | $OCH_3$ | $CH_3$ | N | |
| H | H | n-$C_5H_{11}$ | $CH_2CH_2CH_2Cl$ | $OCH_3$ | $CH_3$ | N | |
| H | H | $CH_2CH=CHCH_3$ | $CHF_2$ | $OCH_3$ | $CH_3$ | N | |
| H | H | cyclopropyl | $CHF_2$ | $OCH_3$ | $CH_3$ | N | |
| H | H | cyclopentyl | $CHF_2$ | $OCH_3$ | $CH_3$ | N | |
| H | H | 4-$CH_3$-cyclohexyl | $CHF_2$ | $OCH_3$ | $CH_3$ | N | |
| H | H | $CH_2$-cyclobutyl | $CH_2CH_2Br$ | $OCH_3$ | $CH_3$ | N | |
| H | H | 1-cyclohexen-1-yl | $CH_2CH_2Br$ | $OCH_3$ | $CH_3$ | N | |
| H | H | $CH_2CH_2Cl$ | $CH_2CH_2Br$ | $OCH_3$ | $CH_3$ | N | |
| H | H | $CH_2CH_2Cl$ | $CH_2CH_2Br$ | $OCH_3$ | $OCH_3$ | CH | |
| H | H | $CH_2CH_2CH_2CH_2Br$ | $CH_2CH_2Br$ | $CH_3$ | $OCH_3$ | N | |
| H | H | $CH_2CH_2CH_2CH_2Br$ | $CH_2CH_2Br$ | $OCH_3$ | $OCH_3$ | CH | |
| H | H | $CH_2CF_3$ | $CHF_2$ | $OCH_3$ | $OCH_3$ | CH | |
| H | H | $CH_2CF_3$ | $CH_2CH_2F$ | $OCH_3$ | $OCH_3$ | CH | |
| H | H | $CH_2CF_3$ | $CH_2CH_2F$ | $CH_3$ | $OCH_3$ | N | |
| H | H | $CH_2CF_3$ | $CH_2CF_3$ | $OCH_3$ | $OCH_3$ | CH | |
| H | H | $CH_2CF_3$ | $CH_2CF_3$ | $OCH_3$ | $CH_3$ | N | |
| H | H | $CH_2CF_3$ | $CH_2CH_2Br$ | $OCH_3$ | $OCH_3$ | CH | |
| H | H | $CH_2CF_3$ | $CH_2CH_2Br$ | $CH_3$ | $OCH_3$ | N | |
| H | H | $OCH_2CH_3$ | $CH_2CH_2Br$ | $OCH_3$ | $OCH_3$ | N | |
| H | H | $OCH_2CH_3$ | $CH_2CH_2Br$ | $OCH_3$ | $OCH_3$ | CH | |
| H | H | $OCH_2CH_2CH_2CH_3$ | $CH_2CH_2Br$ | Cl | $OCH_3$ | CH | |

Table II (continued)

| R | $R_1$ | $R_3$ | $R_4$ | X | Y | Z | m.p. (°C) |
|---|---|---|---|---|---|---|---|
| H | H | $OCH_2CH_2CH_2CH_3$ | $CH_2CH_2Br$ | $CH_3$ | $OCH_3$ | N | |
| H | H | $CH_2$-oxirane | $CH_2CH_2Br$ | $CH_3$ | $OCH_3$ | N | |
| H | H | $CH_2$-oxirane | $CH_2CH_2Br$ | $OC_2H_5$ | $OCH_3$ | CH | |
| H | H | $CH_2CH_2OC(O)N(CH_3)_2$ | $CH_2CH_2Br$ | $OCH_3$ | C≡CH | N | |
| H | H | $CH_2CH_2OC(O)N(CH_3)_2$ | $CH_2CH_2Br$ | Br | $OCH_3$ | CH | |
| H | H | $CH_2CH_2SOCH_3$ | $CH_2CH_2Br$ | $CF_3$ | $CF_3$ | CH | |
| H | H | $CH_2CH_2SOCH_3$ | $CH_2CH_2Br$ | $OCH_3$ | $OCH_3$ | N | |
| H | H | $CH_2CH_2CH_2N(C_2H_5)_2$ | $CH_2CH_2Br$ | $OCH_3$ | $OCH_3$ | CH | |
| H | H | $CH_2CH_2CH_2N(C_2H_5)_2$ | $CH_2CH_2Br$ | $CH_3$ | $OCH_3$ | N | |
| H | H | $CF_2H$ | $CH_2C≡CH$ | $OCH_3$ | $OCH_3$ | CH | |
| H | H | $CH_2CH_2F$ | $CH_2C≡CH$ | $OCH_3$ | $OCH_3$ | CH | |
| H | H | $CH_2CH_2F$ | $CH_2C≡CH$ | $CH_3$ | $OCH_3$ | N | |
| H | H | $CH_2CH_2Br$ | $CH_2C≡CH$ | $OCH_3$ | $OCH_3$ | CH | |
| H | H | $CH_2CH_2Br$ | $CH_2C≡CH$ | $CH_3$ | $OCH_3$ | N | |
| H | H | $CH_2C≡CH$ | $CH_2C≡CH$ | $OCH_3$ | $OCH_3$ | CH | |
| H | H | $CH_2C≡CH$ | $CH_2C≡CH$ | Cl | $OCH_3$ | CH | |
| H | H | $CH_2C≡CH$ | $CH_2C≡CH$ | $CH_3$ | $OCH_3$ | CH | |
| H | H | $CH_2C≡CH$ | $CH_2C≡CH$ | $CH_3$ | $CH_3$ | CH | |
| H | H | $CH_2C≡CH$ | $CH_2C≡CH$ | $CH_3$ | $CH_3$ | N | |
| H | H | $CH_2C≡CH$ | $CH_2C≡CH$ | $CH_3$ | $OCH_3$ | N | |
| H | H | $CH_2C≡CH$ | $CH_2C≡CH$ | $OCH_3$ | $OCH_3$ | N | |
| H | H | $CH_3$ | CN | $OCH_3$ | $OCH_3$ | N | |
| H | H | $CH_3$ | CN | $OCH_3$ | $CH_3$ | N | |
| H | H | $CH_3$ | CN | $CH_3$ | $CH_3$ | CH | |
| H | H | $CH_3$ | CN | $CH_3$ | $OCH_3$ | CH | |
| H | H | $CH_3$ | CN | Cl | $OCH_3$ | CH | |
| H | H | $CH_3$ | CN | $OCH_3$ | $OCH_3$ | CH | |
| H | 4-Cl | $CH_3$ | $CH_2CH_2F$ | $OCH_3$ | $OCH_3$ | CH | |
| H | 5-Cl | $CH_3$ | $CH_2CH_2F$ | $OCH_3$ | $OCH_3$ | CH | |
| H | 6-Cl | $CH_3$ | $CH_2CH_2F$ | $OCH_3$ | $OCH_3$ | CH | |
| H | 5-$OCH_3$ | $CH_3$ | $CH_2CH_2F$ | $OCH_3$ | $OCH_3$ | CH | |

Table II (continued)

| R | $R_1$ | $R_3$ | $R_4$ | X | Y | Z | m.p.(°C) |
|---|---|---|---|---|---|---|---|
| H | 6-$OCH_3$ | $CH_3$ | $CH_2CH_2F$ | $OCH_3$ | $OCH_3$ | CH | |
| H | 6-$SCH_3$ | $CH_3$ | $CH_2CH_2F$ | $OCH_3$ | $OCH_3$ | CH | |
| H | 6-$SO_2CH_3$ | $CH_3$ | $CH_2CH_2F$ | $OCH_3$ | $OCH_3$ | CH | |
| H | 6-$SO_2CH_2CH_2CH_3$ | $CH_3$ | $CH_2CH_2F$ | $OCH_3$ | $OCH_3$ | CH | |
| H | 6-$CH_3$ | $CH_3$ | $CH_2CH_2F$ | $OCH_3$ | $OCH_3$ | CH | |
| H | 6-$SO_2NHCH_3$ | $CH_3$ | $CH_2CH_2F$ | $OCH_3$ | $OCH_3$ | CH | |
| H | 5-CN | $CH_3$ | $CH_2CH_2F$ | $OCH_3$ | $OCH_3$ | CH | |
| $CH_3$ | H | $CH_3$ | $CH_2CH_2F$ | $OCH_3$ | $OCH_3$ | CH | |
| $CH_3$ | H | $CH_3$ | $CH_2CH_2Cl$ | $OCH_3$ | $OCH_3$ | CH | |
| $CH_3$ | H | $CH_3$ | $CH_2$-oxirane | $OCH_3$ | $OCH_3$ | CH | |
| $CH_3$ | H | $CH_2C\equiv CH$ | $CH_2C\equiv CH$ | $OCH_3$ | $OCH_3$ | CH | |
| $CH_3$ | H | $CH_2CF_3$ | $CH_2CF_3$ | $OCH_3$ | $OCH_3$ | CH | |
| H | H | $CH_3$ | 2-Pyridyl | $OCH_3$ | $OCH_3$ | CH | |
| H | H | $CH_3$ | 2-Pyridyl | $CH_3$ | $OCH_3$ | N | |
| H | H | $CH_3$ | $CH_2CH_2$-Pyridin-2-yl | $OCH_3$ | $OCH_3$ | CH | |
| H | H | $CH_3$ | $CH_2CH_2$-Pyridin-2-yl | $CH_3$ | $OCH_3$ | N | |
| H | H | $CH_3$ | Thiazol-2-yl | $OCH_3$ | $OCH_3$ | CH | |
| H | H | $CH_3$ | Thiazol-2-yl | $CH_3$ | $OCH_3$ | N | |
| H | H | $-CH_2CH_2CH_2CH(CH_3)CH_2-$ | | $CH_3$ | $OCH_3$ | CH | |
| H | H | $-CH_2CH_2CH_2CH(CH_3)CH_2-$ | | $CH_3$ | $OCH_3$ | N | |
| H | H | $-CH_2CH_2CH_2CH(CH_3)CH_2-$ | | $OCH_3$ | $OCH_3$ | CH | |
| H | H | $-CH_2CH(Cl)CH(Cl)CH_2-$ | | $CH_3$ | $CH_3$ | CH | |
| H | H | $-CH_2CH(Cl)CH(Cl)CH_2-$ | | $CH_3$ | $OCH_3$ | CH | |
| H | H | $-CH_2CH(Cl)CH(Cl)CH_2-$ | | $OCH_3$ | $OCH_3$ | N | |
| H | H | $-CH_2CH(Cl)CH(Cl)CH_2-$ | | Cl | $OCH_3$ | CH | |
| H | H | $-CH_2CH=CHCH_2-$ | | Cl | $OCH_3$ | CH | 210-217(d) |
| H | H | $-CH_2CH=CHCH_2-$ | | $CH_3$ | $OCH_3$ | CH | |
| H | H | $-CH_2CH=CHCH_2-$ | | $OCH_3$ | $OCH_3$ | CH | 220-230(d) |
| H | H | $-CH_2CH=CHCH_2-$ | | $CH_3$ | $OCH_2CH_2CH_3$ | CH | 193-196(d) |
| H | H | $-CH_2CH=CHCH_2-$ | | $CH_3$ | $OCH(CH_3)_2$ | CH | 173-179(d) |

Table II (continued)

| R | R1 | R3 | R4 | X | Y | Z | m.p.(°C) |
|---|---|---|---|---|---|---|---|
| H | H | $-CH_2CH(Br)CH(Br)CH_2-$ | | $CH_3$ | $OCH_3$ | CH | |
| H | H | $-CH_2CH(Br)CH(Br)CH_2-$ | | $OCH_3$ | $OCH_3$ | CH | |
| H | $3-CH_3$ | $CH_3$ | $CH_2CH_2F$ | $CH_3$ | $CH_3$ | CH | 175-176 |
| H | $3-CH_3$ | $CH_3$ | $CH_2CH_2F$ | $CH_3$ | $OCH_3$ | CH | 170-171 |
| H | $3-CH_3$ | $CH_3$ | $CH_2CH_2F$ | $OCH_3$ | $OCH_3$ | CH | 212-214 |
| H | $3-CH_3$ | $CH_3$ | $CH_2CH_2F$ | Cl | $OCH_3$ | CH | 172-173 |
| H | $3-CH_3$ | $CH_3$ | $CH_2CH_2F$ | $CH_3$ | $OCH_3$ | N | 177-178 |
| H | $3-CH_3$ | $CH_3$ | $CH_2CH_2F$ | $OCH_3$ | $OCH_3$ | N | 182-184 |
| H | $3-CH_3$ | $CH_3$ | $CH_2CH_2F$ | $CH_3$ | $CH_3$ | N | 178-179 |
| H | $3-CH_3$ | $CH_3$ | $CH_2CH_2CH_2F$ | $CH_3$ | $CH_3$ | CH | 152-153 |
| H | $3-CH_3$ | $CH_3$ | $CH_2CH_2CH_2F$ | $CH_3$ | $OCH_3$ | CH | 127-128 |
| H | $3-CH_3$ | $CH_3$ | $CH_2CH_2CH_2F$ | $OCH_3$ | $OCH_3$ | CH | 188-189 |
| H | $3-CH_3$ | $CH_3$ | $CH_2CH_2CH_2F$ | Cl | $OCH_3$ | CH | 155.5-156.5 |
| H | $3-CH_3$ | $CH_3$ | $CH_2CH_2CH_2F$ | $CH_3$ | $OCH_3$ | N | 155-156 |
| H | $3-CH_3$ | $CH_3$ | $CH_2CH_2CH_2F$ | $OCH_3$ | $OCH_3$ | N | 150-151 |
| H | $3-CH_3$ | $CH_3$ | $CH_2CH_2CH_2F$ | $CH_3$ | $CH_3$ | N | 146-148 |

**Table IIa .**

**Structure III, W = S**

| R | $R_1$ | $R_3$ | $R_4$ | X | Y | Z | m.p.(°C) |
|---|---|---|---|---|---|---|---|
| H | H | $CH_3$ | $CH_2CH_2F$ | $CH_3$ | $OCH_3$ | N | |
| H | H | $CF_2H$ | $CH_2C{\equiv}CH$ | $OCH_3$ | $OCH_3$ | CH | |
| H | H | $CH_3$ | $CH_2CH_2F$ | $CH_3$ | $OCH_3$ | CH | |
| H | H | $CH_3$ | $CH_2CH_2Cl$ | $OCH_3$ | $OCH_3$ | CH | |
| H | H | $CF_2H$ | $CH_2CH_2F$ | $OCH_3$ | $OCH_3$ | CH | |
| H | H | $CH_3$ | $CH_2CH_2F$ | $CH_3$ | $OCH_3$ | N | |
| H | H | $CH_3$ | $CH_2CH_2F$ | Cl | $OCH_3$ | CH | |
| H | H | $CH_2CF_3$ | $CH_2CF_3$ | $OCH_3$ | $OCH_3$ | CH | |
| H | H | $CH_2CH_2F$ | $CH_2CH_2F$ | $CH_3$ | $OCH_3$ | CH | |
| H | H | $CH_3$ | $CH_2CH_2Cl$ | $OCH_3$ | $OCH_3$ | CH | |
| H | H | $CH_3$ | $CH_2CH_2F$ | $CH_3$ | $OCH_3$ | N | |
| H | H | $CH_3$ | $CH_2CH_2F$ | $OCH_3$ | $OCH_3$ | CH | |
| H | H | $CH_3$ | $CH_2CH_2F$ | $CH_3$ | $OCH_3$ | CH | |
| H | H | $CH_3$ | $CH_2CH_2F$ | $OCH_3$ | $OCH_3$ | CH | |
| H | H | $CH_3$ | $CH_2CH_2F$ | $OCH_3$ | $OCH_3$ | CH | |

**Table III**

**Structure V, W=O**

| R | $R_1$ | $R'_2$ | $R'_3$ | $R'_4$ | X | Y | Z | m.p. (°C) |
|---|---|---|---|---|---|---|---|---|
| H | H | H | H | H | $CH_3$ | $CH_3$ | CH | |
| H | H | H | H | H | $OCH_3$ | $OCH_3$ | CH | |
| H | H | H | H | H | $OCH_3$ | $CH_3$ | N | |
| H | H | H | H | $CH_3$ | $OCH_3$ | $CH_3$ | N | |
| H | H | H | H | $CH_3$ | $OCH_3$ | $OCH_3$ | N | |
| H | H | H | H | $CH_3$ | $OCH_3$ | $OCH_3$ | CH | |
| H | H | H | H | $CH_3$ | Cl | $OCH_3$ | CH | |
| H | H | H | H | $CH_3$ | $CH_3$ | $OCH_3$ | CH | |
| H | H | H | H | $C_2H_5$ | $CH_3$ | $OCH_3$ | CH | |
| H | H | H | H | $C_2H_5$ | $OCH_3$ | $OCH_3$ | CH | |
| H | H | H | H | $C_2H_5$ | $CH_3$ | $OCH_3$ | N | |
| H | H | H | H | iso-$C_3H_7$ | $CH_3$ | $OCH_3$ | N | |
| H | H | H | H | iso-$C_3H_7$ | $OCH_3$ | $OCH_3$ | CH | |
| H | H | H | H | n-$C_4H_7$ | $OCH_3$ | $OCH_3$ | CH | |
| H | H | H | H | n-$C_4H_7$ | $OCH_3$ | $CH_3$ | N | |
| H | H | H | H | $CH_2CH=CH_2$ | $OCH_3$ | $CH_3$ | N | |
| H | H | H | H | $CH_2CH=CH_2$ | $OCH_3$ | $CH_3$ | CH | |
| H | H | H | H | $CH_2C\equiv CCH_3$ | $OCH_3$ | $CH_3$ | CH | |
| H | H | H | H | $CH_2C\equiv CCH_3$ | $CH_3$ | $OCH_3$ | N | |
| H | H | H | H | $CH_2CF_3$ | $CH_3$ | $OCH_3$ | N | |
| H | H | H | H | $CH_2CF_3$ | $OCH_3$ | $OCH_3$ | CH | |
| H | H | H | H | $CH_2CH_2CH_2CH_2Cl$ | $OCH_3$ | $OCH_3$ | CH | |
| H | H | H | H | $CH_2CH_2CH_2CH_2Cl$ | $OCH_3$ | $CH_3$ | N | |
| H | H | H | $C_6H_5$ | H | $OCH_3$ | $CH_3$ | N | |
| H | H | H | $C_6H_5$ | H | $OCH_3$ | $OCH_3$ | CH | |
| H | H | H | $C_6H_5$ | H | Cl | $OCH_3$ | CH | |
| H | H | H | $C_6H_5$ | H | $CH_3$ | $OCH_3$ | CH | |
| H | H | H | $C_6H_5$ | H | $N(CH_3)_2$ | $OCH_3$ | CH | |
| H | H | H | $C_6H_5$ | H | $CH_3$ | $C\equiv CH$ | CH | |
| H | H | H | 2-F-$C_6H_5$ | H | $CH_3$ | $OCH_3$ | CH | |
| H | H | H | 2-F-$C_6H_4$ | H | $OCH_3$ | $OCH_3$ | CH | |
| H | H | H | 3-F-$C_6H_4$ | H | $OCH_3$ | $OCH_3$ | CH | |

Table III (continued)

| R | $R_1$ | $R'_2$ | $R'_3$ | $R'_4$ | X | Y | Z | m.p. (°C) |
|---|---|---|---|---|---|---|---|---|
| H | H | H | $4\text{-}FC_6H_4$ | H | $OCH_3$ | $OCH_3$ | CH | |
| H | H | H | $2\text{-}ClC_6H_4$ | H | $OCH_3$ | $OCH_3$ | CH | |
| H | H | H | $4\text{-}ClC_6H_4$ | H | $OCH_3$ | $OCH_3$ | CH | |
| H | H | H | $3\text{-}BrC_6H_4$ | H | $OCH_3$ | $OCH_3$ | CH | |
| H | H | H | $4\text{-}BrC_6H_4$ | H | $OCH_3$ | $OCH_3$ | CH | |
| H | H | H | $2\text{-}CH_3C_6H_4$ | H | $OCH_3$ | $OCH_3$ | CH | |
| H | H | H | $3\text{-}NO_2C_6H_4$ | H | $OCH_3$ | $OCH_3$ | CH | |
| H | H | H | $4\text{-}CF_3C_6H_4$ | H | $OCH_3$ | $OCH_3$ | CH | |
| H | H | H | $4\text{-}CNC_6H_4$ | H | $OCH_3$ | $OCH_3$ | CH | |
| H | H | H | $3\text{-}CH_3OC_6H_4$ | H | $OCH_3$ | $OCH_3$ | CH | |
| H | H | H | $4\text{-}C_2H_5OC_6H_4$ | H | $OCH_3$ | $OCH_3$ | CH | |
| H | H | H | $4\text{-}Cl\text{-}2\text{-}CH_3C_6H_3$ | H | $OCH_3$ | $OCH_3$ | CH | |
| H | H | H | $2,4\text{-}diClC_6H_3$ | H | $OCH_3$ | $OCH_3$ | CH | |
| H | H | H | $4\text{-}isoC_3H_7C_6H_4$ | H | $OCH_3$ | $OCH_3$ | CH | |
| H | H | H | 4-pyridyl | H | $OCH_3$ | $OCH_3$ | CH | |
| H | H | H | $4\text{-}CH_3\text{-}6\text{-}CH_3O$ pyrimidin-2-yl | H | $OCH_3$ | $OCH_3$ | CH | |
| H | H | H | $5\text{-}CH_3$-1,3,4-oxadiazol-2-yl | H | $OCH_3$ | $OCH_3$ | CH | |
| H | H | H | 2-thienyl | H | $OCH_3$ | $OCH_3$ | CH | |
| H | H | H | tetrahydro-2-furanyl | H | $OCH_3$ | $OCH_3$ | CH | |
| H | H | H | $2\text{-}CH_2$-thienyl | H | $OCH_3$ | $OCH_3$ | CH | |
| H | H | H | $2\text{-}CH_2CH_2$tetrahydrofuranyl | H | $OCH_3$ | $OCH_3$ | CH | |
| H | H | $CH_3$ | H | H | $OCH_3$ | $OCH_3$ | CH | |
| H | H | $CH_3$ | H | H | $OCH_3$ | $CH_3$ | CH | |
| H | H | $CH_3$ | H | H | $OCH_3$ | $CH_3$ | N | |
| H | H | $COCH_3$ | H | H | $OCH_3$ | $CH_3$ | N | |
| H | H | $COCH_2CH_3$ | H | H | $OCH_3$ | $CH_3$ | N | |
| H | H | $COC_6H_5$ | H | H | $OCH_3$ | $CH_3$ | N | |
| H | H | $COC_6H_4\text{-}4\text{-}Cl$ | H | H | $OCH_3$ | $CH_3$ | N | |
| H | H | $CO_2C_2H_5$ | H | H | $OCH_3$ | $CH_3$ | N | |
| H | H | $CON(C_2H_5)_2$ | H | H | $OCH_3$ | $CH_3$ | N | |
| H | H | $CSN(CH_3)_2$ | H | H | $OCH_3$ | $CH_3$ | N | |
| H | H | $C_2H_5$ | $CH_3$ | H | $OCH_3$ | $CH_3$ | N | |

Table III (Continued)

| R | $R_1$ | $R'_2$ | $R'_3$ | $R'_4$ | X | Y | Z | m.p. (°C) |
|---|---|---|---|---|---|---|---|---|
| H | H | $C_2H_5$ | $CH_3$ | H | $OCH_3$ | $OCH_3$ | CH | |
| H | H | $C_2H_4OH$ | $CH_3$ | H | $OCH_3$ | $OCH_3$ | CH | |
| H | H | $C_2H_4OC(O)CH_3$ | $CH_3$ | H | $OCH_3$ | $OCH_3$ | CH | |
| H | H | $C_2H_4OP(O)(OCH_3)_2$ | $CH_3$ | H | $OCH_3$ | $OCH_3$ | CH | |
| H | H | $CH_3$ | $C_6H_5$ | H | $OCH_3$ | $OCH_3$ | CH | |
| H | H | $C_2H_5$ | $C_6H_5$ | H | $OCH_3$ | $OCH_3$ | CH | |
| H | H | $n-C_4H_9$ | $C_6H_5$ | H | $OCH_3$ | $OCH_3$ | CH | |
| H | H | $CH_2CH=CH_2$ | $C_6H_5$ | H | $OCH_3$ | $OCH_3$ | CH | |
| H | H | $CH_2CH_2F$ | $C_6H_5$ | H | $OCH_3$ | $OCH_3$ | CH | |
| H | H | $COCH_3$ | $C_6H_5$ | H | $OCH_3$ | $OCH_3$ | CH | |
| H | H | $COCH_2CH_3$ | $C_6H_5$ | H | $OCH_3$ | $OCH_3$ | CH | |
| H | H | $COCH_2CH_2CH_3$ | $C_6H_5$ | H | $OCH_3$ | $OCH_3$ | CH | |
| H | H | $CO_2C_2H_5$ | $C_6H_5$ | H | $OCH_3$ | $OCH_3$ | CH | |
| H | H | $CONHCH_2CH_3$ | $C_6H_5$ | H | $OCH_3$ | $OCH_3$ | CH | |
| H | H | $C(NH)N(CH_3)_2$ | $C_6H_5$ | H | $OCH_3$ | $OCH_3$ | CH | |
| H | H | $CH_2CH_2OH$ | $C_6H_5$ | H | $OCH_3$ | $OCH_3$ | CH | |
| H | H | $CH_2CH_2OSO_2CH_3$ | $C_6H_5$ | H | $OCH_3$ | $OCH_3$ | CH | |
| H | H | $CH_3$ | $CH_3$ | H | $OCH_3$ | $OCH_3$ | CH | |
| H | H | $CH_3$ | $CH_3$ | H | Cl | $OCH_3$ | CH | |
| H | H | $CH_3$ | $CH_3$ | H | $CH_3$ | $OCH_3$ | CH | |
| H | H | $CH_3$ | $CH_3$ | H | $CH_3$ | $CH_3$ | CH | |
| H | H | $CH_3$ | $CH_3$ | H | $CH_3$ | $CH_3$ | N | |
| H | H | $CH_3$ | $CH_3$ | H | $CH_3$ | $OCH_3$ | N | |
| H | H | $CH_3$ | $CH_3$ | H | $OCH_3$ | $OCH_3$ | N | |
| H | H | $CH_3$ | $CH_3$ | H | $OCH_3$ | $C\equiv CH$ | N | |
| H | H | $CH_3$ | $CH_3$ | H | $OC_2H_5$ | $C\equiv CH$ | CH | |

Table III (Continued)

| R | $R_1$ | $R'_2$ | $R'_3$ | $R'_4$ | X | Y | Z | m.p. (°C) |
|---|---|---|---|---|---|---|---|---|
| H | H | $CH_3$ | $CH_3$ | H | $CH_3$ | C≡CH | CH | |
| H | H | $CH_3$ | $CH_3$ | H | $N(CH_3)_2$ | C≡CH | CH | |
| H | H | $CH_3$ | $CH_3$ | H | Br | $OCH_3$ | CH | |
| H | H | $CH_3$ | $CH_3$ | H | $OCH_2CF_3$ | $OCH_3$ | CH | |
| H | H | $CH_3$ | $CH_3$ | H | $CF_3$ | $CF_3$ | CH | |
| H | H | $CH_3$ | $CH_3$ | H | $CH_3$ | $N(CH_3)_2$ | CH | |
| H | H | $CH_3$ | $CH_3$ | H | $CH_3$ | $NH(CH_3)_2$ | CH | |
| H | H | $CH_3$ | $CH_3$ | H | $CH_3$ | $CH_2OCH_3$ | CH | |
| H | H | $CH_3$ | $CH_3$ | H | $CH_3$ | $OC_2H_5$ | CH | |
| H | H | $CH_3$ | $CH_3$ | H | $CH_3$ | $CH_2SCH_3$ | CH | |
| H | H | $CH_3$ | $CH_3$ | H | $CH_3$ | $SCH_3$ | CH | |
| H | H | $CH_3$ | $CH_3$ | H | $CH_2Cl$ | $CH_3$ | CH | |
| H | H | $CH_3$ | $CH_3$ | H | $OCH_2CHF_2$ | $CH_3$ | CH | |
| H | H | $CH_3$ | $CH_3$ | H | $CH_3$ | $OCH_2CH_2OCH_3$ | CH | |
| H | H | $C_6H_5$ | $CH_3$ | H | $OCH_3$ | $OCH_3$ | CH | |
| H | H | H | $CH_3$ | $CH_3$ | I | $OCH_3$ | CH | |
| H | H | H | $CH_3$ | $CH_3$ | Cl | $OCH_3$ | CH | 115-116(d) |
| H | H | H | $CH_3$ | $CH_3$ | $OCH_2CH_2F$ | $OCH_3$ | CH | |
| H | H | H | $CH_3$ | $CH_3$ | $OCH_2CHF_2$ | $OCH_3$ | CH | |
| H | H | H | $CH_3$ | $CH_3$ | $OCH_2CF_3$ | $OCH_3$ | CH | |
| H | H | H | $CH_3$ | $CH_3$ | $OCH_3$ | $OCH_3$ | CH | 127-128(d) |
| H | H | H | $CH_3$ | $CH_3$ | $CH_3$ | $OCH_3$ | CH | 120-122(d) |
| H | H | H | $CH_3$ | $CH_3$ | $CH_3$ | $CH_3$ | CH | 125-127(d) |
| H | H | H | $CH_3$ | $CH_3$ | $CH_3$ | $CF_3$ | CH | |
| H | H | H | $CH_3$ | $CH_3$ | $CH_3$ | H | CH | |

Table III (Continued)

| R | $R_1$ | $R'_2$ | $R'_3$ | $R'_4$ | X | Y | Z | m.p. (°C) |
|---|---|---|---|---|---|---|---|---|
| H | H | H | $CH_3$ | $CH_3$ | $CH_3$ | n-$C_3H_7$ | CH | |
| H | H | H | $CH_3$ | $CH_3$ | $CH_3$ | $OC_2H_5$ | CH | |
| H | H | H | $CH_3$ | $CH_3$ | $CH_3$ | $CH_2OCH_3$ | CH | |
| H | H | H | $CH_3$ | $CH_3$ | $CH_3$ | $NHCH_3$ | CH | |
| H | H | H | $CH_3$ | $CH_3$ | $CH_3$ | $N(OCH_3)CH_3$ | CH | |
| H | H | H | $CH_3$ | $CH_3$ | $CH_3$ | $N(CH_3)_2$ | CH | |
| H | H | H | $CH_3$ | $CH_3$ | $CH_3$ | $SCH_3$ | CH | |
| H | H | H | $CH_3$ | $CH_3$ | $CH_3$ | $OCH_2C{\equiv}CH$ | CH | |
| H | H | H | $CH_3$ | $CH_3$ | $CH_3$ | cyclopropyl | CH | |
| H | H | H | $CH_3$ | $CH_3$ | $CH_3$ | $C{\equiv}CH$ | CH | |
| H | H | H | $CH_3$ | $CH_3$ | $CH_3$ | $C{\equiv}CH$ | N | |
| H | H | H | $CH_3$ | $CH_3$ | $CH_3$ | $CH_3$ | N | |
| H | H | H | $CH_3$ | $CH_3$ | $OCH_3$ | $CH_3$ | N | 123-124(d) |
| H | H | H | $CH_3$ | $CH_3$ | $OCH_3$ | $OCH_3$ | N | 132-133(d) |
| H | H | H | $CH_3$ | $CH_3$ | $OC_2H_5$ | $OCH_3$ | N | |
| H | H | H | $CH_3$ | $CH_3$ | $NHCH_3$ | $NHCH_3$ | N | |
| H | H | H | $CH_3$ | $CH_3$ | $CF_3$ | $CF_3$ | CH | |
| H | H | H | $CH_3$ | $CH_3$ | $CF_3$ | $CH_3$ | CH | |
| H | H | H | $CH_3$ | $CH_3$ | $CH_3$ | $C_2H_5$ | CH | |
| H | H | H | $CH_3$ | $CH_3$ | $CH_3$ | n-$C_3H_7$ | CH | |
| H | H | H | $CH_3$ | $CH_3$ | $CH_3$ | $SCH_3$ | CH | |
| H | H | H | $CH_3$ | $CH_3$ | $CH_3$ | $OCH_2CH{=}CH_2$ | CH | |
| H | H | H | $CH_3$ | $CH_3$ | $CH_3$ | $CH_2OCH_2CH_3$ | N | |
| H | H | H | $CH_3$ | $CH_3$ | $CH_3$ | $OCH_2CH_2OCH_3$ | N | |
| H | H | H | $CH_3$ | $CH_3$ | $CH_3$ | $CH_2SCH_3$ | N | |
| H | H | H | $CH_3$ | $CH_3$ | $CH_3$ | $NH_2$ | N | |
| H | H | H | $CH_3$ | $CH_3$ | $CH_3$ | $OCH_2CF_3$ | N | |
| H | H | H | $CH_3$ | $CH_3$ | $CH_3$ | $SCH_2F$ | N | |
| H | H | H | $CH_3$ | $CH_3$ | $CH_3$ | $OCH_2C{\equiv}CH$ | N | |
| H | H | H | $CH_3$ | $CH_3$ | $CH_3$ | $COCH_3$ | CH | |

Table III (Continued)

| R | $R_1$ | $R'_2$ | $R'_3$ | $R'_4$ | X | Y | Z | m.p. (°C) |
|---|---|---|---|---|---|---|---|---|
| H | H | H | $CH_3$ | $CH_3$ | $CH_3$ | $SCF_2H$ | CH | |
| H | H | H | $CH_3$ | $CH_3$ | $CH_3$ | $C{\equiv}CCH_3$ | CH | |
| H | H | H | $CH_3$ | $CH_2CH_2CH_3$ | $CH_3$ | $OCH_3$ | N | |
| H | H | H | $CH_3$ | $CH{=}CH_2$ | $CH_3$ | $OCH_3$ | N | |
| H | H | H | $CH_3$ | $C_6H_5$ | $CH_3$ | $OCH_3$ | N | |
| H | H | H | $-CH_2CH_2CH_2CH_2-$ | | $CH_3$ | $OCH_3$ | N | |
| H | H | H | $-CH_2CH_2CH_2CH_2CH_2-$ | | $CH_3$ | $OCH_3$ | N | |
| H | H | H | $-CH_2CH_2OCH_2CH_2-$ | | $CH_3$ | $OCH_3$ | N | |
| H | H | H | $-CH{=}CH-CH{=}CH-$ | | $CH_3$ | $OCH_3$ | N | |
| H | H | H | $-CH{=}NN{=}CH-$ | | $CH_3$ | $OCH_3$ | N | |
| H | H | H | $=CHCH_3$ | | $CH_3$ | $OCH_3$ | N | |
| H | H | H | $=CHC_6H_5$ | | $CH_3$ | $OCH_3$ | N | |
| H | H | H | $=C(CH_3)_2$ | | $CH_3$ | $OCH_3$ | N | |
| H | H | H | $=C(CH_3)CH_2C_6H_5$ | | $CH_3$ | $OCH_3$ | N | |
| H | H | H | $=C(C_2H_5)C_6H_5$ | | $CH_3$ | $OCH_3$ | N | |
| H | H | H | $=C(C_2H_5)C_6H_4-4-CH_3O$ | | $CH_3$ | $OCH_3$ | N | |
| H | H | $COCH_3$ | $-CH_2CH_2CH_2CH_2-$ | | $CH_3$ | $OCH_3$ | N | |
| H | H | $COCH_3$ | $CH_3$ | $CH_3$ | $CH_3$ | $OCH_3$ | N | |
| H | H | $COCH_3$ | $CH_3$ | $C_6H_5$ | $CH_3$ | $OCH_3$ | N | |
| H | H | $COCH_3$ | $CH_3$ | $C_6H_4-3-Cl$ | $CH_3$ | $OCH_3$ | N | |
| H | H | $CH_3$ | $CH_3$ | $CH_3$ | $CH_3$ | $OCH_3$ | CH | |
| H | H | $C_2H_5$ | $CH_3$ | $CH_3$ | $CH_3$ | $OCH_3$ | CH | |
| H | H | $CH_3$ | $CH_3$ | $C_6H_5$ | $CH_3$ | $OCH_3$ | CH | |

Table III (Continued)

| R | $R_1$ | $R'_2$ | $R'_3$ | $R'_4$ | X | Y | Z | m.p. (°C) |
|---|---|---|---|---|---|---|---|---|
| H | 3-$CH_3$ | H | $CH_3$ | $CH_3$ | $OCH_3$ | $OCH_3$ | CH | |
| H | 4-$C_2H_5$ | H | $CH_3$ | $CH_3$ | $OCH_3$ | $OCH_3$ | CH | |
| H | 5-iso-$C_3H_7$ | H | $CH_3$ | $CH_3$ | $OCH_3$ | $OCH_3$ | CH | |
| H | 5-$CF_3$ | H | $CH_3$ | $CH_3$ | $OCH_3$ | $OCH_3$ | CH | |
| H | 4-F | H | $CH_3$ | $CH_3$ | $OCH_3$ | $OCH_3$ | CH | |
| H | 4-Cl | H | $CH_3$ | $CH_3$ | $OCH_3$ | $OCH_3$ | CH | |
| H | 4-Br | H | $CH_3$ | $CH_3$ | $OCH_3$ | $OCH_3$ | CH | |
| H | 4-$CH_3O$ | H | $CH_3$ | $CH_3$ | $OCH_3$ | $OCH_3$ | CH | |
| H | 5-F | H | $CH_3$ | $CH_3$ | $OCH_3$ | $OCH_3$ | CH | |
| H | 5-Cl | H | $CH_3$ | $CH_3$ | $OCH_3$ | $OCH_3$ | CH | |
| H | 5-Br | H | $CH_3$ | $CH_3$ | $OCH_3$ | $OCH_3$ | CH | |
| H | 5-I | H | $CH_3$ | $CH_3$ | $OCH_3$ | $OCH_3$ | CH | |
| H | 5-$NO_2$ | H | $CH_3$ | $CH_3$ | $OCH_3$ | $OCH_3$ | CH | |
| H | 5-$CH_3O$ | H | $CH_3$ | $CH_3$ | $OCH_3$ | $OCH_3$ | CH | |
| H | 5-$C_2H_5O$ | H | $CH_3$ | $CH_3$ | $OCH_3$ | $OCH_3$ | CH | |
| H | 5-$C_3H_7O$ | H | $CH_3$ | $CH_3$ | $OCH_3$ | $OCH_3$ | CH | |
| H | 5-$SCH_3$ | H | $CH_3$ | $CH_3$ | $OCH_3$ | $OCH_3$ | CH | |
| H | 5-$SOCH_3$ | H | $CH_3$ | $CH_3$ | $OCH_3$ | $OCH_3$ | CH | |
| H | 5-$SO_2CH_3CH_2CH_3$ | H | $CH_3$ | $CH_3$ | $OCH_3$ | $OCH_3$ | CH | |
| H | 5-$SO_2N(CH_3)_2$ | H | $CH_3$ | $CH_3$ | $OCH_3$ | $OCH_3$ | CH | |
| H | 5-CN | H | $CH_3$ | $CH_3$ | $OCH_3$ | $OCH_3$ | CH | |
| H | 5-$CO_2CH_2CH_3$ | H | $CH_3$ | $CH_3$ | $OCH_3$ | $OCH_3$ | CH | |
| H | 5-$CO_2CH_2CH_3I$ | H | $CH_3$ | $CH_3$ | $OCH_3$ | $OCH_3$ | CH | |
| H | 5-$CO_2CH_2CH_2CN$ | H | $CH_3$ | $CH_3$ | $OCH_3$ | $OCH_3$ | CH | |
| H | 5-$CO_2$cyclohexyl | H | $CH_3$ | $CH_3$ | $OCH_3$ | $OCH_3$ | CH | |
| H | 5-$OCH_2CF_3$ | H | $CH_3$ | $CH_3$ | $OCH_3$ | $OCH_3$ | CH | |
| H | 5-$SCF_2CF_3$ | H | $CH_3$ | $CH_3$ | $OCH_3$ | $OCH_3$ | CH | |
| H | 6-Cl | H | $CH_3$ | $CH_3$ | $OCH_3$ | $OCH_3$ | CH | |
| H | 6-Br | H | $CH_3$ | $CH_3$ | $OCH_3$ | $OCH_3$ | CH | |
| H | 6-$CH_3$ | H | $CH_3$ | $CH_3$ | $OCH_3$ | $OCH_3$ | CH | |
| H | 6-$CH_3O$ | H | $CH_3$ | $CH_3$ | $OCH_3$ | $OCH_3$ | CH | |

Table III (Continued)

| R | $R_1$ | $R'_2$ | $R'_3$ | $R'_4$ | X | Y | Z | m.p. (°C) |
|---|---|---|---|---|---|---|---|---|
| H | 6-$SO_2CH_3$ | H | $CH_3$ | $CH_3$ | $OCH_3$ | $OCH_3$ | CH | |
| H | 6-$SO_2CH_2CH_2CH_3$ | H | $CH_3$ | $CH_3$ | $OCH_3$ | $OCH_3$ | CH | |
| H | 6-$CO_2CH_3$ | H | $CH_3$ | $CH_3$ | $OCH_3$ | $OCH_3$ | CH | |
| H | 6-$SO_2N(CH_3)OCH_3$ | H | $CH_3$ | $CH_3$ | $OCH_3$ | $OCH_3$ | CH | |
| H | 6-$SO_2N(CH_3)_2$ | H | $CH_3$ | $CH_3$ | $OCH_3$ | $OCH_3$ | CH | |
| H | 6-$SO_2N$-pyrrolidino | H | $CH_3$ | $CH_3$ | $OCH_3$ | $OCH_3$ | CH | |
| H | 6-$SO_2N$-morpholino | H | $CH_3$ | $CH_3$ | $OCH_3$ | $CH_3$ | CH | |
| H | 6-$SO_2N$-morpholino | H | $CH_3$ | $CH_3$ | $OCH_3$ | $OCH_3$ | CH | |
| $CH_3$ | H | H | $CH_3$ | $CH_3$ | $OCH_3$ | $OCH_3$ | CH | |
| $CH_3$ | H | H | $CH_3$ | $CH_3$ | $CH_3$ | $OCH_3$ | CH | |
| $CH_3$ | H | H | $CH_3$ | $CH_3$ | Cl | $OCH_3$ | CH | |
| $CH_3$ | H | H | $CH_3$ | $CH_3$ | $CH_3$ | $CH_3$ | CH | |
| $CH_3$ | H | H | $CH_3$ | $CH_3$ | $CH_3$ | $CH_3$ | N | |
| $CH_3$ | H | H | $CH_3$ | $CH_3$ | $CH_3$ | $OCH_3$ | N | |
| $CH_3$ | H | H | $CH_3$ | $CH_3$ | $OCH_3$ | $OCH_3$ | N | |
| $CH_3$ | H | H | $CH_3$ | $CH_3$ | $OCH_3$ | $CH_3$ | N | |
| $CH_3$ | 6-Cl | H | $CH_3$ | $CH_3$ | $OCH_3$ | $CH_3$ | N | |
| $CH_3$ | H | $COCH_3$ | $CH_3$ | $CH_3$ | $OCH_3$ | $CH_3$ | N | |
| $CH_3$ | H | $COCH_3$ | $C_6H_5$ | $C_6H_5$ | $OCH_3$ | $CH_3$ | N | |
| $CH_3$ | H | $CH_3$ | $CH_3$ | $CH_3$ | $OCH_3$ | $CH_3$ | N | |
| $CH_3$ | H | H | $-CH_2CH_2OCH_2CH_2-$ | | $OCH_3$ | $CH_3$ | N | |
| $CH_3$ | H | H | $-CH_2CH_2CH_2CH_2-$ | | $OCH_3$ | $CH_3$ | N | |
| $CH_3$ | H | H | $-CH_3$ | $CH_2CH=CH_2$ | $OCH_3$ | $CH_3$ | CH | |
| $CH_3$ | H | H | 2-Cl-$C_6H_4$ | H | $OCH_3$ | $CH_3$ | CH | |
| $CH_3$ | H | H | $C_6H_5$ | H | $OCH_3$ | $CH_3$ | CH | |
| $CH_3$ | H | H | $C_2H_5$ | H | $OCH_3$ | $CH_3$ | CH | |
| $CH_3$ | H | H | $CH_2CF_3$ | H | $OCH_3$ | $CH_3$ | CH | |
| $CH_3$ | H | $CH_3$ | $CH_3$ | H | $OCH_3$ | $CH_3$ | CH | |
| $CH_3$ | H | $CH_3$ | $C_2H_5$ | H | $OCH_3$ | $CH_3$ | CH | |
| $CH_3$ | H | $CH_3$ | $C_6H_5$ | H | $OCH_3$ | $CH_3$ | CH | |

Table III (Continued)

| R | $R_1$ | $R'_2$ | $R'_3$ | $R'_4$ | X | Y | Z | m.p. (°C) |
|---|---|---|---|---|---|---|---|---|
| H | $5-CH_2OCH_3$ | H | $C_6H_5$ | H | $OCH_3$ | $OCH_3$ | CH | |
| H | $5-CH_2OCH_2CH_2Cl$ | H | $2-Cl-C_6H_4$ | H | $OCH_3$ | $CH_3$ | CH | |
| H | $5-CH_2OCH_2CF_3$ | H | $CH_3$ | H | $OCH_3$ | $OCH_3$ | CH | |
| H | $5-CH_2CH_2SCH_3$ | H | H | H | $OCH_3$ | $CH_3$ | CH | |
| H | $5-CH_2SCH_2CH_2F$ | H | $CH_3$ | $CH_3$ | $OCH_3$ | $OCH_3$ | CH | |
| H | $5-CH_2CN$ | H | $CH_3$ | $CH_3$ | $OCH_3$ | $CH_3$ | CH | |
| H | $5-CH_2CH_2CN$ | H | $CH_3$ | $CH_3$ | $OCH_3$ | $OCH_3$ | N | |
| $CH_3$ | H | $CH_3$ | H | H | $OCH_3$ | $CH_3$ | CH | |
| $CH_3$ | H | $C_2H_5$ | H | H | $OCH_3$ | $CH_3$ | CH | |
| H | H | H | $C_6H_5$ | H | $OCH_3$ | $OCH_3$ | N | |
| H | H | H | $C_6H_5$ | H | $CH_3$ | $CH_3$ | CH | |
| H | H | $CH_3$ | $CH_3$ | $CH_3$ | $CH_3$ | $CH_3$ | CH | |
| H | H | $CH_3$ | $CH_3$ | $CH_3$ | $OCH_3$ | $OCH_3$ | CH | |
| H | H | $CH_3$ | $CH_3$ | $CH_3$ | Cl | $OCH_3$ | CH | |
| H | H | $CH_3$ | $CH_3$ | $CH_3$ | $OCH_3$ | $OCH_3$ | N | |
| H | $5-NH_2$ | H | $C_6H_5$ | H | $OCH_3$ | $OCH_3$ | CH | |
| H | $5-N(CH_3)_2$ | H | H | H | $OCH_3$ | $CH_3$ | CH | |
| H | $5-N(CH_3)CH_2CH_3$ | H | $CH_3$ | $CH_3$ | $OCH_3$ | $OCH_3$ | CH | |

Table IIIa

**Structure V, W=S**

| R | $R_1$ | $R'_2$ | $R'_3$ | $R'_4$ | X | Y | Z | m.p.(°C) |
|---|---|---|---|---|---|---|---|---|
| H | H | H | $CH_3$ | $CH_3$ | $CH_3$ | $OCH_3$ | CH | |
| H | H | H | $CH_3$ | $CH_3$ | $OCH_3$ | $OCH_3$ | CH | |
| H | H | H | $CH_3$ | $CH_3$ | $CH_3$ | $OCH_3$ | N | |
| H | H | $CH_3$ | $CH_3$ | $CH_3$ | $CH_3$ | $OCH_3$ | CH | |
| H | H | $CH_3$ | $CH_3$ | $CH_3$ | $OCH_3$ | $OCH_3$ | CH | |
| H | H | $CH_3$ | $CH_3$ | $CH_3$ | Cl | $OCH_3$ | CH | |
| H | H | $CH_3$ | $CH_3$ | $CH_3$ | $CH_3$ | $OCH_3$ | N | |
| H | H | H | $C_6H_5$ | H | $OCH_3$ | $OCH_3$ | CH | |
| H | H | H | $COC_6H_5$ | H | $OCH_3$ | $OCH_3$ | CH | |
| H | H | H | H | H | $OCH_3$ | $OCH_3$ | CH | |
| H | H | H | $CH_3$ | H | $OCH_3$ | $OCH_3$ | CH | |

Formulations

Useful herbicidal and plant growth regulant formulations of the compounds of Formula I can be prepared in conventional ways. They include e.g. dusts, granules, pellets, solutions, suspensions, emulsions, wettable powders and emulsifiable concentrates. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

**Table VII**

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0 °C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives e.g. to reduce foaming, caking, corrosion or microbiological growth.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering . December 4, 1967, pp. 147ff. and "Perry's Chemical Engineer's Handbook", 4th Ed., McGraw-Hill, New York, 1963, pp. 8-59ff.

In the following examples, all parts are by weight unless otherwise indicated.

## Example 5

**Wettable Powder**

| | |
|---|---|
| N-Cyclopropyl-N'-[(4,6-dimethoxypyrimidin-2-yl)aminocarbonyl]-1,2-benzenedisulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

## Example 6

**Granule**

| | |
|---|---|
| Wettable Powder of Example 5 | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules are dried and packaged.

## Example 7

**Extruded Pellet**

| | |
|---|---|
| N-Cyclopropyl-N'-[(4-methoxy-6-methyl-1,3,5-triazin-2-yl)aminocarbonyl]-1,2-benzenedisulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

## Example 8

**Low Strength Granule**

| | |
|---|---|
| N-Cyclopropyl-N'-[(4,6-dimethoxypyrimidin-2-yl)aminocarbonyl]-1,2-benzenedisulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for

a short period and then the granules are packaged.

## Example 9

**Aqueous Suspension**

| | |
|---|---|
| N-Cyclopropyl-N'-[(4-methoxy-6-methyl-1,3,5-triazin-2-yl)aminocarbonyl]-1,2-benzenedisulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

## Example 10

**Oil Suspension**

| | |
|---|---|
| N-Cyclopropyl-N'-[(4,6-dimethoxypyrimidin-2-yl)aminocarbonyl]-1,2-benzenedisulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

## Example 11

**Granule**

| | |
|---|---|
| N-Cyclopropyl-N'-[(4-methoxy-6-methyl-1,3,5-triazin-2-yl)aminocarbonyl]-1,2-benzenedisulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

## Example 12

**High Strength Concentrate**

| | |
|---|---|
| N-Cyclopropyl-N'-[(4,6-dimethoxypyrimidin-2-yl)aminocarbonyl]-1,2-benzenedisulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

## Example 13

**Wettable Powder**

| | |
|---|---|
| N-Cyclopropyl-N'-[(4-methoxy-6-methyl-1,3,5-triazin-2-yl)aminocarbonyl]-1,2-benzenedisulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

## Example 14

**Wettable Powder**

| | |
|---|---|
| N-Cyclopropyl-N'-[(4,6-dimethoxypyrimidin-2-yl)aminocarbonyl]-1,2-benzenedisulfonamide | 40% |
| sodium ligninsulfonate | 20% |
| montmorillonite clay | 40% |

The ingredients are thoroughly blended. coarsely hammer-milled and then air-milled to produce particles essentially all below 10 microns in size. The material is reblended and then packaged.

## Example 15

**Dust**

| | |
|---|---|
| N-Cyclopropyl-N'-[(4-methoxy-6-methyl-1,3,5-triazin-2-yl)aminocarbonyl]-1,2-benzenedisulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 16

Wettable Powder

| | |
|---|---|
| 2-(2,2-Dimethylhydrazinosulfonyl)N-[(4-methoxy-6-methyl-1,3,5-triazin-2-yl)aminocarbonyl]-benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended. hammer-milled until all the solids are essentially under 50 microns, reblended, and packaged.

Example 17

Wettable Powder

| | |
|---|---|
| 2-(2,2-Dimethylhydrazinosulfonyl)-N-[(4,6-dimethoxypyrimidin-2-yl)aminocarbonyl]-benzenesulfonamide | 50% |
| sodium alkylnaphthalenesulfonate | 2% |
| low viscosity methyl cellulose | 2% |
| diatomaceous earth | 46% |

The ingredients are blended. coarsely hammer-milled and then air-milled to produce particles essentially all below 10 microns in diameter. The product is reblended before packaging.

Example 18

Granule

| | |
|---|---|
| Wettable Powder of Example 17 | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules are dried and packaged.

Example 19

Extruded Pellet

| | |
|---|---|
| 2-(2,2-Dimethylhydrazinosulfonyl)-N-[(4,6-dimethoxypyrimidin-2-yl)aminocarbonyl]-benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

## Example 20

**Low Strength Granule**

| | |
|---|---|
| 2-(2,2-Dimethylhydrazinosulfonyl)N-[(4-methoxy-6-methyl-1,3,5-triazin-2-yl)aminocarbonyl]-benzenesulfonamide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed. the material is warmed to evaporate the solvent. The material is allowed to cool and then packaged

## Example 21

**Granule**

| | |
|---|---|
| 2-(2,2-Dimethylhydrazinosulfonyl)N-[(4-methoxy-6-methyl-1,3,5-triazin-2-yl)aminocarbonyl]-benzenesulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

## Example 22

**Low Strength Granule**

| | |
|---|---|
| 2-(2,2-Dimethylhydrazinosulfonyl)-N-[(4,6-dimethoxypyrimidin-2-yl)aminocarbonyl]-benzenesulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed. the blender is allowed to run for a short period and then the granules are packaged.

### Example 23

**Aqueous Suspension**

| | |
|---|---|
| 2-(2,2-Dimethylhydrazinosulfonyl)-N-[(4,6-dimethoxypyrimidin-2-yl)aminocarbonyl]-benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

### Example 24

**Solution**

| | |
|---|---|
| 2-(2,2-Dimethylhydrazinosulfonyl)N-[(4-methoxy-6-methyl-1,3,5-triazin-2-yl)aminocarbonyl]-benzenesulfonamide, sodium salt | 5% |
| water | 95% |

The salt is added directly to the water with stirring to produce the solution, which may then be packaged for use.

### Example 25

**High Strength Concentrate**

| | |
|---|---|
| 2-(2,2-Dimethylhydrazinosulfonyl)-N-[(4,6-dimethoxypyrimidin-2-yl)aminocarbonyl]-benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

## Example 26

Wettable Powder

| | |
|---|---|
| 2-(2,2-Dimethylhydrazinosulfonyl)N-[(4-methoxy-6-methyl-1,3,5-triazin-2-yl)aminocarbonyl]-benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

## Example 27

Wettable Powder

| | |
|---|---|
| 2-(2,2-Dimethylhydrazinosulfonyl)-N-[(4,6-dimethoxypyrimidin-2-yl)aminocarbonyl]-benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

## Example 28

Oil Suspension

| | |
|---|---|
| 2-(2,2-Dimethylhydrazinosulfonyl)N-[(4-methoxy-6-methyl-1,3,5-triazin-2-yl)aminocarbonyl]-benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

## Example 29

Dust

| | |
|---|---|
| 2-(2,2-Dimethylhydrazinosulfonyl)-N-[(4,6-dimethoxy-pyrimidin-2-yl)aminocarbonyl]benzenesulfonamide 10% | |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

## Example 30

**Oil Suspension**

| | |
|---|---|
| 2-(2,2-Dimethylhydrazinosulfonyl)N-[(4-methoxy-6-methyl-1,3,5-triazin-2-yl)aminocarbonyl]-benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

## Example 31

**Wettable Powder**

| | |
|---|---|
| 2-(2,2-Dimethylhydrazinosulfonyl)-N-[(4,6-dimethoxy-pyrimidin-2-yl)aminocarbonyl]benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

## Utility

Test results indicate that the compounds of the present invention are highly active preemergent or post emergent herbicides or plant growth regulants. Many of them have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Some of the compounds have utility for selective weed control in crops such as wheat. Alternatively, the subject compounds are useful to modify plant growth.

The rates of application for the compounds of the invention are determined by a number of factors. including their use as plant growth modifiers or as herbicides, the crop species involved, the types of weeds to be controlled, weather and climate, formulations selected, mode application and amount of foliage present. In general terms, the subject compounds should be applied at levels of around 0.01 to 10 kg/ha, the lower rates being suggested for use on lighter soils and/or those having a low organic matter content, for plant growth modification or for situations where only short-term persistence is required.

The compounds of the invention may be used in combination with any other commercial herbicide, examples of which are those of the triazine, triazole, uracil, urea, amide, diphenylether, carbamate and bipyridylium types.

The herbicidal properties of the subject compounds were discovered in a number of greenhouse tests. The test procedures and results follow.

Test A

Seeds of crabgrass (Digitaria spp.), barnyard-grass (Echinochloa crusgalli ). wild oats (Avena fatua ). cheatgrass (Bromus secalinus ), velvetleaf (Abutilon theophrasti ), morningglory (Ipomoea spp.), cocklebur (Xanthium Pensylvanicum ), sorghum, corn, soybean, sugar beet, cotton, rice, wheat and purple nutsedge (Cyperus rotundus ) tubers were planted and treated preemergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

B = burn:
C = chlorosis/necrosis;
D = defoliation;
E = emergence inhibition;
G = growth retardation;
H = formative effect;
U = unusual pigmentation;
X = axillary stimulation;
S = albinism; and
6Y = abscised buds or flowers.

Compounds

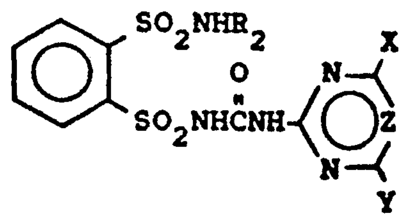

| Compound | $R_2$ | X | Y | Z |
|---|---|---|---|---|
| 1 | $CH_2C_6H_5$ | $CH_3$ | $CH_3$ | CH |
| 2 | $CH_2C_6H_5$ | $OCH_3$ | $CH_3$ | CH |
| 3 | $CH_2C_6H_5$ | $OCH_3$ | $OCH_3$ | CH |
| 4 | $CH_2C_6H_5$ | Cl | $OCH_3$ | CH |
| 5 | $CH_2C_6H_5$ | $OCH_3$ | $CH_3$ | N |
| 6 | $CH_2C_6H_5$ | $OCH_3$ | $OCH_3$ | N |
| 7 | cyclopropyl | $CH_3$ | $CH_3$ | CH |
| 8 | cyclopropyl | $CH_3$ | $OCH_3$ | CH |
| 9 | cyclopropyl | $OCH_3$ | $OCH_3$ | CH |
| 10 | cyclopropyl | Cl | $OCH_3$ | CH |
| 11 | cyclopropyl | $OCH_3$ | $CH_3$ | N |
| 12 | cyclopropyl | $OCH_3$ | $OCH_3$ | N |
| 13 | cyclobutyl | $CH_3$ | $CH_3$ | CH |
| 14 | cyclobutyl | $CH_3$ | $OCH_3$ | CH |
| 15 | cyclobutyl | $OCH_3$ | $OCH_3$ | CH |
| 16 | cyclobutyl | Cl | $OCH_3$ | CH |
| 17 | cyclobutyl | $OCH_3$ | $CH_3$ | N |
| 18 | cyclobutyl | $OCH_3$ | $OCH_3$ | N |
| 19 | $OCH_3$ | $CH_3$ | $OCH_3$ | CH |
| 20 | $OCH_3$ | $OCH_3$ | $OCH_3$ | CH |
| 21 | $OCH_3$ | Cl | $OCH_3$ | CH |
| 22 | $OCH_3$ | $CH_3$ | $OCH_3$ | N |
| 23 | $OCH_3$ | $OCH_3$ | $OCH_3$ | N |
| 24 | $CH_2CH(OCH_3)_2$ | $CH_3$ | $CH_3$ | CH |
| 25 | $CH_2CH(OCH_3)_2$ | $CH_3$ | $OCH_3$ | CH |

## Compounds (continued)

| Compound | $R_2$ | X | Y | Z |
|---|---|---|---|---|
| 26 | $CH_2CH(OCH_3)_2$ | $OCH_3$ | $OCH_3$ | CH |
| 27 | $CH_2CH(OCH_3)_2$ | Cl | $OCH_3$ | CH |
| 28 | $CH_2CH(OCH_3)_2$ | $CH_3$ | $OCH_3$ | N |
| 29 | $CH_2CH(OCH_3)_2$ | $OCH_3$ | $OCH_3$ | N |
| 30 | $CH_2C{\equiv}CH$ | $CH_3$ | $CH_3$ | CH |
| 31 | $CH_2C{\equiv}CH$ | $CH_3$ | $OCH_3$ | CH |
| 32 | $CH_2C{\equiv}CH$ | $OCH_3$ | $OCH_3$ | CH |
| 33 | $CH_2C{\equiv}CH$ | Cl | $OCH_3$ | CH |
| 34 | $CH_2C{\equiv}CH$ | $CH_3$ | $OCH_3$ | N |
| 35 | $CH_2C{\equiv}CH$ | $OCH_3$ | $OCH_3$ | N |
| 36 | $CH_2CH_2F$ | $CH_3$ | $CH_3$ | CH |
| 37 | $CH_2CH_2F$ | $OCH_3$ | $OCH_3$ | CH |
| 38 | $CH_2CH_2F$ | Cl | $OCH_3$ | CH |
| 39 | $CH_2CH_2F$ | $CH_3$ | $OCH_3$ | CH |
| 40 | $CH_2CH_2F$ | $CH_3$ | $OCH_3$ | N |
| 41 | $CH_2CH_2F$ | $OCH_3$ | $OCH_3$ | N |
| 42 | cyclopentyl | $CH_3$ | $CH_3$ | CH |
| 43 | cyclopentyl | $CH_3$ | $OCH_3$ | CH |
| 44 | cyclopentyl | $OCH_3$ | $OCH_3$ | CH |
| 45 | cyclopentyl | Cl | $OCH_3$ | CH |
| 46 | cyclopentyl | $CH_3$ | $OCH_3$ | N |
| 47 | cyclopentyl | $OCH_3$ | $OCH_3$ | N |
| 48 | $CH_2CH_2OH$ | $CH_3$ | $CH_3$ | CH |
| 49 | $CH_2CH_2OH$ | $CH_3$ | $OCH_3$ | CH |
| 50 | $CH_2CH_2OH$ | $OCH_3$ | $OCH_3$ | CH |
| 51 | $CH_2CH_2OH$ | Cl | $OCH_3$ | CH |
| 52 | $CH_2CH_2OH$ | $CH_3$ | $OCH_3$ | N |
| 53 | $CH_2CH_2N(CH_3)_2$ | $CH_3$ | $OCH_3$ | CH |
| 54 | $CH_2$ 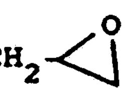 | $OCH_3$ | $OCH_3$ | CH |

Compounds (continued)

| Compound | $R_2$ | X | Y | Z |
|---|---|---|---|---|
| 55 | $CH_2CH_2CH_2Br$ | $CH_3$ | $CH_3$ | CH |
| 56 | $CH_2CH_2CH_2Br$ | $CH_3$ | $OCH_3$ | CH |
| 57 | $CH_2CH_2CH_2Br$ | $OCH_3$ | $OCH_3$ | CH |
| 58 | $CH_2CH_2CH_2Br$ | $CH_3$ | $OCH_3$ | CH |
| 59 | $CH_2CH_2CH_2Br$ | Cl | $OCH_3$ | N |
| 60 | $CH_2$-cyclopropyl | $CH_3$ | $CH_3$ | CH |
| 61 | $CH_2$-cyclopropyl | $CH_3$ | $OCH_3$ | CH |
| 62 | $CH_2$-cyclopropyl | $OCH_3$ | $OCH_3$ | CH |
| 63 | $CH_2$-cyclopropyl | $CH_3$ | $OCH_3$ | CH |
| 64 | $CH_2$-cyclopropyl | $OCH_3$ | $OCH_3$ | N |
| 65 | $CH_2$-cyclopropyl | Cl | $OCH_3$ | N |
| 66 | $CH_2CF_2CF_3$ | $CH_3$ | $CH_3$ | CH |
| 67 | $CH_2CF_2CF_3$ | $CH_3$ | $OCH_3$ | CH |
| 68 | $CH_2CF_2CF_3$ | $OCH_3$ | $OCH_3$ | CH |
| 69 | $CH_2CF_2CF_3$ | $CH_3$ | $OCH_3$ | N |
| 70 | $CH_2CF_2CF_3$ | $OCH_3$ | $OCH_3$ | N |

Compounds (continued)

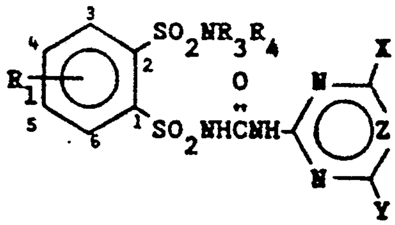

| Compound | $R_1$ | $R_2$ | $R_3$ | X | Y | Z | m.p.(°C) |
|---|---|---|---|---|---|---|---|
| 71 | 3-$CH_3$ | $CH_3$ | $CH_2CH_2F$ | $CH_3$ | $CH_3$ | CH | 175 to 176 |
| 72 | 3-$CH_3$ | $CH_3$ | $CH_2CH_2F$ | $CH_3$ | $OCH_3$ | CH | 170 to 171 |
| 73 | 3-$CH_3$ | $CH_3$ | $CH_2CH_2F$ | $OCH_3$ | $OCH_3$ | CH | 212 to 214 |
| 74 | 3-$CH_3$ | $CH_3$ | $CH_2CH_2F$ | Cl | $OCH_3$ | CH | 172 to 173 |
| 75 | 3-$CH_3$ | $CH_3$ | $CH_2CH_2F$ | $CH_3$ | $OCH_3$ | N | 177 to 178 |
| 76 | 3-$CH_3$ | $CH_3$ | $CH_2CH_2F$ | $OCH_3$ | $OCH_3$ | N | 182 to 184 |
| 77 | 3-$CH_3$ | $CH_3$ | $CH_2CH_2F$ | $CH_3$ | $CH_3$ | N | 178 to 179 |
| 78 | 3-$CH_3$ | $CH_3$ | $CH_2CH_2CH_2F$ | $CH_3$ | $CH_3$ | CH | 152 to 153 |
| 79 | 3-$CH_3$ | $CH_3$ | $CH_2CH_2CH_2F$ | $CH_3$ | $OCH_3$ | CH | 127 to 128 |
| 80 | 3-$CH_3$ | $CH_3$ | $CH_2CH_2CH_2F$ | $OCH_3$ | $OCH_3$ | CH | 188 to 189 |
| 81 | 3-$CH_3$ | $CH_3$ | $CH_2CH_2CH_2F$ | Cl | $OCH_3$ | CH | 155.5 to 156.5 |
| 82 | 3-$CH_3$ | $CH_3$ | $CH_2CH_2CH_2F$ | $CH_3$ | $OCH_3$ | N | 155 to 156 |
| 83 | 3-$CH_3$ | $CH_3$ | $CH_2CH_2CH_2F$ | $OCH_3$ | $OCH_3$ | N | 150 to 151 |
| 84 | 3-$CH_3$ | $CH_3$ | $CH_2CH_2CH_2F$ | $CH_3$ | $CH_3$ | N | 146 to 148 |
| 85 | H | $-CH_2CH=CHCH_2-$ | | Cl | $OCH_3$ | CH | 210 to 217(d) |
| 86 | H | $-CH_2CH=CHCH_2-$ | | $OCH_3$ | $OCH_3$ | CH | 220 to 230(d) |

Compounds (continued)

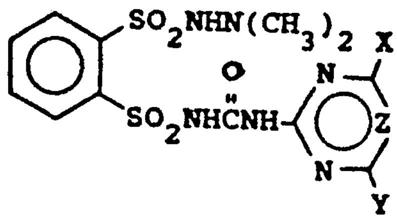

| Compound | X | Y | Z |
|---|---|---|---|
| 87 | $CH_3$ | $CH_3$ | CH |
| 88 | $OCH_3$ | $CH_3$ | CH |
| 89 | $OCH_3$ | $OCH_3$ | CH |
| 90 | Cl | $OCH_3$ | CH |
| 91 | $CH_3$ | $OCH_3$ | N |
| 92 | $OCH_3$ | $OCH_3$ | N |

Table A

| | Cmpd. 1 | Cmpd. 2 | Cmpd. 3 | Cmpd. 4 |
|---|---|---|---|---|
| Rate kg/ha | 0.05 | 0.05 | 0.05 | 0.05 |
| POSTEMERGENCE | | | | |
| Morningglory | 0 | 4C,9G | 9C | 4C,9G |
| Cocklebur | 4C,9G | 5C,9G | 10C | 5C,9G |
| Velvetleaf | 4C,8H | 9C | 9C | 4C,8H |
| Nutsedge | 2G | 3C,8G | 3C,9G | 3C,8G |
| Crabgrass | 0 | 3G | 2C,8G | 2G |
| Barnyardgrass | 5H | 2C,7H | 9C | 3C,7H |
| Cheatgrass | 2C,7G | 9C | 5C,9G | 6G |
| Wild Oats | 6G | 6G | 8G | 0 |
| Wheat | 3G | 2G | 6G | 0 |
| Corn | 2C,5H | 2C,8G | 2U,9G | 2G |
| Soybean | 4C,8H | 9C | 9C | 3C,7G |
| Rice | 5C,9G | 6C,9G | 6C,9G | 3C,8G |
| Sorghum | 2C,8H | 3C,9H | 9C | 4C,9H |
| Sugar beet | 9C | 4C,8G | 9C | 4C,8H |
| Cotton | 4C,9H | 4C,9G | 5C,9G | 4C,9H |
| PREEMERGENCE | | | | |
| Morningglory | 6G | 6G | 9G | 6G |
| Cocklebur | 5H | 8H | 8H | 0 |
| Velvetleaf | 6H | 4C,9G | 5C,9G | 5G |
| Nutsedge | 0 | 6G | 10E | 5G |
| Crabgrass | 2G | 2G | 5G | 2G |
| Barnyardgrass | 0 | 3C,8H | 3C,9H | 3H |
| Cheatgrass | 8G | 5C,9H | 4C,8H | 6G |
| Wild Oats | 2C,6G | 2C,9G | 3C,9H | 3G |
| Wheat | 6G | 8G | 2C,9G | 3G |
| Corn | 6G | 2C,9G | 9G | 3C,6G |
| Soybean | 0 | 3C,7G | 3C,6G | 0 |
| Rice | 9H | 10E | 10E | 9H |
| Sorghum | 4C,5G | 4C,9H | 9H | 2C,9G |
| Sugar beet | 4C,8G | 5C,9G | 9G | 8G |
| Cotton | 4G | 7G | 8G | 4G |

Table A (continued)

| | Cmpd. 5 | Cmpd. 6 | Cmpd. 7 | Cmpd. 8 |
|---|---|---|---|---|
| Rate kg/ha | 0.05 | 0.05 | 0.05 | 0.05 |
| POSTEMERGENCE | | | | |
| Morningglory | 4C,8G | 4C,8H | 5C,9G | 10C |
| Cocklebur | 3C,5G | 0 | 9C | 10C |
| Velvetleaf | 5C,9G | 4C,8H | 9C | 9C |
| Nutsedge | 0 | 0 | 8G | 2C,7G |
| Crabgrass | 2G | 0 | 4G | 4G |
| Barnyardgrass | 3C,9H | 3C,6H | 3C,9H | 3C,8H |
| Cheatgrass | 4C,9G | 2C,7G | 3C,8G | 4C,8G |
| Wild Oats | 5C,9G | 5C,9H | 8G | 2C,5G |
| Wheat | 4C,9G | 2G | 5G | 2G |
| Corn | 5U,9C | 3C,9H | 7H | 4C,9H |
| Soybean | 9C | 5C,9G | 9C | 9C |
| Rice | 6C,9G | 6C,9G | 5C,9G | 5C,9G |
| Sorghum | 3C,8H | 3C,6H | 3U,9H | 2C,8G |
| Sugar beet | 9C | 5C,9H | 3C,6G | 5C,9G |
| Cotton | 5C,9G | 5C,9G | 9C | 5C,9G |
| PREEMERGENCE | | | | |
| Morningglory | 7G | 4G | 9G | 8G |
| Cocklebur | 4G | 0 | 8H | 8H |
| Velvetleaf | 2C,8G | 3C,8H | 4C,9G | 9G |
| Nutsedge | 0 | 0 | 7G | 10E |
| Crabgrass | 0 | 0 | 5G | 8G |
| Barnyardgrass | 3H | 0 | 6G | 4C,9H |
| Cheatgrass | 3C,8H | 2G | 7G | 9H |
| Wild Oats | 3C,8G | 0 | 8G | 3C,7G |
| Wheat | 2C,8G | 0 | 8G | 7G |
| Corn | 8G | 3C,7G | 8G | 2C,9G |
| Soybean | 3C,3G | 2C | 5H | 3C,6H |
| Rice | 9H | 4C,8H | 10E | 5C,9H |
| Sorghum | 3C,9H | 4C,6H | 10E | 3C,9H |
| Sugar beet | 9G | 5C,9G | 6G | 8G |
| Cotton | 7G | 7G | 8G | 9G |

Table A (continued)

| | Cmpd. 9 | Cmpd. 10 | Cmpd. 11 | Cmpd. 12 |
|---|---|---|---|---|
| Rate kg/ha | 0.05 | 0.05 | 0.05 | 0.05 |
| POSTEMERGENCE | | | | |
| Morningglory | 9C | 9C | 10C | 9C |
| Cocklebur | 10C | 9C | 10C | 10C |
| Velvetleaf | 9C | 2C,8G | 9C | 5C,9G |
| Nutsedge | 5C,9G | 2C,7G | 3G | 4G |
| Crabgrass | 7G | 4G | 7G | 6G |
| Barnyardgrass | 5C,9G | 3C,9H | 5C,9H | 3C,7H |
| Cheatgrass | 5C,9G | 8G | 5C,9G | 3C,8G |
| Wild Oats | 3C,7G | 0 | 5C,9G | 4C,8G |
| Wheat | 0 | 0 | 2C,9G | 5G |
| Corn | 4U,8H | 3H | 10C | 10C |
| Soybean | 5C,9G | 3C,8G | 9C | 9C |
| Rice | 4C,9G | 7G | 5C,9G | 5C,9G |
| Sorghum | 4U,9H | 3U,9G | 5U,9C | 6C,9H |
| Sugar beet | 9C | 3C,6H | 9C | 9C |
| Cotton | 4C,9H | 5C,9H | 5C,9G | 5C,9H |
| PREEMERGENCE | | | | |
| Morningglory | 9G | 9G | 9G | 9G |
| Cocklebur | - | 4C,5H | 9H | - |
| Velvetleaf | 5C,9G | 3G | 5C,9G | 5C,9G |
| Nutsedge | 10E | 10E | 3G | 4G |
| Crabgrass | 5C,9G | 5G | 7G | 8G |
| Barnyardgrass | 9H | 3C,7H | 3C,8H | 3C,8H |
| Cheatgrass | 9H | 5G | 2C,7H | 2C,5G |
| Wild Oats | 3C,6G | 2G | 3C,8H | 2C,6G |
| Wheat | 3G | 0 | 2C,8G | 3C,8G |
| Corn | 9G | 2C,5G | 5C,9G | 3C,9G |
| Soybean | 5H | 0 | 4C,6H | 3C,6H |
| Rice | 10E | 3C,8H | 10E | 5C,9H |
| Sorghum | 2C,9G | 3C,8H | 10E | 5C,9H |
| Sugar beet | 9G | 7G | 5C,9G | 5C,9G |
| Cotton | 9G | 8G | 9G | 8G |

Table A (continued)

| | Cmpd. 13 | Cmpd. 14 | Cmpd. 15 | Cmpd. 16 |
|---|---|---|---|---|
| Rate kg/ha | 0.05 | 0.05 | 0.05 | 0.05 |
| POSTEMERGENCE | | | | |
| Morningglory | 2H | 4C,9G | 9C | 4C,8H |
| Cocklebur | 4C,9G | 9C | 9C | 5C,9G |
| Velvetleaf | 4C,8H | 4C,8H | 5C,9G | 4G |
| Nutsedge | 5G | 3C,8G | 7G | 7G |
| Crabgrass | 0 | 4G | 6G | 2G |
| Barnyardgrass | 2H | 3C,8H | 3C,9H | 2C,8H |
| Cheatgrass | 3C,8G | 4C,8G | 4C,8G | 5G |
| Wild Oats | 8G | 7G | 8G | 2G |
| Wheat | 0 | 0 | 5G | 0 |
| Corn | 3C,6H | 4U,9G | 4C,9G | 6H |
| Soybean | 3C,8G | 9C | 5C,9G | 2C,5H |
| Rice | 5C,9G | 5C,9G | 5C,9G | 8G |
| Sorghum | 3C,7H | 4C,9H | 4C,9H | 2C,9H |
| Sugar beet | 4C,8H | 9C | 5C,9G | 2C,3G |
| Cotton | 4C,7H | 4C,7G | 4C,9G | 4C,8G |
| PREEMERGENCE | | | | |
| Morningglory | 5G | 8G | 9G | 7G |
| Cocklebur | 4G | 7H | 8H | 1C |
| Velvetleaf | 0 | 8G | 9G | 0 |
| Nutsedge | 0 | 8G | 10E | 0 |
| Crabgrass | 4G | 4G | 3G | 3G |
| Barnyardgrass | 0 | 8G | 9H | 2G |
| Cheatgrass | 6G | 9H | 10E | 5G |
| Wild Oats | 2C,5G | 4C,7H | 2C,8H | 3G |
| Wheat | 4G | 8G | 8H | 0 |
| Corn | 2C,5G | 4C,9H | 9G | 2C,5G |
| Soybean | 1H | 4C,6H | 3C,6H | 0 |
| Rice | 8H | 9H | 10E | 3C,8H |
| Sorghum | 3C,7G | 5C,9H | 4C,9H | 3C,9H |
| Sugar beet | 8G | 5C,9G | 9G | 8G |
| Cotton | 3G | 9G | 9G | 0 |

Table A (continued)

| | Cmpd. 17 | Cmpd. 18 | Cmpd. 19 |
|---|---|---|---|
| Rate kg/ha | 0.05 | 0.05 | 0.05 |
| POSTEMERGENCE | | | |
| Morningglory | 9C | 5C,9G | 6C,9G |
| Cocklebur | 3C,9G | 5G | 9C |
| Velvetleaf | 5C,9G | 3C,7G | 9C |
| Nutsedge | 0 | 4G | 4C,9G |
| Crabgrass | 3G | 2G | 4C,8G |
| Barnyardgrass | 4C,9H | 3C,7H | 4C,8H |
| Cheatgrass | 2C,7G | 5G | 9C |
| Wild Oats | 4G | 8G | 3G |
| Wheat | 4G | 7G | 5G |
| Corn | 5C,9G | 3U,9G | 8H |
| Soybean | 9C | 5C,9G | 9C |
| Rice | 5C,9G | 9G | 9C |
| Sorghum | 3U,9G | 5C,9G | 8H |
| Sugar beet | 9C | 3C,7G | 5C,9G |
| Cotton | 4C,9G | 4C,7G | 9C |
| PREEMERGENCE | | | |
| Morningglory | 9G | 7H | 9G |
| Cocklebur | 5H | 1C | - |
| Velvetleaf | 2G | 0 | 5C,9G |
| Nutsedge | 0 | 0 | 9G |
| Crabgrass | 5G | 2G | 7G |
| Barnyardgrass | 2G | 0 | 3C,8H |
| Cheatgrass | 5G | 0 | 9H |
| Wild Oats | 2H | 0 | 2C,8G |
| Wheat | 6G | 3G | 3C,8H |
| Corn | 8G | 3C,7G | 5U,9H |
| Soybean | 2C,5H | 1C | 8H |
| Rice | 7H | 5G | 10E |
| Sorghum | 4C,9H | 3C,5G | 9H |
| Sugar beet | 4C,8G | 8G | 10E |
| Cotton | 7G | 3G | 9G |

Table A (continued)

| | Cmpd. 20 | Cmpd. 21 | Cmpd. 22 | Cmpd. 23 |
|---|---|---|---|---|
| Rate kg/ha | 0.05 | 0.05 | 0.05 | 0.05 |
| POSTEMERGENCE | | | | |
| Morningglory | 10C | 10C | 10C | 10C |
| Cocklebur | 10C | 9C | 9C | 4G |
| Velvetleaf | 9C | 5C,9G | 9C | 9C |
| Nutsedge | 6C,9G | 5C,9G | 4G | 3G |
| Crabgrass | 3C,5G | 2C,5G | 5C,9G | 3C,8G |
| Barnyardgrass | 9C | 4C,7H | 5C,9H | 6C,9H |
| Cheatgrass | 9C | 6G | 3C,6G | 5G |
| Wild Oats | 3C,7G | 0 | 6C,9G | 5C,9G |
| Wheat | 4G | 0 | 5C,9G | 2C,7G |
| Corn | 6C,9H | 6H | 9C | 10C |
| Soybean | 9C | 5C,9G | 9C | 9C |
| Rice | 6C,9G | 4C,7G | 9C | 9C |
| Sorghum | 9C | 4C,8H | 9C | 6C,9H |
| Sugar beet | 10C | 3C,6G | 10C | 9C |
| Cotton | 10C | 9C | 10C | 9C |
| PREEMERGENCE | | | | |
| Morningglory | 9G | 8G | 9G | 9G |
| Cocklebur | 8H | 9H | 9H | 8H |
| Velvetleaf | 5C,9G | 8G | 9C | 3C,7G |
| Nutsedge | 10E | 10E | 5G | 0 |
| Crabgrass | 3C,7G | 0 | 3C,7G | 2C,5G |
| Barnyardgrass | 8H | 4C,4H | 3C,8H | 3C,7H |
| Cheatgrass | 9H | 2C,5G | 3C,7H | 3C,6G |
| Wild Oats | 3C,6G | 2C,4G | 5C,9H | 3C,8G |
| Wheat | 5G | 2G | 4C,9H | 4C,9G |
| Corn | 5U,9G | 3C,8G | 3C,9H | 5C,9H |
| Soybean | 8H | 2H | 8H | 4C,8H |
| Rice | 10E | 9H | 10E | 10E |
| Sorghum | 5C,9H | 3C,8G | 10E | 5C,9H |
| Sugar beet | 9G | 3C,8G | 10C | 5C,9G |
| Cotton | 9G | 8G | 4C,9G | 5C,9G |

Table A (continued)

| | Cmpd. 24 | Cmpd. 25 | Cmpd. 26 | Cmpd. 27 |
|---|---|---|---|---|
| Rate kg/ha | 0.05 | 0.05 | 0.05 | 0.05 |
| POSTEMERGENCE | | | | |
| Morningglory | 2C,7G | 3C,8G | 3C,9H | 2C,4G |
| Cocklebur | 3C,9H | 3C,9H | 5C,9G | 8H |
| Velvetleaf | 6H | 3C,8G | 9C | 1H |
| Nutsedge | 2G | 4G | 2C,9G | 0 |
| Crabgrass | 3G | 2G | 3C,7G | 2G |
| Barnyardgrass | 3C,9H | 3C,9H | 9H | 3C,9H |
| Cheatgrass | 2C,7G | 3C,6G | 3C,8G | 3G |
| Wild Oats | 2C,4G | 2C,8G | 7G | 0 |
| Wheat | 0 | 0 | 4G | 0 |
| Corn | 4G | 2C,8H | 3C,9H | 5G |
| Soybean | 2C,9G | 5C,9G | 5C,9G | 5H |
| Rice | 4C,9G | 4C,9G | 6C,9G | 4C,9G |
| Sorghum | 4C,8H | 4C,9H | 9H | 4C,8H |
| Sugar beet | 3C,7H | 3C,6G | 3C,9G | 2G |
| Cotton | 2C,5G | 2C,5G | 4C,9G | 2G |
| PREEMERGENCE | | | | |
| Morningglory | 2C,4G | 7G | 3C,5G | 2C,9G |
| Cocklebur | 2C | 10C | 8H | 2H |
| Velvetleaf | 3G | 8G | 5C,9G | 2G |
| Nutsedge | 0 | 4G | 8G | 0 |
| Crabgrass | 0 | 0 | 4G | 0 |
| Barnyardgrass | 0 | 3C,8H | 4C,9H | 3C,7H |
| Cheatgrass | 2G | 8G | 8G | 4G |
| Wild Oats | 2C,5G | 3C,7G | 2C,8G | 3C,3G |
| Wheat | 0 | 2C,5G | 3C,9G | 2G |
| Corn | 2C,5G | 3C,8H | 4C,9G | 2C,5G |
| Soybean | 2C,2H | 6G | 3C,3H | 3C,3G |
| Rice | 3C,8H | 3C,8H | 4C,9H | 8H |
| Sorghum | 3C,7H | 3C,8H | 4C,9H | 3C,8G |
| Sugar beet | 2C,5G | 2C,8G | 6G | 2G |
| Cotton | 0 | 7G | 3C,5G | 3G |

Table A (continued)

| | Cmpd. 28 | Cmpd. 29 | Cmpd. 30 | Cmpd. 31 |
|---|---|---|---|---|
| Rate kg/ha | 0.05 | 0.05 | 0.05 | 0.05 |
| POSTEMERGENCE | | | | |
| Morningglory | 3C,8G | 2C,5G | 10C | 10C |
| Cocklebur | 2G | 0 | 9C | 9C |
| Velvetleaf | 2G | 0 | 10C | 10C |
| Nutsedge | 0 | 0 | 7G | 2C,8G |
| Crabgrass | 2G | 0 | 6G | 3C,7H |
| Barnyardgrass | 4H | 0 | 3C,8H | 9H |
| Cheatgrass | 0 | 0 | 3C,9G | 5C,9G |
| Wild Oats | 0 | 0 | 3C,8H | 4C,8G |
| Wheat | 0 | 0 | 3G | 0 |
| Corn | 5H | 5H | 2H | 9H |
| Soybean | 3C,9G | 2C,8H | 2C,5G | 5C,9G |
| Rice | 2C,8G | 5G | 5C,9G | 5C,9G |
| Sorghum | 2H | 2G | 4C,9H | 2C,9H |
| Sugar beet | 3C,7G | 2C,5G | 9C | 9C |
| Cotton | 1H | 2C,6H | 4C,8G | 9C |
| PREEMERGENCE | | | | |
| Morningglory | 2C,4G | 2C,4G | 9G | 9G |
| Cocklebur | 5G | 2H | 9H | 9H |
| Velvetleaf | 5G | 2G | 9G | 9C |
| Nutsedge | 0 | 0 | 4G | 7G |
| Crabgrass | 2G | 3G | 3G | 5G |
| Barnyardgrass | 0 | 1C | 3C,6G | 2C,8H |
| Cheatgrass | 0 | 0 | 8H | 10E |
| Wild Oats | 0 | 2C | 2C,8G | 5C,9G |
| Wheat | 0 | 2G | 7G | 8G |
| Corn | 2G | 0 | 2C,7G | 9H |
| Soybean | 0 | 1C | 2C,3G | 3C,7H |
| Rice | 3G | 0 | 9H | 10E |
| Sorghum | 2C,4G | 0 | 3C,9G | 10H |
| Sugar beet | 0 | 7G | 8G | 9C |
| Cotton | 0 | 2G | 9C | 9C |

Table A (continued)

| | Cmpd. 32 | Cmpd. 33 | Cmpd. 34 | Cmpd. 35 |
|---|---|---|---|---|
| Rate kg/ha | 0.05 | 0.05 | 0.05 | 0.05 |
| POSTEMERGENCE | | | | |
| Morningglory | 10C | 10C | 10C | 10C |
| Cocklebur | 9C | 9C | 10C | 8G |
| Velvetleaf | 10C | 10C | 10C | 9C |
| Nutsedge | 2C,9G | 9G | 4G | 5G |
| Crabgrass | 3C,8G | 6G | 4C,8G | 4C,8G |
| Barnyardgrass | 5C,9H | 4C,9H | 9C | 3C,5H |
| Cheatgrass | 9C | 2C,9G | 9C | 8G |
| Wild Oats | 4C,9G | 0 | 9C | 5C,8G |
| Wheat | 3G | 0 | 5C,9G | 8G |
| Corn | 2C,9G | 5H | 9C | 4U,9G |
| Soybean | 9C | 3C,8G | 5C,9G | 5C,9G |
| Rice | 6C,9G | 5C,9G | 9C | 9C |
| Sorghum | 3C,9G | 4C,9H | 4U,9H | 3C,9H |
| Sugar beet | 9C | 3C,8G | 9C | 9C |
| Cotton | 9C | 9C | 9C | 4C,9H |
| PREEMERGENCE | | | | |
| Morningglory | 3C,9H | 9G | 9H | 9G |
| Cocklebur | 9H | 8H | 9H | 8G |
| Velvetleaf | 9G | 9C | 9C | 9C |
| Nutsedge | 10E | 3C,9G | 0 | 0 |
| Crabgrass | 7G | 4G | 2C,4G | 4G |
| Barnyardgrass | 9H | 3C,7H | 3C,8H | 2C,3G |
| Cheatgrass | 10H | 2C,8G | 3C,7G | 3G |
| Wild Oats | 3C,8G | 2C,5G | 3C,6G | 2C,3G |
| Wheat | 2C,8G | 0 | 3C,8G | 3C,6G |
| Corn | 2U,9H | 3C,7G | 3C,9H | 3C,9G |
| Soybean | 9H | 2C,2H | 2C,3G | 3C,3H |
| Rice | 10E | 5C,9H | 9H | 5C,9H |
| Sorghum | 5C,9H | 3C,9G | 5C,9H | 4C,9H |
| Sugar beet | 9G | 4C,8G | 9C | 9C |
| Cotton | 9G | 9G | 9G | 9G |

Table A (continued)

| | Cmpd. 36 | Cmpd. 37 | Cmpd. 38 |
|---|---|---|---|
| Rate kg/ha | 0.05 | 0.05 | 0.05 |
| POSTEMERGENCE | | | |
| Morningglory | 9C | 10C | 10C |
| Cocklebur | 9C | 10C | 10C |
| Velvetleaf | 10C | 10C | 9C |
| Nutsedge | 2C,9G | 3C,9G | 5C,9G |
| Crabgrass | 6G | 7G | 4G |
| Barnyardgrass | 9H | 9C | 9H |
| Cheatgrass | 4C,9G | 9C | 2C,9G |
| Wild Oats | 5C,9H | 4C,8G | 0 |
| Wheat | 3G | 2G | 0 |
| Corn | 8H | 10C | 7H |
| Soybean | 4C,9G | 9C | 3C,8G |
| Rice | 9C | 9C | 5C,9G |
| Sorghum | 2C,9H | 5C,9G | 5C,9H |
| Sugar beet | 4C,8G | 9C | 3C,7H |
| Cotton | 9C | 10C | 4C,9G |
| PREEMERGENCE | | | |
| Morningglory | 9G | 9G | 9G |
| Cocklebur | 9H | 9H | 9H |
| Velvetleaf | 4C,9G | 9C | 9C |
| Nutsedge | 6G | 10E | 10E |
| Crabgrass | 4G | 4G | 0 |
| Barnyardgrass | 4G | 5C,9H | 4C,8H |
| Cheatgrass | 8H | 9H | 8H |
| Wild Oats | 2C,8G | 5C,9G | 2C,5G |
| Wheat | 2C,5G | 2C,9G | 0 |
| Corn | 4C,8G | 3U,9G | 3C,7G |
| Soybean | 3C,5H | 9H | 3C,3H |
| Rice | 10E | 10E | 4C,9H |
| Sorghum | 9H | 9H | 4C,9H |
| Sugar beet | 4C,8G | 9G | 9G |
| Cotton | 9G | 9G | 9G |

Table A (continued)

| | Compound 39 | | Cmpd. 40 |
|---|---|---|---|
| Rate kg/ha | 0.05 | 0.01 | 0.05 |
| POSTEMERGENCE | | | |
| Morningglory | 10C | 10C | 10C |
| Cocklebur | 10C | 9C | 10C |
| Velvetleaf | 10C | 10C | 9C |
| Nutsedge | 3C,9G | 2C,8G | 8G |
| Crabgrass | 5G | 2G | 2C,6G |
| Barnyardgrass | 9H | 4C,8H | 5C,9H |
| Cheatgrass | 9C | 5C,9G | 4C,9G |
| Wild Oats | 3C,9G | 4C,9G | 4C,9G |
| Wheat | 5G | 3G | 2C,9G |
| Corn | 2C,9H | 2C,9H | 5U,9G |
| Soybean | 9C | 9C | 5C,9G |
| Rice | 9C | 5C,9G | 5C,9G |
| Sorghum | 3C,9G | 3C,9H | 4C,9H |
| Sugar beet | 4C,9G | 4C,8G | 9C |
| Cotton | 9C | 4C,9G | 4C,8G |
| PREEMERGENCE | | | |
| Morningglory | 3C,9G | 8G | 9G |
| Cocklebur | 8H | 8H | 8H |
| Velvetleaf | 9C | 9C | 2G |
| Nutsedge | 9G | 8G | 5G |
| Crabgrass | 5G | 0 | 4G |
| Barnyardgrass | 8G | 3C,6G | 2C,5G |
| Cheatgrass | 4C,9H | 9H | 2C,7G |
| Wild Oats | 4C,6G | 2C,8G | 4C,8G |
| Wheat | 5G | 5G | 2C,8G |
| Corn | 3C,8H | 8G | 3C,9H |
| Soybean | 3C,6H | 2C,4G | 3C,4H |
| Rice | 10E | 4C,9H | 10E |
| Sorghum | 5C,9H | 4C,8H | 5C,9H |
| Sugar beet | 10E | 10C | 5C,9G |
| Cotton | 9G | 8G | 2C,8G |

Table A (continued)

| | Cmpd. 41 | Cmpd. 42 | Cmpd. 43 | Cmpd. 44 |
|---|---|---|---|---|
| Rate kg/ha | 0.05 | 0.05 | 0.05 | 0.05 |
| POSTEMERGENCE | | | | |
| Morningglory | 5C,9G | 1C,3G | 2C,7G | 4C,8H |
| Cocklebur | 3G | 2C,8H | 8G | 2C,8G |
| Velvetleaf | 4C,9G | 2C,5G | 4C,9H | 4C,9G |
| Nutsedge | 5G | 5G | 4C,9G | 4C,9G |
| Crabgrass | 3C,6G | 0 | 3C,6G | 2C,6G |
| Barnyardgrass | 2H | 2C,5H | 3C,6H | 5C,9H |
| Cheatgrass | 5G | 3C,9G | 5C,9G | 5C,9G |
| Wild Oats | 5C,9G | 3C,9G | 7G | 2C,8G |
| Wheat | 8G | 3G | 2G | 2G |
| Corn | 10C | 7H | 3C,9G | 4U,9G |
| Soybean | 9C | 3C,7H | 9C | 9C |
| Rice | 9C | 4C,9G | 5C,9G | 5C,9G |
| Sorghum | 9C | 4C,9H | 9C | 9C |
| Sugar beet | 9C | 4C,8G | 9C | 9C |
| Cotton | 5C,9G | 4C,7G | 4C,8H | 4C,9G |
| PREEMERGENCE | | | | |
| Morningglory | 2C,9G | 0 | 6G | 5G |
| Cocklebur | 4G | 0 | 3H | 8H |
| Velvetleaf | 3G | 3G | 9C | 9C |
| Nutsedge | 0 | 0 | 4C,9G | 10E |
| Crabgrass | 2C | 0 | 2C,5G | 6G |
| Barnyardgrass | 2C,6G | 0 | 3C,8H | 9H |
| Cheatgrass | 3C,5G | 8G | 9H | 10E |
| Wild Oats | 2C,7G | 2C,6G | 4C,8G | 8G |
| Wheat | 2C,7G | 2G | 4C,8G | 8G |
| Corn | 4C,9H | 2G | 4C,9G | 2C,8G |
| Soybean | 3C,5G | 1H | 3C,4H | 3H,5G |
| Rice | 10E | 4C,6G | 10E | 9H |
| Sorghum | 4C,9H | 2C,3G | 3C,9G | 3C,9G |
| Sugar beet | 10E | 3C,6G | 9C | 10C |
| Cotton | 3C,5G | 2G | 8G | 4G |

## Table A (continued)

| | Cmpd. 45 | Cmpd. 46 | Cmpd. 47 | Cmpd. 48 |
|---|---|---|---|---|
| Rate kg/ha | 0.05 | 0.05 | 0.05 | 0.05 |
| POSTEMERGENCE | | | | |
| Morningglory | 6G | 5C,9G | 2G | 2G |
| Cocklebur | 2C,9G | 3G | 0 | 4C,9G |
| Velvetleaf | 0 | 3C,5G | 3G | 1H |
| Nutsedge | 9G | 0 | 0 | 2C,5G |
| Crabgrass | 0 | 2G | 0 | 0 |
| Barnyardgrass | 8H | 7H | 2H | 3C,8H |
| Cheatgrass | 5G | 2C,8G | 0 | 3G |
| Wild Oats | 0 | 9G | 0 | 0 |
| Wheat | 0 | 4G | 0 | 2G |
| Corn | 3H | 4U,9C | 2C,7H | 7H |
| Soybean | 2C,5G | 9C | 4C,9G | 5C,9G |
| Rice | 5G | 4C,9G | 8G | 9C |
| Sorghum | 3C,8H | 4C,9H | 6H | 2C,8G |
| Sugar beet | 2G | 9C | 4C,8G | 3C,4H |
| Cotton | 1H | 4C,9H | 2C,5G | 3C,5H |
| PREEMERGENCE | | | | |
| Morningglory | 3G | 4H | 0 | 7G |
| Cocklebur | 2G | 0 | 0 | 4G |
| Velvetleaf | 5G | 4C,9G | 0 | 5G |
| Nutsedge | 5G | 0 | 0 | 3G |
| Crabgrass | 2G | 0 | 0 | 0 |
| Barnyardgrass | 3H | 1H | 0 | 0 |
| Cheatgrass | 8G | 7H | 0 | 3G |
| Wild Oats | 0 | 4G | 0 | 0 |
| Wheat | 0 | 2C,6G | 0 | 0 |
| Corn | 5G | 2C,6G | 0 | 0 |
| Soybean | 0 | 2H | 0 | 2C,3H |
| Rice | 9H | 2C,5G | 0 | 8H |
| Sorghum | 3C,8H | 3C,5G | 0 | 3C,6H |
| Sugar beet | 7G | 4C,9G | 6G | 3H |
| Cotton | 0 | 6G | 0 | 0 |

Table A (continued)

| | Cmpd. 49 | Cmpd. 50 | Cmpd. 51 | Cmpd. 52 |
|---|---|---|---|---|
| Rate kg/ha | 0.05 | 0.05 | 0.05 | 0.05 |
| POSTEMERGENCE | | | | |
| Morningglory | 8G | 6H | 1C,2G | 2C,5G |
| Cocklebur | 5C,9G | 10C | 4C,9G | 0 |
| Velvetleaf | 5C,9H | 5C,9G | 1C | 3C,7G |
| Nutsedge | 4C,9G | 3C,9G | 4G | 0 |
| Crabgrass | 3G | 5G | 0 | 0 |
| Barnyardgrass | 6C,9H | 9C | 9H | 3H |
| Cheatgrass | 8G | 3C,8G | 0 | 0 |
| Wild Oats | 2C,5G | 3G | 0 | 0 |
| Wheat | 8G | 3G | 0 | 0 |
| Corn | 3U,9G | 9C | 6H | 2C,9G |
| Soybean | 9C | 9C | 3C,7G | 4C,9G |
| Rice | 6C,9G | 9C | 6G | 8G |
| Sorghum | 3U,9G | 3U,9H | 3C,8G | 3G |
| Sugar beet | 5C,9G | 4C,9G | 2C,3H | 9C |
| Cotton | 5C,9G | 4C,9G | 2C,4G | 4C,9G |
| PREEMERGENCE | | | | |
| Morningglory | 9G | 9G | 6G | 2G |
| Cocklebur | 9H | 8H | 7H | 3H |
| Velvetleaf | 9C | 8G | 5C,9G | 3G |
| Nutsedge | 9G | 4C,9G | 3G | 0 |
| Crabgrass | 2C,6G | 2G | 0 | 0 |
| Barnyardgrass | 5C,9H | 3C,9H | 3C,7H | 0 |
| Cheatgrass | 5C,9H | 8G | 5G | 0 |
| Wild Oats | 2C,8G | 3C,8G | 0 | 0 |
| Wheat | 7G | 3C,9H | 0 | 0 |
| Corn | 3C,9H | 3C,9H | 4G | 3G |
| Soybean | 9H | 3C,8H | 3C,3H | 2C,2H |
| Rice | 10E | 3C,9H | 4C,8H | 0 |
| Sorghum | 10E | 5C,9H | 3C,7H | 2C,3H |
| Sugar beet | 10C | 9G | 5G | 3C,5G |
| Cotton | 10C | 9G | 2G | 3G |

Table A (continued)

| | Cmpd. 53 | Compound 54 | |
|---|---|---|---|
| Rate kg/ha | 0.05 | 0.05 | 0.01 |
| POSTEMERGENCE | | | |
| Morningglory | 2C | 4C,9G | 3C,5G |
| Cocklebur | 4C,5G | 2C,9G | 2C,7G |
| Velvetleaf | 5C,8H | 9C | 4C,8H |
| Nutsedge | 0 | 6G | 2C,4G |
| Crabgrass | 2G | 2C,8G | 2G |
| Barnyardgrass | 3C,6H | 4C,9H | 2H |
| Cheatgrass | 2C,5G | 2C,9G | 2C,8G |
| Wild Oats | 0 | 2C,7G | 0 |
| Wheat | 0 | 0 | 0 |
| Corn | 1H | 3C,7H | 4H |
| Soybean | 3C,4H | 4C,9G | 5C,9G |
| Rice | 4C,6G | 4C,9G | 4C,9G |
| Sorghum | 3C,4G | 3C,9G | 3C,6G |
| Sugar beet | 3C,4H | 4C,8H | 2C |
| Cotton | 4C,7G | 4C,9H | 4C,8G |
| PREEMERGENCE | | | |
| Morningglory | 4G | 9G | 3G |
| Cocklebur | 0 | 9H | 3G |
| Velvetleaf | 0 | 8G | 3G |
| Nutsedge | 0 | 0 | 0 |
| Crabgrass | 0 | 5G | 4G |
| Barnyardgrass | 0 | 7G | 1C |
| Cheatgrass | 0 | 3C,8G | 5G |
| Wild Oats | 0 | 9G | 5G |
| Wheat | 0 | 0 | 0 |
| Corn | 0 | 2C,8G | 3G |
| Soybean | 0 | 3C,7H | 3C,4H |
| Rice | 0 | 3C,8H | 2G |
| Sorghum | 0 | 3C,7G | 2G |
| Sugar beet | 5G | 8G | 6G |
| Cotton | 0 | 6G | 4G |

Table A (continued)

| | Cmpd. 55 | Cmpd. 56 | Cmpd. 57 | Cmpd.58 |
|---|---|---|---|---|
| Rate kg/ha | 0.05 | 0.05 | 0.05 | 0.05 |
| POSTEMERGENCE | | | | |
| Morningglory | 0 | 5G | 8G | 0 |
| Cocklebur | 2G | 7G | 7G | 0 |
| Velvetleaf | 0 | 2C,7G | 3C,8H | 0 |
| Nutsedge | 2C | 3C,8G | 5G | 0 |
| Crabgrass | 0 | 0 | 7G | 0 |
| Barnyardgrass | 0 | 3C,7H | 3C,9H | 0 |
| Cheatgrass | 8G | 4C,9G | 2C,9G | 0 |
| Wild Oats | 2C,8G | 3C,8G | 3C,9G | 2C,8G |
| Wheat | 0 | 0 | 2G | 0 |
| Corn | 2G | 3C,9H | 3C,9H | 3C,9G |
| Soybean | 1H | 3C,9G | 5C,9G | 0 |
| Rice | 3G | 2C,9G | 5C,9G | 8G |
| Sorghum | 3C,7G | 3C,9H | 4C,9H | 2C,7G |
| Sugar beet | 2C,6G | 5C,9G | 5C,9G | 0 |
| Cotton | 2G | 2C,5G | 4C,8H | 0 |
| PREEMERGENCE | | | | |
| Morningglory | 0 | 3H | 3C,6G | 0 |
| Cocklebur | 0 | 5H | - | 0 |
| Velvetleaf | 0 | 5G | 5G | 0 |
| Nutsedge | 0 | 5G | 10E | 0 |
| Crabgrass | 0 | 0 | 2G | 0 |
| Barnyardgrass | 0 | 0 | 4G | 0 |
| Cheatgrass | 0 | 2G | 8G | 0 |
| Wild Oats | 0 | 2C,5G | 3C,8G | 0 |
| Wheat | 0 | 0 | 2G | 0 |
| Corn | 0 | 2C,5G | 3C,6G | 3G |
| Soybean | 0 | 1C | 2C | 0 |
| Rice | 0 | 2C,5G | 3C,8G | 3G |
| Sorghum | 0 | 2C,4G | 3C,7G | 2G |
| Sugar beet | 0 | 3C,8G | 3C,7G | 0 |
| Cotton | 0 | 6G | 4G | 0 |

## Table A (continued)

| | Cmpd. 59 | Cmpd. 60 | Cmpd. 61 |
|---|---|---|---|
| Rate kg/ha | 0.05 | 0.05 | 0.05 |
| POSTEMERGENCE | | | |
| Morningglory | 3G | 2C,5G | 9C |
| Cocklebur | 2C,7G | 9C | 10C |
| Velvetleaf | 0 | 4C,9G | 9C |
| Nutsedge | 9G | 4C,9G | 4C,9G |
| Crabgrass | 0 | 0 | 4G |
| Barnyardgrass | 2C,7H | 1C,4H | 4C,9H |
| Cheatgrass | 4G | 8G | 9C |
| Wild Oats | 0 | 10C | 4C,8G |
| Wheat | 0 | 5G | 4G |
| Corn | 2H | 5G | 5C,9G |
| Soybean | 1H | 4C,9G | 4C,9G |
| Rice | 0 | 9C | 9C |
| Sorghum | 3C,7H | 4C,9H | 4C,9H |
| Sugar beet | 2H | 9C | 9C |
| Cotton | 0 | 4C,9H | 4C,9G |
| PREEMERGENCE | | | |
| Morningglory | 0 | 1C | 8H |
| Cocklebur | - | - | 9H |
| Velvetleaf | 0 | 0 | 9C |
| Nutsedge | 0 | 0 | 3C,8G |
| Crabgrass | 0 | 0 | 2C |
| Barnyardgrass | 0 | 0 | 5C,9H |
| Cheatgrass | 0 | 8G | 10H |
| Wild Oats | 0 | 2C,7H | 5C,9G |
| Wheat | 0 | 0 | 7G |
| Corn | 0 | 5G | 5C,9H |
| Soybean | 0 | 1C | 4C,8H |
| Rice | 0 | 3C,8G | 10E |
| Sorghum | 0 | 3C,7G | 10H |
| Sugar beet | 0 | 8G | 9G |
| Cotton | 0 | 2G | 9G |

Table A (continued)

| | Cmpd. 62 | Cmpd. 63 | Cmpd. 64 | Cmpd. 65 |
|---|---|---|---|---|
| Rate kg/ha | 0.05 | 0.05 | 0.05 | 0.05 |
| POSTEMERGENCE | | | | |
| Morningglory | 10C | 5C,9G | 3C,9H | 4C,8G |
| Cocklebur | 10C | 4C,9H | 3G | 5C,9G |
| Velvetleaf | 10C | 3C,8G | 4C,9G | 2C,6G |
| Nutsedge | 5C,9G | 3C,7G | 0 | 4C,9G |
| Crabgrass | 7G | 2C,5G | 2G | 0 |
| Barnyardgrass | 9C | 9C | 9C | 3C,8H |
| Cheatgrass | 9C | 4C,9G | 8G | 5G |
| Wild Oats | 10C | 9C | 9C | 0 |
| Wheat | 4G | 9G | 9G | 0 |
| Corn | 9C | 10C | 9C | 4G |
| Soybean | 5C,9G | 5C,9G | 5C,9G | 3C,7G |
| Rice | 9C | 9C | 9C | 4C,9G |
| Sorghum | 9C | 9C | 4C,9G | 9H |
| Sugar beet | 9C | 9C | 9C | 3C,6G |
| Cotton | 4C,9G | 4C,9H | 4C,9H | 4C,9G |
| PREEMERGENCE | | | | |
| Morningglory | 9G | 10E | 7H | 7G |
| Cocklebur | 9H | 3G | 6H | - |
| Velvetleaf | 5C,9G | 2C | 1C | 2G |
| Nutsedge | 9G | 3C,5G | 0 | 8G |
| Crabgrass | 4C,5G | 2C | 0 | 0 |
| Barnyardgrass | 9H | 4C,8H | 0 | 3C,8H |
| Cheatgrass | 10H | 8G | 2G | 2C,8G |
| Wild Oats | 9G | 9C | 2C,4G | 0 |
| Wheat | 9G | 3C,9H | 2C,5G | 0 |
| Corn | 9G | 4C,9G | 3C,9G | 5G |
| Soybean | 9H | 2C,4G | 1C | 1C |
| Rice | 10E | 9H | 9H | 9H |
| Sorghum | 5C,9H | 4C,9H | 3C,8H | 4C,9H |
| Sugar beet | 9C | 3C,8G | 8G | 8G |
| Cotton | 8G | 4G | 3C,3H | 6G |

Table A (continued)

| | Cmpd. 66 | Cmpd. 67 | Cmpd. 68 | Cmpd. 69 |
|---|---|---|---|---|
| Rate kg/ha | 0.05 | 0.05 | 0.05 | 0.05 |
| POSTEMERGENCE | | | | |
| Morningglory | 0 | 4C,9G | 10C | 5G |
| Cocklebur | 2G | 7H | 7G | 2G |
| Velvetleaf | 2G | 8G | 4C,9G | 0 |
| Nutsedge | 5G | 3C,8G | 4C,8G | 2G |
| Crabgrass | 0 | 2C,3G | 3C,8H | 0 |
| Barnyardgrass | 2H | 3C,8H | 5C,9H | 0 |
| Cheatgrass | 3C,7G | 2C,8G | 8G | 4G |
| Wild Oats | 3C,7G | 2C,3G | 3C,5G | 2C,8G |
| Wheat | 0 | 0 | 3G | 0 |
| Corn | 2C,3H | 4C,9H | 2C,9H | 3C,9H |
| Soybean | 3C,5G | 4C,9H | 9C | 2C,8G |
| Rice | 3C,7G | 5C,9G | 4C,9G | 6G |
| Sorghum | 3C,5G | 3C,9H | 4C,9H | 3C,8H |
| Sugar beet | 10C | 10C | 10C | 3C,6G |
| Cotton | 3C,7H | 3C,9H | 9H | 0 |
| PREEMERGENCE | | | | |
| Morningglory | 0 | 4G | 8G | 0 |
| Cocklebur | 0 | 0 | 7G | 2G |
| Velvetleaf | 0 | 4G | 7G | 0 |
| Nutsedge | 0 | 0 | 0 | 0 |
| Crabgrass | 0 | 4G | 5G | 0 |
| Barnyardgrass | 0 | 3C,7G | 3C,8H | 0 |
| Cheatgrass | 0 | 3C,8G | 9H | 0 |
| Wild Oats | 0 | 6G | 2C,6G | 0 |
| Wheat | 0 | 0 | 2G | 0 |
| Corn | 0 | 3C,7G | 3C,8G | 0 |
| Soybean | 0 | 3C,7H | 2C,7H | 0 |
| Rice | 0 | 2C,8H | 3C,9H | 0 |
| Sorghum | 0 | 3C,7G | 3C,8H | 0 |
| Sugar beet | 5G | 3C,9G | 7G | 0 |
| Cotton | 0 | 8G | 8G | 0 |

Table A (continued)

| | Cmpd. 70 | Cmpd. 71 | Cmpd. 72 | Cmpd. 73 |
|---|---|---|---|---|
| Rate kg/ha | 0.05 | 0.05 | 0.05 | 0.05 |
| POSTEMERGENCE | | | | |
| Morningglory | 2C,8H | 2C,5G | 10C | 10C |
| Cocklebur | 2H | 2C,6H | 10C | 9C |
| Velvetleaf | 3C,8H | 2C,5G | 9C | 9C |
| Nutsedge | 0 | 0 | 2C,8G | 2C,8G |
| Crabgrass | 0 | 0 | 2G | 0 |
| Barnyardgrass | 0 | 0 | 8H | 3C,9H |
| Cheatgrass | 0 | 0 | 3C,6G | 8G |
| Wild Oats | 0 | 0 | 3G | 3C,5G |
| Wheat | 0 | 0 | 2G | 2C,5G |
| Corn | 2C,6H | 0 | 2C,9H | 4C,9H |
| Soybean | 2C,9G | 2C,8H | 2C,9G | 9C |
| Rice | 3G | 7G | 2C,9G | 5C,9G |
| Sorghum | 0 | 2C,5G | 9H | 4C,9H |
| Sugar beet | 9C | 1H | 2C,5H | 3C,8H |
| Cotton | 3C,8H | 7G | 4C,9H | 9C |
| PREEMERGENCE | | | | |
| Morningglory | 0 | 0 | 8H | 9H |
| Cocklebur | 6G | 0 | 9H | 9H |
| Velvetleaf | 5G | 0 | 9C | 10C |
| Nutsedge | 0 | 0 | 5G | 4C,9G |
| Crabgrass | 0 | 0 | 0 | 3G |
| Barnyardgrass | 0 | 2G | 3C,9H | 3C,5G |
| Cheatgrass | 0 | 0 | 8G | 9G |
| Wild Oats | 0 | 0 | 3C,8H | 8G |
| Wheat | 0 | 0 | 6G | 7G |
| Corn | 3G | 0 | 3C,9H | 3C,9G |
| Soybean | 2C,1H | 0 | 3C,7H | 8H |
| Rice | 2C,3G | 0 | 3C,9H | 3C,9H |
| Sorghum | 0 | 0 | 3C,8H | 9C |
| Sugar beet | 6G | 5G | 9G | 7G |
| Cotton | 0 | 0 | 2C,8H | 7G |

Table A (continued)

| | Cmpd. 74 | Cmpd. 75 | Cmpd. 76 | Cmpd. 77 |
|---|---|---|---|---|
| Rate kg/ha | 0.05 | 0.05 | 0.05 | 0.05 |
| POSTEMERGENCE | | | | |
| Morningglory | 2C,7G | 9C | 10C | 3C,7H |
| Cocklebur | 10C | 9C | 9C | 3C,5G |
| Velvetleaf | 2C,8G | 9C | 9C | 3C,4G |
| Nutsedge | 2G | 5G | 4G | 0 |
| Crabgrass | 0 | 3G | 0 | 0 |
| Barnyardgrass | 0 | 3C,8H | 7H | 0 |
| Cheatgrass | 0 | 4G | 0 | 0 |
| Wild Oats | 0 | 2G | 0 | 0 |
| Wheat | 0 | 3G | 0 | 0 |
| Corn | 2G | 3C,8H | 3C,9H | 0 |
| Soybean | 3H,7G | 5C,9G | 5C,9G | 3C,8H |
| Rice | 7G | 5C,9G | 5C,9G | 7G |
| Sorghum | 5G | 3C,9H | 4C,9H | 2C,5G |
| Sugar beet | 0 | 10C | 9C | 3C,5G |
| Cotton | 8G | 4C,9G | 5C,9G | 3C,6H |
| PREEMERGENCE | | | | |
| Morningglory | 8G | 9G | 9G | 0 |
| Cocklebur | 5G | - | 9H | 0 |
| Velvetleaf | 5G | 8G | 9C | 0 |
| Nutsedge | 0 | 0 | 0 | 0 |
| Crabgrass | 0 | 0 | 0 | 0 |
| Barnyardgrass | 0 | 8H | 0 | 0 |
| Cheatgrass | 0 | 3G | 0 | 0 |
| Wild Oats | 0 | 2C,5G | 0 | 0 |
| Wheat | 0 | 0 | 0 | 0 |
| Corn | 0 | 2C,7G | 2C,7G | 0 |
| Soybean | 0 | 9H | 3C,8H | 2C |
| Rice | 5G | 3C,7G | 2C,8G | 0 |
| Sorghum | 2G | 10H | 3C,9H | 3C |
| Sugar beet | 8G | 9C | 4C,9G | 5G |
| Cotton | 5G | 4C,8H | 8G | 0 |

Table A (continued)

| | Cmpd. 78 | Cmpd. 79 | Cmpd. 80 | Cmpd. 81 |
|---|---|---|---|---|
| Rate kg/ha | 0.05 | 0.05 | 0.05 | 0.05 |
| POSTEMERGENCE | | | | |
| Morningglory | 2C,5G | 5C,9G | 3C,9H | 3C,8G |
| Cocklebur | 3C,4G | 5C,9G | 10C | 5C,9G |
| Velvetleaf | 3C,3G | 4C,8H | 10C | 5G |
| Nutsedge | 0 | 3C,6G | 5C,9G | 5G |
| Crabgrass | 0 | 0 | 0 | 0 |
| Barnyardgrass | 2H | 2C | 3C,9H | 0 |
| Cheatgrass | 0 | 3G | 2C,5G | 0 |
| Wild Oats | 0 | 0 | 2G | 0 |
| Wheat | 0 | 0 | 3G | 0 |
| Corn | 0 | 3C,7H | 9G | 3G |
| Soybean | 2C,5G | 4C,8H | 5C,9G | 2H,5G |
| Rice | 4G | 5G | 8G | 5G |
| Sorghum | 4G | 2C,4G | 3C,3H | 4G |
| Sugar beet | 2H | 2C,2H | 3C,8H | 0 |
| Cotton | 2C,3G | 3G | 5C,9G | 2G |
| PREEMERGENCE | | | | |
| Morningglory | 2C | 7H | 5G | 3C,3H |
| Cocklebur | 3H | 4C,4H | 9C | 3C,7H |
| Velvetleaf | 9C | 9C | 9C | 7G |
| Nutsedge | 0 | 2C | 5G | 0 |
| Crabgrass | 0 | 2G | 3G | 0 |
| Barnyardgrass | 2G | 3C | 3C,6G | 0 |
| Cheatgrass | 2G | 7G | 8G | 0 |
| Wild Oats | 0 | 2C,5G | 7G | 0 |
| Wheat | 0 | 4G | 5G | 0 |
| Corn | 0 | 3C,6H | 2C,8G | 0 |
| Soybean | 0 | 3C,4H | 4C,5H | 0 |
| Rice | 2C,3G | 3C,7H | 2C,7G | 5G |
| Sorghum | 0 | 3C,7H | 2C,8G | 2G |
| Sugar beet | 9C | 8H | 8G | 8G |
| Cotton | 0 | 6G | 5G | 2C |

Table A (continued)

| | Cmpd. 82 | Cmpd. 83 | Cmpd. 84 |
|---|---|---|---|
| Rate kg/ha | 0.05 | 0.05 | 0.05 |
| POSTEMERGENCE | | | |
| Morningglory | 4C,9G | 4C,9H | 3C,4H |
| Cocklebur | 6C,9G | 4C,9H | 2C |
| Velvetleaf | 4C,8H | 4C,8H | 0 |
| Nutsedge | 4G | 3G | 0 |
| Crabgrass | 4G | 0 | 0 |
| Barnyardgrass | 3C,7H | 3C,7H | 0 |
| Cheatgrass | 0 | 2G | 0 |
| Wild Oats | 3C,7G | 0 | 0 |
| Wheat | 0 | 0 | 0 |
| Corn | 3C,7H | 3C,6H | 0 |
| Soybean | 5C,9G | 5C,9G | 2C,2H |
| Rice | 4C,9G | 2C,8G | 2G |
| Sorghum | 4C,9H | 3C,9H | 2C,3H |
| Sugar beet | 5C,9G | 9C | 3C,5H |
| Cotton | 4C,8H | 3C,7H | 1C |
| PREEMERGENCE | | | |
| Morningglory | 4C,8H | 3C,5H | 0 |
| Cocklebur | 8H | 9H | 0 |
| Velvetleaf | 10E | 8G | 4G |
| Nutsedge | 7G | - | 0 |
| Crabgrass | 1C | 0 | 0 |
| Barnyardgrass | 3C,5G | 2C | 0 |
| Cheatgrass | 0 | 0 | 0 |
| Wild Oats | 2C,6G | 0 | 0 |
| Wheat | 0 | 0 | 0 |
| Corn | 2C,5G | 0 | 0 |
| Soybean | 3C,7G | 3C,7G | 3C,3G |
| Rice | 3C,6H | 3C,5G | 0 |
| Sorghum | 3C,9H | 3C,7H | 3G |
| Sugar beet | 4C,8G | 9C | 0 |
| Cotton | 6G | 5G | 0 |

Table A (continued)

| | Cmpd. 85 | Cmpd. 86 |
|---|---|---|
| Rate kg/ha | 0.05 | 0.05 |
| POSTEMERGENCE | | |
| Morningglory | 4C,8H | 10C |
| Cocklebur | 3C,9G | 9C |
| Velvetleaf | 3C,8G | 10C |
| Nutsedge | 3C,5G | 2C,8G |
| Crabgrass | 0 | 4G |
| Giant Foxtail | 0 | 4C,9G |
| Barnyardgrass | 0 | 9H |
| Cheatgrass | 0 | 5C,9G |
| Wild Oats | 0 | 2C,4G |
| Wheat | 0 | 6G |
| Corn | 0 | 3C,9H |
| Barley | 0 | 7G |
| Soybean | 4G | 9C |
| Rice | 7G | 9C |
| Sorghum | 8H | 9C |
| Sugar beet | 3C,7G | 10C |
| Cotton | 3C,7G | 10C |
| PREEMERGENCE | | |
| Morningglory | 8G | 9G |
| Cocklebur | 7G | 8H |
| Velvetleaf | 5G | 9G |
| Nutsedge | 0 | 8G |
| Crabgrass | 0 | 5G |
| Giant Foxtail | 2C | 9H |
| Barnyardgrass | 0 | 8H |
| Cheatgrass | 0 | 8G |
| Wild Oats | 0 | 8G |
| Wheat | 0 | 8G |
| Corn | 0 | 9G |
| Barley | 0 | 9G |
| Soybean | 0 | 7H |
| Rice | 4G | 9H |
| Sorghum | 2C,7G | 5C,9G |
| Sugar beet | 7G | 9C |
| Cotton | 5G | 8G |

Table A (continued)

| | Cmpd. 87 | Cmpd. 88 | Cmpd. 89 |
|---|---|---|---|
| Rate (kg/ha) | 0.05 | 0.05 | 0.05 |
| POSTEMERGENCE | | | |
| Morningglory | 1C,5G | 2C,3H | 9C |
| Cocklebur | 1H | 7G | 7G |
| Velvetleaf | 7G | 5C,8G | 9C |
| Nutsedge | 5G | 0 | 2C,9G |
| Crabgrass | 0 | 3G | 4G |
| Barnyardgrass | 9H | 3C,9H | 3C,8H |
| Cheatgrass | 5G | 6G | 4C,9G |
| Wild Oats | 0 | 3G | 6G |
| Wheat | 0 | 5G | 2G |
| Corn | 2C,8H | 4C,9G | 2C,8G |
| Soybean | 2C,6G | 9C | 9C |
| Rice | 2C,9G | 5C,9G | 6C,9G |
| Sorghum | 3C,9H | 3C,9H | 4C,9H |
| Sugar Beets | 5C,9G | 9C | 9C |
| Cotton | 2C,5G | 2C,6G | 2C,6G |
| PREEMERGENCE | | | |
| Morningglory | 4G | 7G | 7G |
| Cocklebur | 2G | 5G | - |
| Velvetleaf | 2G | 8G | 9G |
| Nutsedge | 0 | 10E | 8G |
| Crabgrass | 0 | 0 | 0 |
| Barnyardgrass | 0 | 2C,8H | 2C |
| Cheatgrass | 6G | 8G | 6G |
| Wild Oats | 0 | 2C,6G | 2C,6G |
| Wheat | 3G | 5G | 2C,6G |
| Corn | 2C,6G | 2C,8G | 5G |
| Soybean | 0 | 7G | 2C,5G |
| Rice | 5G | 8H | 3C,7H |
| Sorghum | 2C,3G | 2C,8H | 5G |
| Sugar Beets | 7G | 9G | 8G |
| Cotton | 5G | 8G | 7G |

Table A (continued)

| | Cmpd. 90 | | Cmpd. 91 | | Cmpd. 92 | |
|---|---|---|---|---|---|---|
| Rate (kg/ha) | 0.05 | 0.10 | 0.05 | 0.10 | 0.05 | 0.40 |
| POSTEMERGENCE | | | | | | |
| Morningglory | 2C,5G | 4C,9G | 3C,8H | 9C | 3C,8H | 4C,9G |
| Cocklebur | 5G | 6G | 8G | 2C,8G | 2G | 5G |
| Velvetleaf | 2G | 4G | 5C,9G | 10C | 1C,3G | 5C,8G |
| Nutsedge | 5G | 5G | 0 | 5G | 0 | 0 |
| Crabgrass | 0 | 0 | 0 | 5G | 0 | 2G |
| Barnyardgrass | 3H | 4H | 2H | 8H | 0 | 2H |
| Cheatgrass | 0 | 4G | 0 | 4G | 0 | 0 |
| Wild Oats | 0 | 0 | 0 | 0 | 0 | 0 |
| Wheat | 0 | 0 | 0 | 0 | 0 | 0 |
| Corn | 2H | 3H | 2C,9G | 2U,9G | 5H | 2U,9G |
| Soybean | 1H | 2H | 9C | 5C,9G | 2C,8G | 5C,9G |
| Rice | 4G | 2C,8G | 5G | 2C,9G | 0 | 6C,9G |
| Sorghum | 5H | 3G | 2C,6H | 3C,9H | 2G | 2C,7G |
| Sugar Beets | 3H | 3C,7H | 5C,9G | 9C | 4C,8H | 9C |
| Cotton | 2G | 5C,9G | 4C,8H | 5C,9G | 4C,6G | 4C,8G |
| PREEMERGENCE | | | | | | |
| Morningglory | 7H | 7G | 5G | 7G | 0 | 7G |
| Cocklebur | 5G | 7G | 2G | 6G | 0 | 7H |
| Velvetleaf | 4G | 5G | 2G | 5G | 0 | 7G |
| Nutsedge | 0 | 0 | 0 | 0 | 0 | 0 |
| Crabgrass | 0 | 0 | 0 | 0 | 0 | 0 |
| Barnyardgrass | 0 | 0 | 0 | 0 | 0 | 0 |
| Cheatgrass | 0 | 0 | 0 | 0 | 0 | 0 |
| Wild Oats | 0 | 0 | 0 | 0 | 0 | 0 |
| Wheat | 0 | 0 | 0 | 0 | 0 | 0 |
| Corn | 3G | 2C,7G | 2C,5G | 8G | 0 | 2C,8G |
| Soybean | 0 | 2G | 2C,5G | 6G | 0 | 5G |
| Rice | 3G | 6G | 0 | 5G | 0 | 6G |
| Sorghum | 2G | 3G | 4G | 2C,7G | 0 | 3C,7G |
| Sugar Beets | 4G | 8G | 3C,7G | 4C,9G | 6G | 4C,8G |
| Cotton | 3G | 7G | 8G | 7G | 4G | 2C,7G |

## Claims

1. A compound of the formula:

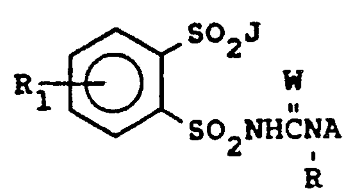

(I)

and agriculturally suitable salts thereof,

characterized in that:
J is $NHR_2$ $NR_3R_4$ or $NR_2'$ $NR_3'$ $R_4'$ ;
W is O or S;
R is H or $CH_3$;
$R_1$ is H, $C_1$ to $C_3$ alkyl, $C_1$ to $C_3$ haloalkyl, halogen. nitro. $C_1$ to $C_3$ alkoxy. $SO_2NR_aR_b$, $C_1$ to $C_3$ alkylthio, $C_1$ to $C_3$ alkylsulfinyl, $C_1$ to $C_3$ alkylsulfonyl, CN, $CO_2R_c$, $C_1$ to $C_3$ haloalkoxy, $C_1$ to $C_3$ haloalkylthio, $C_2$ to $C_3$ alkoxyalkyl, $C_2$ to $C_3$ haloalkoxyalkyl, $C_2$ to $C_3$ alkylthioalkyl, $C_2$ to $C_3$ haloalkylthioalkyl, $C_2$ to $C_3$ cyanoalkyl or $NR_dR_e$;
$R_a$ is H $C_1$ to $C_4$ alkyl, $C_2$ to $C_3$ cyanoalkyl, methoxy or ethoxy;
$R_b$ is H, $C_1$ to $C_4$ alkyl or $C_3$ to $C_4$ alkenyl; or
$R_a$ and $R_b$ can be taken together as $-(CH_2)_3-$, $-(CH_2)_4-$, $-(CH_2)_5-$ or $-CH_2CH_2OCH_2cCH_2-$;
$R_c$ is $C_1$ to $C_4$ alkyl, $C_3$ to $C_4$ alkenyl, $C_3$ to $C_4$ alkynyl. $C_2$ to $C_4$ haloalkyl, $C_2$ to $C_3$ cyanoalkyl. $C_5$ to $C_6$ cycloalkyl, $C_4$ to $C_7$ cycloalkylalkyl or $C_2$ to $C_4$ alkoxyalkyl;
$R_d$ and $R_e$ are independently H or $C_1$ to $C_2$ alkyl;
$R_2$ is $C_5$ to $C_6$ alkyl, $C_5$ to $C_6$ alkenyl, $C_2$ to $C_6$ haloalkenyl, $C_3$ to $c_6$ alkynyl, $C_3$ to $C_6$ cycloalkyl, $C_3$ to $C_6$ cycloalkyl optionally substituted with 1 or 2 $CH_3$ groups, $C_4$ to $C_7$ cycloalkylalkyl, $C_5$ to $C_6$ cycloalkenyl, $c_3$ to $C_6$ epoxyalkyl, $C_2$ to $C_6$ haloalkyl, $CH_2CH_2(OR_5)_2$,

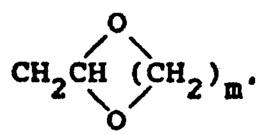

$(CH_2)_3OCH_3$, phenyl which can be optionally substituted with $R_7$,

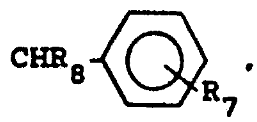

$CH_2C(O)CH_3$, CN, $OR_6$, $C_1$ to $C_6$ alkyl substituted with $OR_9$, $S(O)_nR_{10}$ or $NR_{11}R_{12}$, Q, $CHR_8Q$ or $CH_2CH_2Q$;
$R_3$ is $C_1$ to $C_6$ alkyl, $C_3$ to $C_6$ alkenyl, $C_3$ to $C_6$ $R_3$ is $C_1$ to $C_6$ alkyl, $C_3$ to $C_6$ alkenyl, $C_3$ to $C_6$ alkynyl, $C_3$ to $C_6$ cycloalkyl which can be optionally substituted with 1 or 2 $CH_3$ groups, $C_4$ to $C_7$ cycloalkylalkyl, $C_5$ to $C_6$ cycloalkenyl, $C_1$ to $C_6$ haloalkyl, $C_1$ to $C_5$ alkoxy, $C_3$ to $C_6$ epoxyalkyl or $C_1$ to $c_6$ alkyl substituted with $OR_9$, $S(O)_nR_{10}$, $NR_{11}R_{12}$ or $P(O)(OR_5)_2$;
$R_4$ is $C_1$ to $C_3$ alkyl substituted with 1 to 3 atoms of F, Cl or Br, $C_3$ to $C_4$ alkynyl, $CH_2CH_2(OR_5)_2$,

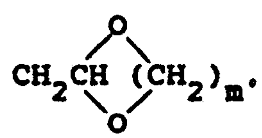

$C_2$ to $C_6$ haloalkenyl, $C_3$ to $C_6$ epoxyalkyl, $CH_2C(O)CH_3$, CN, $C_1$ to $C_6$ alkyl substituted with $OR_9$, $S(O)_nR_{10}$ or $NR_{11}R_{12}$, Q, $CHR_8Q$ or $CH_2CH_2Q$;
$R_3$ and $R_4$ can be taken together with the sulfonamide nitrogen to form a saturated 5- or 6-membered ring substituted by one or more groups selected from L or a partially saturated 5- or 6-membered ring optionally substituted by one or more groups selected from L;
$R_5$ is $C_1$ to $C_3$ alkyl;
$R_6$ is $C_6$ to $C_5$ alkyl;
$R_7$ is H, $C_1$ to $C_3$ alkyl, halogen, $NO_2$, $CF_3$, CN or $C_1$ to $C_3$ alkoxy;
$R_8$ is H or $CH_3$;
$R_9$ is H, $SO_2R_5$, $C(O)R_5$, $co_2R_5$, $C(O)NR_{11}R_{12}$ or $P(O)(OR_5)_2$;
$R_{10}$ is $C_1$ to $C_3$ alkyl;
$R_{11}$ is H or $C_1$ to $C_3$ alkyl;
$R_{12}$ is H or $C_1$ to $C_3$ alkyl;
m is 2 or 3;

n is 0, 1 or 2;

$R_2'$ , $R_3'$ and $R_4'$ are independently H, $C_1$ to $C_4$ alkyl, $C_3$ to $C_4$ alkenyl, $C_3$ to $C_4$ alkynyl, $C_1$ to $C_4$ $C_3$ to $C_4$ alkynyl, $C_3$ to $C_4$ alkynyl, $C_1$ to $C_4$ haloalkyl, $C(O)R_5'$ , $CO_2R_6'$ , $C(O)NR_7'$ $R_8'$ , $C(S)NR_7'$ $R_8'$ , Q, CHRQ, $CH_2CH_2Q$, $C_2$ to $C_3$ alkyl substituted with $OR_9'$ , phenyl which can be optionally substituted with $R_{10}'$ and $R_{11}'$

or 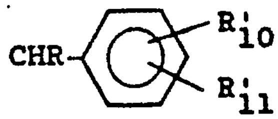 ;

$R_3'$ and $R_4'$ can be taken together to form $-(CH_2)_4-$, $-(CH_2)_5-$, $CH_2CH_2OCH_2CH_2$, $CH=CHCH=CH$, $CH=N-N=CH$

or 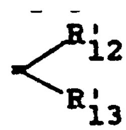 ;

$R_5'$ is $C_1$ to $C_3$ alkyl or phenyl which can be optionally substituted with $R_{10}'$ and $R_{11}'$ ;

$R_6'$ is $C_1$ to $C_3$ alkyl;

$R_7'$ and $R_8'$ are independently H or $C_1$ to $C_3$ alkyl;

$R_9'$ is H, $SO_2R_6'$ , $C(O)R_6'$ , $CO_2R_6'$ , $C(O)NR_7'$ $R_8'$ , $C_1$ to $C_3$ alkyl, $C_1$ to $C_3$ haloalkyl or $P(O)(OR_6')_2$;

$R_{10}'$ and $R_{11}'$ are independently H, $C_1$ to $C_3$ alkyl, Cl, F, Br, $NO_2$, $CF_3$, CN or $C_1$ to $C_3$ alkoxy;

$R_{12}'$ and $R_{13}'$ are independently H, $C_1$ to $C_3$ alkyl, phenyl which can optionally substituted with $R_{10}'$ and $R_{11}'$

or 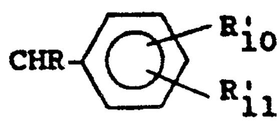 ;

$R_{12}'$ and $R_{13}'$ can be taken together to form $-(CH_2)_4-$or $-(CH_2)_5-$;

Q is a saturated 5- or 6-membered ring which is bonded through a carbon atom and contains 1 heteroatom selected from oxygen, sulfur, or nitrogen or an unsaturated or partially unsaturated 5- or 6-membered ring which is bonded through a carbon atom and contains 1 to 3 heteroatoms selected from 1 sulfur, 1 oxygen or 1 to 3 nitrogen: and Q can be optionally substituted by one or more groups selected from L;

L is $C_1$ to $C_4$ alkyl, $C_1$ to $C_3$ haloalkyl, halogen, $C_1$ to $C_3$ alkoxy, $C_1$ to $C_3$ alkylthio, $C_3$ to $C_4$ alkenyloxy. $C_3$ to $C_4$ alkenylthio, $C_1$ to $C_2$ haloalkoxy or $C_1$ to $C_2$ haloalkylthio;

A is 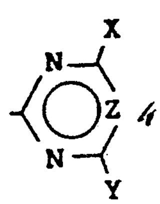

X is H, $C_1$ to $C_4$ alkyl, $C_1$ to $C_4$ alkoxy, $C_1$ to $C_4$ haloalkoxy, $C_1$ to $C_4$ haloalkyl, $C_1$ to $C_4$ haloalkylthio, $C_1$ to $C_4$ alkylthio, halogen, $C_2$ to $C_5$ alkoxyalkyl, $C_2$ to $C_5$ alkoxyalkoxy, amino, $C_1$ to $C_3$ alkylamino or di($C_1$ to $C_3$ alkyl)amino;

Y is H, $C_1$ to $C_4$ alkyl, $C_1$ to $C_4$ alkoxy, $C_1$ to $C_4$ haloalkoxy, $C_1$ to $C_4$ haloalkylthio, $C_1$ to $C_4$ alkylthio, $C_2$ to $C_5$ alkoxyalkyl, $C_2$ to $C_5$ alkoxyalkoxy, amino, $C_1$ to $C_3$ alkylamino, di($C_1$ to $C_3$ alkyl)amino, $C_3$ to $C_4$ alkenyloxy, $C_3$ to $C_4$ alkynyloxy, $C_2$ to $C_5$ alkylthioalkyl. $C_1$ to $C_4$ haloalkyl. $C_3$ to $C_5$ cycloalkyl. $C_2$ to $C_4$ alkynyl. or $N(OCH_3)CH_3$;

Z is CH or N;

characterized further in that:

a) when X is Cl, F, Br or I, then Z is CH and Y is $OCH_3$, $OC_2H_5$, $N(OCH_3)CH_3$, $NHCH_3$ or $N(CH_3)_2$ ;
b) the total number of carbon atoms in $R'_2$ , $R'_3$ and $R'_4$ does not exceed 10;
c) $R_2$ is not $CH_2CF_3$;
d) when $R_3$ or $R_4$ is $CH_2CF_3$, then the other is $CHF_2$, $CH_2CH_2F$, $CH_2CH_2Cl$, $CH_2CH_2Br$ or $CH_2CF_3$;
e) when $R_4$ is $CF_2H$, then $R_3$ is other than $C_1$ to $C_3$ alkyl;
f) when $R_4$ is $C_3$ to $C_4$ alkynyl, then $R_3$ is $CHF_2$, $CH_2CH_2F$, $CH_2CH_2Cl$, $CH_2CH_2Br$ or $C_3$ to $C_4$ alkynyl;
g) when $R_2$ is $OR_6$ and Z is CH, then one or both of X and Y is other than $C_1$ to $C_4$ alkyl; and
h) neither of X and Y is halomethoxy.

2. A compound according to Claim 1 wherein:
W is O;
J is $NHR_2$; and
$R_2$ is $C_5$ to $C_6$ alkyl, $C_5$ to $C_6$ alkenyl, $C_2$ to $C_6$ haloalkenyl, $C_3$ to $C_6$ alkynyl, $C_3$ to $C_6$ cycloalkyl, $C_3$ to $C_6$ cycloalkyl optionally substituted with 1 or 2 $CH_3$ groups, $C_4$ to $C_7$ cycloalkylalkyl, $C_5$ to $C_6$ cycloalkenyl, $C_3$ to $C_6$ epoxyalkyl, $C_2$ to $C_6$ haloalkyl, $CH_2CH_2(OR_5)_2$, $(CH_2)_3OCH_3$, $OR_6$, phenyl which can be optionally substituted with

$R_7$, 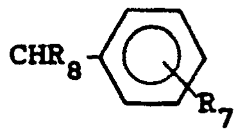 ,

$CH_2C(O)CH_3$, CN or $C_1$ to $C_6$ alkyl substituted with $OR_9$, $S(O)_nR_{10}$ or $NR_{11}R_{12}$.

3. A compound according to Claim 1 wherein:
W is O:
J is $NHR_2$; and
$R_2$ is Q, $CHR_8Q$ or $CH_2CH_2Q$.

4. A compound according to Claim 1 wherein:
W is O; and
J is $NR_3R_4$.

5. A compound according to Claim 1 wherein:
W is O:
J is $NR'_2$ $NR'_3$ $R'_4$ ;
R is H;
$R_1$ is H, $C_1$ to $C_2$ alkyl, F, Cl, Br, $NO_2$, $C_1$ to $C_2$ alkoxy, $C_1$ to $C_2$ alkylthio, $C_1$ to $C_2$ haloalkoxy, $CH_2OCH_3$, $CH_2SCH_3$ or $CH_2CN$ and is not para to the sulfonylurea bridge;
X is $CH_3$, $OCH_3$, $OCH_2CH_3$, Cl, F, Br, I, $CH_2F$, $OCH_2CH_2F$,. $OCH_2CHF_2$, $OCH_2CF_3$, $CF_3$, $CH_2Cl$ or $CH_2Br$;
Y is H, $C_1$ to $C_3$ alkyl, $OCH_3$, $OC_2H_5$, $CH_2OCH_3$, $NHCH_3$, $N(OCH_3)CH_3$, $N(CH_3)_2$, $CF_3$ $SCH_3$, $OCH_2CH=CH_2$, $OCH_2C{\equiv}CH$, $CH_2OCH_2CH_3$, $OCH_2CH_2OCH_3$, $CH_2SCH_3$, $SCF_2H$, cyclopropyl. $C{\equiv}CH$ or $C{\equiv}CCH_3$

6. A compound according to Claim 2 wherein:
R is H;
$R_1$ is H, $C_1$ to $C_2$ alkyl, F, Cl, Br, $NO_2$, $C_1$ to $C_2$ alkoxy, $C_1$ to $C_2$ alkylthio, $C_1$ to $C_2$ haloalkoxy, $CH_2OCH_3$, $CH_2SCH_3$ or $CH_2CN$ and is not para to the sulfonylureas bridge;
X is $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ or $CH_2Br$; and
Y is H, $C_1$ to $C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C{\equiv}CH$, $CH_2OCH_2CH_3$, $OCH_2CH_2OCH_3$, $CH_2SCH_3$, $SCF_2H$, cyclopropyl, $C{\equiv}CH$ or $C{\equiv}CCH_3$

7. A compound according to Claim 3 wherein:
R is H;

$R_1$ is H, $C_1$ to $C_2$ alkyl, F, Cl, Br, $NO_2$, $C_1$ to $C_2$ alkoxy, $C_1$ to $C_2$ alkylthio, $C_1$ to $C_2$ haloalkoxy, $CH_2OCH_3$, $CH_2SCH_3$ or $CH_2CN$ and is not para to the sulfonylurea bridge;
X is $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ or $CH_2Br$; and
Y is H, $C_1$ to $C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C\equiv CH$, $CH_2OCH_2CH_3$, $OCH_2CH_2OCH_3$, $CH_2SCH_3$, $SCF_2H$, cyclopropyl, $C\equiv CH$ or $C\equiv CCH_3$

**8.** A compound according to Claim 4 Wherein:
R is H;
$R_1$ is H, $C_1$ to $C_2$ alkyl, F, Cl, Br, $NO_2$, $C_1$ to $C_2$ alkoxy, $C_1$ to $C_2$ alkylthio, $C_1$ to $C_2$ haloalkoxy, $CH_2OCH_3$, $CH_2SCH_3$ or $CH_2CN$ and is not para to the sulfonylurea bridge;
X is $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ or $CH_2Br$; and
Y is H, $C_1$ to $C_3$ alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C\equiv CH$, $CH_2OCH_2CH_3$, $OCH_2CH_2OCH_3$, $CH_2SCH_3$, $SCF_2H$, cyclopropyl, $C\equiv CH$ or $C\equiv CCH_3$,

**9.** A compound according to Claim 5 wherein:
$R_2'$, $R_3'$ and $R_4'$ are independently H, $C_1$ to $C_3$ alkyl or phenyl provided that one of $R_2'$, $R_3'$ and $R_4'$ is H.

**10.** A compound according to Claim 6 wherein:
$R_1$ is H, Cl, $OCH_3$, $OCF_2H$, $CH_2OCH_3$ or $CH_2CN$;
$R_2$ is $C_3$ to $c_4$ alkynyl, $C_3$ to $C_6$ cycloalkyl, $C_2$ to $C_4$ haloalkenyl, $CH_2CH_2F$, $CH_2CH_2Cl$ or $CH_2CH_2Br$;
X is $CH_3$, $OCH_3$, $OCH_2CH_3$, Cl, or $OCH_2CF_3$; and
Y is $CH_3$, $OCH_3$, $CH_2CH_3$, $CH_2OCH_3$ or $NHCH_3$.

**11.** A compound according to claim 7 wherein;
$R_1$ is H, Cl, $OCH_3$, $OCF_2H$, $CH_2OCH_3$ or $CH_2CN$;
$R_2$ is Q or $CH_2Q$;
X is $CH_3$, $OCh_3$, $OCH_2CH_3$, Cl, or $OCH_2CF_3$;
and
Y is $CH_3$, $OCH_3$, $CH_2CH_3$, $CH_2OCH_3$ or $NHCH_3$,

**12.** A compound according to Claim 8 wherein:
$R_1$ is H, Cl, $OCH_3$, $OCF_2H$, $CH_2OCH_3$ or $CH_2CN$;
$R_3$ is $C_1$ to $C_3$ alkyl, $C_3$ to $c_4$ alkenyl, $C_3$ to $C_4$ alkynyl, $C_3$ to $C_6$ cycloalkyl or $C_1$ to $C_2$ haloalkyl;
$R_4$ is $C_2$ to $C_4$ haloalkenyl or $C_3$ to $C_4$ epoxyalkyl;
X is $CH_3$, $OCH_3$, $OCH_2CH_3$, CL, $OCH_2CF_3$;
and
Y is $CH_3$, $OCH_3$, $CH_2CH_3$, $CH_2OCH_3$ or $NHCH_3$.

**13.** A compound according to Claim 9 wherein:
$R_1$ is H, Cl, $OCH_3$, $OCF_2H$, $CH_2OCH_3$ or $CH_2CN$;
X is $CH_3$, $OCH_3$, $OCH_2CH_3$, Cl, or $OCH_2CF_3$;
and
Y is $CH_3$, $OCH_3$, $CH_2CH_3$, $CH_2OCH_3$ or $NHCH_3$,

**14.** A compound according to Claim 2, N-cyclopropyl-N'-[(4,6-dimethoxypyrimidin-2-yl)aminocarbonyl]-1,2-benzenedisulfonamide.

**15.** A compound according to Claim 2. N-cyclopropyl-N'-[(4-methoxy-6-methyl-1,3,5-triazin-2-yl)-aminocarbonyl]-1,2-benzenedisulfonamide.

**16.** A compound according to Claim 5, 2-(2,2-dimethylhydrazinosulfonyl)-N-[(4,6-dimethoxypyrimidin-2-yl)-aminocarbonyl]benzenesulfonamide.

**17.** A compound according to Claim 5, 2-(2,2-dimethylhydrazinsulfonyl)-N-[(4-methoxy-6-methyl-1,3,5-triazin-2-yl)aminocarbonyl]benzenesulfonamide.

**18.** A composition for the control of undesirable vegetation or for use as a plant growth regulant comprising a compound according to any of claims 1 to 17 and at least one of (i) a surface active agent and (ii) a solid or liquid diluent.

**19.** A method for controlling undesirable vegetation comprising applying to the locus of such vegetation an herbicidally effective amount of a compound according to any of claims 1 to 17.

**20.** A method for regulating the growth of plants comprising applying to the locus of such plants an effective but substantially non-phytotoxic amount of a plant growth regulant selected from compounds of any of claims 1 to 17.

**21.** A process for the preparation of a compound of claim 1 which comprises:
(a) reacting an isocyanate or isothiocyanate of formula

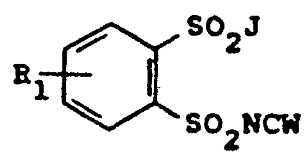

(II)

or a phenylcarbamate or phenylthiocarbamate of formula

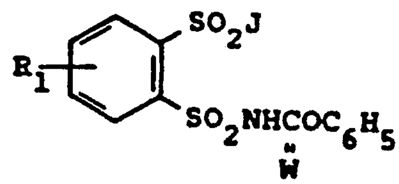

(VI)

with a heterocyclic amine of formula

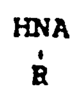

(III) ; or

(b) reacting a sulfonamide of formula

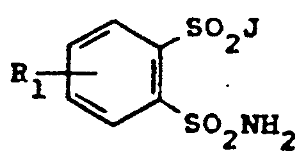

(IV)

with a heterocyclic phenylcarbamate or phenylthiocarbamate of formula

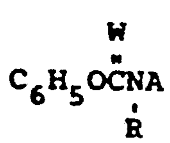

(VII)

wherein J, R, $R_1$, A and W are as defined in claim 1; or
(c) oxidising a compound of Formula (I) wherein $R_2$, $R_3$ or $R_4$ is $C_1$ to $C_6$ alkyl substituted with $SR_7$

and $R_1$ is other than $C_1$ to $C_3$ alkylthio, $C_1$ to $C_3$ alkylsulfinyl, $C_1$ to $C_3$ haloalkylthio, $C_2$ to $C_3$ alkylthioalkyl or $C_2$ to $C_3$ haloalkylthioalkyl, to oxidise said substituent $SR_7$ to $SOR_7$ or $SO_2R_7$; or
(d) transforming a compound of Formula (I) into another compound of Formula (I) by methods known per se .

**22.** Compounds of formulae (II), (IV) and (VI) as defined in claim 21.

## Revendications

**1.** Un composé de la formule :

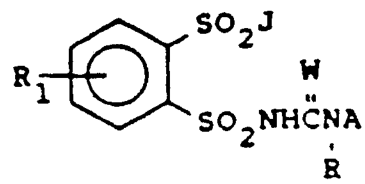

et ses sels utilisables en agriculture,
caractérisé en ce que :
J est $NHR_2$, $NR_3R_4$ ou $NR_2' NR_3' R_4'$ ;
W est O ou S ;
R H ou $CH_3$ ;
$R_1$ est H, un groupe alkyle en $C_1$ à $C_3$ , halogénalkyle en $C_1$ à $C_3$ , halogéno, nitro, alcoxy en $C_1$ à $C_3$ , $SO_2NR_aR_b$ , alkylthio en $C_1$ à $C_3$ , alkylsulfinyle en $C_1$ à $C_3$ , alkylsulfonyle en $C_1$ à $C_3$ , CN, $CO_2R_c$ , halogénalcoxy en $C_1$ à $C_3$ , halogénalkylthio en $C_1$ à $C_3$ , alcoxyalkyle en $C_2$ ou $C_3$ , halogénalcoxyalkyle en $C_2$ ou $C_3$ , alkylthioalkyle en $C_2$ ou $C_3$ , halogénalkylthioalkyle en $C_2$ ou $C_3$ , cyanalkyle en $C_2$ ou $C_3$ ou $NR_dR_e$ ;
$R_a$ est H, un groupe alkyle en $C_1$ à $C_4$ , cyanalkyle en $C_2$ ou $C_3$ , méthoxy ou éthoxy ;
$R_b$ est H, un groupe alkyle en $C_1$ à $C_4$ ou alcényle en $C_3$ ou $C_4$ ; ou bien $R_a$ et $R_b$ peuvent être pris ensemble sous la forme $-(CH_2)_3-$, $-(CH_2)_4-$, $-(CH_2)_5-$ ou $-CH_2CH_2OCH_2CH_2-$ ;
$R_c$ est un groupe alkyle en $C_1$ à $C_4$ , alcényle en $C_3$ ou $C_4$ , alcynyle en $C_3$ ou $C_4$ , halogénalkyle en $C_2$ à $C_4$ , cyanalkyle en $C_2$ ou $C_3$ , cycloalkyle en $C_5$ ou $C_6$, cycloalkylalkyle en $C_4$ à $C_7$ ou alcoxyalkyle en $C_2$ à $C_4$ ;
$R_d$ et $R_e$ sont indépendamment H ou un groupe alkyle en $C_1$ ou $C_2$ ;
$R_2$ est un groupe alkyle en $C_5$ ou $C_6$ , alcényle en $C_5$ ou $C_6$, halogénalcényle en $C_2$ à $C_6$, alcynyle en $C_3$ à $C_6$, cycloalkyle en $C_3$ à $C_6$ , cycloalkyle en $C_3$ à $C_6$ facultativement substitué par 1 ou 2 groupes $CH_3$ , cycloalkylalkyle en $C_4$ à $C_7$, cycloalcényle en $C_5$ ou $C_6$ , époxyalkyle en $C_3$ à $C_6$ , halogénalkyle en $C_2$ à $C_6$, $CH_2CH_2(OR_5)_2$,

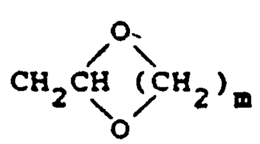 ,

$(CH_2)_3OCH_3$, phényle qui peut être facultativement substitué par $R_7$,

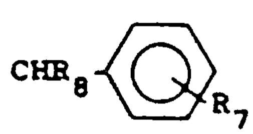 ,

$CH_2C(O)CH_3$, CN, $OR_6$ , alkyle en $C_1$ à $C_6$ substitué par $OR_9$, $S(O)_nR_{10}$ ou $NR_{11}R_{12}$, Q, $CHR_8Q$ ou $CH_2CH_2Q$ ;
$R_3$ est un groupe alkyle en $C_1$ à $C_6$, alcényle en $C_3$ à $C_6$ , alcynyle en $C_3$ à $C_6$, cycloalkyle en $C_3$ à $C_6$ qui peut être facultativement substitué par 1 ou 2 groupes $CH_3$ , cycloalkylalkyle en $C_4$ à $C_7$,

cycloalcényle en $C_5$ ou $C_6$, halogénalkyle en $C_1$ à $C_6$, alcoxy en $C_1$ à $C_5$, époxyalkyle en $C_3$ à $C_6$ ou alkyle en $C_1$ à $C_6$ substitué par $OR_9$ , $S(O)nR_{10}$ , $NR_{11}R_{12}$ ou $P(O)(OR_5)_2$ ;

$R_4$ est un groupe alkyle en $C_1$ à $C_3$ substitué par 1 à 3 atomes de F, Cl ou Br, alcynyle en $C_3$ ou $C_4$, $CH_2CH_2(OR_5)_2$,

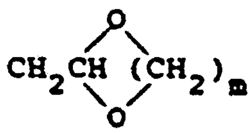

,

halogénalcényle en $C_2$ à $C_5$ , époxyalkyle en $C_3$ à $C_6$, $CH_2C(O)CH_3$ , CN, alkyle en $C_1$ à $C_6$ substitué par $OR_9$, $S(O)_nR_{10}$ ou $NR_{11}R_{12}$, Q, $CHR_8Q$ ou $CH_2CH_2Q$ ;

$R_3$ et $R_4$ peuvent être pris avec 1'azote de sulfonamide pour former un cycle penta- ou hexagonal saturé substitué par un ou plusieurs groupes choisis parmi L ou un cycle penta- ou hexagonal partiellement saturé facultativement substitué par un ou plusieurs groupes choisis parmi L ;

$R_5$ est un groupe alkyle en $C_1$ a $C_3$ ;

$R_6$ est un groupe alkyle en $C_1$ à $C_5$ ;

$R_7$ est H, un groupe alkyle en $C_1$ à $C_3$, halogéno, $NO_2$, $CF_3$, CN ou alcoxy en $C_1$ à $C_3$ ;

$R_8$ est H ou $CH_3$ ;

$R_9$ est H, $SO_2R_5$, $C(O)R_5$, $CO_2R_5$, $C(O)NR_{11}R_{12}$ ou $P(O)(OR_5)_2$ ;

$R_{10}$ est un groupe alkyle en $C_1$ à $C_3$ ;

$R_{11}$ est H ou un groupe alkyle en $C_1$ à $C_3$ ;

$R_{12}$ est H ou un groupe alkyle en $C_1$ à $C_3$ ;

m est 2 ou 3 ;

n est 0, 1 ou 2 ;

$R_2'$ , $R_3'$ et $R_4'$ sont indépendamment H, un groupe alkyle en $C_1$ à $C_4$, alcényle en $C_3$ ou $C_4$, alcynyle en $C_3$ ou $C_4$, halogénalkyle en $C_1$ à $C_4$, $C(O)R_5'$ , $CO_2R_6'$ , $C(O)NR_7'$ $R_8'$ , $C(S)NR_7'$ $R_8'$ , Q, CHRQ, $CH_2CH_2Q$, alkyle en $C_2$ ou $C_3$ substitué par $OR_9'$ , phényle qui peut être facultativement substitué par $R_{10}'$ et $R_{11}'$ ou

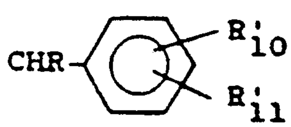

;

$R_3'$ et $R_4'$ peuvent être pris ensemble pour former $-(CH_2)_4-$, $-(CH_2)_5-$, $CH_2CH_2OCH_2CH_2$, CH = CHCH = CH, CH = N-N = CH ou

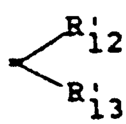

;

$R_5'$ est un groupe alkyle en $C_1$ à $C_3$ ou phényle qui peut être facultativement substitué par $R_{10}'$ et $R_{11}'$ ;

$R_6'$ est un groupe alkyle en $C_1$ à $C_3$ ;

$R_7'$ et $R_8'$ sont indépendamment H ou un groupe alkyle en $C_1$ à $C_3$ ;

$R_9'$ est H, $SO_2R_6'$ , $C(O)R_6'$ , $CO_2R_6'$ , $CO_2R_6'$ , $C(O)NR_7'$ $R_8'$ , un groupe alkyle en $C_1$ à $C_3$, halogénalkyle en $C_1$ à $C_3$ ou $P(O)(OR_6')_2$ ;

$R_{10}'$ et $R_{11}'$ sont indépendamment H, un groupe alkyle en $C_1$ à $C_3$, Cl, F, Br, $NO_2$, $CF_3$, CN ou alcoxy en $C_1$ à $C_3$ ;

$R_{12}'$ et $R_{13}'$ sont indépendamment H, un groupe alkyle en $C_1$ à $C_3$, phényle qui peut être facultativement substitué par $R_{10}'$ et $R_{11}'$ ou

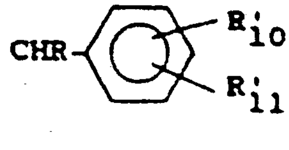

;

$R'_{12}$ et $R'_{13}$ peuvent être pris ensemble pour former $-(CH_2)_4-$ ou $-(CH_2)_5-$ ;

Q est un cycle penta- ou hexagonal saturé qui est lié par un atome de carbone et contient 1 hétéroatome choisi parmi l'oxygène, le soufre et l'azote, ou un cycle penta- ou hexagonal insaturé ou partiellement insaturé qui est lié par un atome de carbone et contient 1 à 3 hétéroatomes choisis parmi 1 atome de soufre, 1 atome d'oxygène et 1 à 3 atomes d'azote ; et Q peut être facultativement substitué par un ou plusieurs groupes choisis parmi L ;

L est un groupe alkyle en $C_1$ à $C_4$, halogénalkyle en $C_1$ à $C_3$, halogéno, alcoxy en $C_1$ à $C_3$, alkylthio en $C_1$ à $C_3$, alcényloxy en $C_3$ ou $C_4$, alcénylthio en $C_3$ ou $C_4$, halogénalcoxy en $C_1$ ou $C_2$ ou halogénoalkylthio en $C_1$ ou $C_2$ ,

A

est 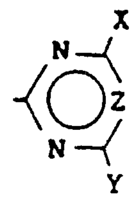

X est H, un groupe alkyle en $C_1$ à $C_4$, alcoxy en $C_1$ à $C_4$ , halogénalcoxy en $C_1$ à $C_4$ , halogénalkyle en $C_1$ à $C_4$, halogénalkylthio en $C_1$ à $C_4$, alkylthio en $C_1$ à $C_4$, halogéno, alcoxyalkyle en $C_2$ à $C_5$, alcoxyalcoxy en $C_2$ à $C_5$, amino, alkylamino en $C_1$ à $C_3$ ou di(alkyle en $C_1$ à $C_3$ )amino ;

Y est H, un groupe alkyle en $C_1$ à $C_4$, alcoxy en $C_1$ à $C_4$ , halogénalcoxy en $C_1$ à $C_4$ , halogénalkylthio en $C_1$ à $C_4$, alkylthio en $C_1$ à $C_4$, alcoxyalkyle en $C_2$ à $C_5$, alcoxyalcoxy en $C_2$ à $C_5$, amino, alkylamino en $C_1$ à $C_3$, di(alkyle en $C_1$ à $C_3$ )amino, alcényloxy en $C_3$ ou $C_4$, alcynyloxy en $C_3$ ou $C_4$, alkylthioalkyle en $C_2$ à $C_5$, halogénalkyle en $C_1$ à $C_4$, cycloalkyle en $C_3$ à $C_5$, alcynyle en $C_2$ à $C_4$ ou $N(OCH_3)CH_3$ ;

Z est CH ou M ;

caractérisé de plus en ce que :

a) si X est Cl, F, Br ou I, alors Z est CH et Y est $OCH_3$ $OC_2H_5$ , $N(OCH_3)CH_3$, $NHCH_3$ ou $N(CH_3)_2$ ;

b) le nombre total d'atomes de carbone dans $R'_2$ , $R'_3$ et $R'_4$ ne dépasse pas 10 ;

c) $R_2$ n'est pas $CH_2CF_3$ ;

d) si $R_3$ ou $R_4$ est $CH_2CF_3$, alors l'autre est $CHF_2$, $CH_2CH_2F$, $CH_2CH_2Cl$, $CH_2CH_2Br$ ou $CH_2CF_3$ ;

e) si $R_4$ est $CF_2H$, alors $R_3$ est autre chose qu'un groupe alkyle en $C_1$ à $C_3$ ;

f) si $R_4$ est un groupe alcynyle en $C_3$ ou $C_4$, alors $R_3$ est $CHF_2$, $CH_2CH_2F$, $CH_2CH_2Cl$, $CH_2CH_2Br$ ou un groupe alcynyle en $C_3$ ou $C_4$ ;

g) si $R_2$ est $OR_6$ et Z est CH, alors l'un ou chacun de X et Y est autre chose qu'un groupe alkyle en $C_1$ à $C_4$ ; et

h) ni X ni Y n'est un groupe halogénométhoxy.

**2.** Un composé selon la revendication 1, dans lequel :

W est O ;

J est $NHR_2$ ; et

$R_2$ est un groupe alkyle en $C_5$ ou $C_6$, alcényle en $C_5$ ou $C_6$, halogénalcényle en $C_2$ à $C_6$, alcynyle en $C_3$ à $C_6$ , cycloalkyle en $C_3$ à $C_6$ , cycloalkyle en $C_1$ à $C_6$ facultativement substitué par 1 ou 2 groupes $CH_3$ , cycloalkylalkyle en $C_4$ à $C_7$, cycloalcényle en $C_5$ ou $C_6$ , époxyalkyle en $C_3$ à $C_6$, halogénalkyle en $C_2$ à $C_6$, $CH_2CH_2(OR_5)_2$, $(CH_2)_3OCH_3$, $OR_6$, phényle qui peut être facultativement substitué par $R_7$,

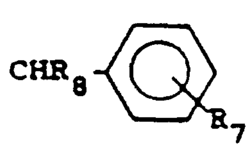 ,

$CH_2C(O)CH_3$, CN ou alkyle en $C_1$ à $C_6$ substitué par $OR_9$, $S(O)_nR_{10}$ ou $NR_{11}R_{12}$.

**3.** Un composé selon la revendication 1, dans lequel :

W est O ;

J est $NHR_2$ ; et

$R_2$ est Q, $CHR_8Q$ ou $CH_2CH_2Q$.

**4.** Un composé selon la revendication 1, dans lequel :
W est O ; et
J est $NR_3R_4$.

**5.** Un composé selon la revendication 1, dans lequel :
W est O ;
J est $NR_2' NR_3' R_4'$ ;
R est H ;
$R_1$ est H, un groupe alkyle en $C_1$ ou $C_2$, F, Cl, Br, $NO_2$, alcoxy en $C_1$ ou $C_2$, alkylthio en $C_1$ ou $C_2$, halogénalcoxy en $C_1$ ou $C_2$, $CH_2OCH_3$, $CH_2SCH_3$ ou $CH_2CN$ et n'est pas en position para par rapport au pont de sulfonylurée ;
X est $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ ou $CH_2Br$ ;
Y est H, un groupe alkyle en $C_1$ à $C_3$, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C≡CH$, $CH_2OCH_2CH_3$, $OCH_2CH_2OCH_3$, $CH_2SCH_3$, $SCF_2H$, cyclopropyle, $C≡CH$ ou $C≡CCH_3$ .

**6.** Un composé selon la revendication 2, dans lequel :
R est H ;
$R_1$ est H, un groupe alkyle en $C_1$ ou $C_2$, F, Cl, Br, $NO_2$, alcoxy en $C_1$ ou $C_2$, alkylthio en $C_1$ ou $C_2$, halogénalcoxy en $C_1$ ou $C_2$, $CH_2OCH_3$, $CH_2SCH_3$ ou $CH_2CN$ et n'est pas en position para par rapport au pont de sulfonylurée ;
X est $CH_3$, $OCH_3$ , $OCH_2CH_3$, Cl, F, Br, I, $CH_2F$, $OCH_2CH_2F$, $OCH_2CHF_2$, $OCH_2CF_3$, $CF_3$, $CH_2Cl$ ou $CH_2Br$ ; et
Y est H, un groupe alkyle en $C_1$ à $C_3$, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C≡CH$, $CH_2OCH_2CH_3$, $OCH_2CH_2OCH_3$, $CH_2SCH_3$, $SCF_2H$, cyclopropyle, $C≡CH$ ou $C≡CCH_3$.

**7.** Un composé selon la revendication 3, dans lequel :
R est H ;
$R_1$ est H, un groupe alkyle en $C_1$ ou $C_2$, F, Cl, Br, $NO_2$, alcoxy en $C_1$ ou $C_2$, alkylthio en $C_1$ ou $C_2$, halogénalcoxy en $C_1$ ou $C_2$, $CH_2OCH_3$, $CH_2SCH_3$ ou $CH_2CN$ et n'est pas en position para par rapport au pont de sulfonylurée ;
x est $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ ou $CH_2Br$ ; et
Y est H, un groupe alkyle en $C_1$ à $C_3$, $OCH_3$, $OC_2H$, $CH_2OCH_3$, $NHCH_3$, $M(OCH_3)CH_3$, $N(CH_3)_2$, $CF_3$, $SCH_3$, $OCH_2CH=CH_2$, $OCH_2C≡CH$, $CH_2OCH_2CH_3$, $OCH_2CH_2OCH_3$, $CH_2SCH_3$, $SCF_2H$, cyclopropyle, $C≡CH$ ou $C≡CCH_3$ .

**8.** Un composé selon la revendication 4, dans lequel :
R est H ;
$R_1$ est H, un groupe alkyle en $C_1$ ou $C_2$, F, Cl, Br, $NO_2$, alcoxy en $C_1$ ou $C_2$, alkylthio en $C_1$ ou $C_2$, halogénalcoxy en $C_1$ ou $C_2$, $CH_2OCH_3$, $CH_2SCH_3$ ou $CH_2CN$ et n'est pas en position para par rapport au pont de sulfonylurée ;
X est $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ ou $CH_2Br$ ; et
Y est H, un groupe alkyle en $C_1$ à $C_3$, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C≡CH$, $CH_2OCH_2CH_3$, $OCH_2CH_2OCH_3$, $CH_2SCH_3$, $SCF_2H$, cyclopropyle, $C≡CH$ ou $C≡CCH_3$.

**9.** Un composé selon la revendication 5, dans lequel :
$R_2'$ , $R_3'$ et R4 sont indépendamment H, un groupe alkyle en $C_1$ à $C_3$ ou phényle, avec la condition que l'un de $R_2'$ , $R_3'$ et $R_4'$ soit H.

**10.** Un composé selon la revendication 6, dans lequel :
$R_1$ est H, Cl, $OCH_3$, $OCF_2H$, $CH_2OCH_3$ ou $CH_2CN$ ;
$R_2$ est un groupe alcynyle en $C_3$ ou $C_4$, cycloalkyle en $C_3$ à $C_6$, halogénalcényle en $C_2$ à $C_1$, $CH_2CH_2F$, $CH_2CH_2Cl$ ou $CH_2CH_2Br$ ;

X est $CH_3$, $OCH_3$, $OCH_2CH_3$, Cl ou $OCH_2CF_3$ ; et
Y est $CH_3$, $OCH_3$, $CH_2CH_3$, $CH_2OCH_3$ ou $NHCH_3$ .

**11.** Un composé selon la revendication 7, dans lequel :
$R_1$ est H, Cl, $OCH_3$, $OCF_2H$, $CH_2OCH_3$ ou $CH_2CN$ ;
$R_2$ est Q ou $CH_2Q$ ;
X est $CH_3$, $OCH_3$, $OCH_2CH_3$, Cl ou $OCH_2CF_3$ ; et
Y est $CH_3$, $OCH_3$, $CH_2CH_3$, $CH_2OCH_3$ ou $NHCH_3$.

**12.** Un composé selon la revendication 8, dans lequel :
$R_1$ est H, Cl, $OCH_3$, $OCF_2H$, $CH_2OCH_3$ ou $CH_2CN$ ;
$R_3$ est un groupe alkyle en $C_1$ à $C_3$, alcényle en $C_3$ ou $C_4$, alcynyle en $C_3$ ou $C_4$, cycloalkyle en $C_3$ à $C_6$ ou halogénalkyle en $C_1$ ou $C_2$ ;
$R_4$ est un groupe halogénalcényle en $C_2$ à $C_4$ ou époxyalkyle en $C_3$ ou $C_4$ ;
X est $CH_3$, $OCH_3$, $OCH_2CH_3$, Cl ou $OCH_2CF_3$ ; et
Y est $CH_3$, $OCH_3$, $CH_2CH_3$, $CH_2OCH_3$ ou $NHCH_3$.

**13.** Un composé selon la revendication 9, dans lequel :
$R_1$ est H, Cl, $OCH_3$, $OCF_2H$, $CH_2OCH_3$ ou $CH_2CN$ ;
X est $CH_3$, $OCH_3$, $OCH_2CH_3$, Cl ou $OCH_2CF_3$ ; et
Y est $CH_3$, $OCH_3$, $CH_2CH_3$, $CH_2OCH_3$ ou $NHCH_3$.

**14.** Un composé selon la revendication 2, le N-cyclopropyl-N'-[(4,6 -diméthoxypyrimidine-2-Yl)-aminocarbonyl]-1,2-benzène-disulfonamide.

**15.** Un composé selon la revendication 2, le N-cyclopropyl-N'-[(4-méthoxy-6-méthyl-1,3,5-triazine-2-yl)-aminocarbonyl]-1,2-benzène-disulfonamide.

**16.** Un composé selon la revendication 5, le 2-(2,2-diméthylhydrazinosulfonyl)-N-[(4,6-diméthoxypyrimidine-2-Yl)aminocarbonyl]benzène-sulfonamide.

**17.** Un composé selon la revendication 5, le 2-(2,2-diméthylhydrazinosulfonyl)-N-[(4-méthoxy-6-méthyl-1,3,5-triazine-2-yl)aminocarbonyl]benzène-sulfonamide.

**18.** Une composition pour lutter contre une végétation indésirable ou à utiliser comme régulateur de la croissance de plantes, comprenant un composé selon l'une quelconque des revendications 1 à 17 et au moins l'un de (i) un agent tensio-actif et (ii) un diluant solide ou liquide.

**19.** Un procédé pour lutter contre une végétation indésirable, consistant à appliquer à l'emplacement où se trouve cette végétation une quantité exerçant une action herbicide d'un composé selon l'une quelconque des revendications 1 à 17.

**20.** Un procédé pour réguler la croissance de plantes, consistant à appliquer à l'emplacement où se trouvent ces plantes une quantité efficace mais sensiblement non phytotoxique d'un régulateur de croissance de plantes choisi parmi les composés de l'une quelconque des revendications 1 à 17.

**21.** Un procédé pour la préparation d'un composé de la revendication 1, qui consiste à :
(a) faire réagir un isocyanate ou isothiocyanate de formule

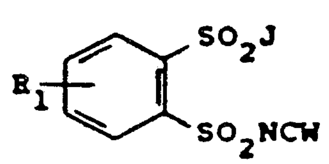

(II)

ou un carbamate de phényle ou thiocarbamate de phényle de formule

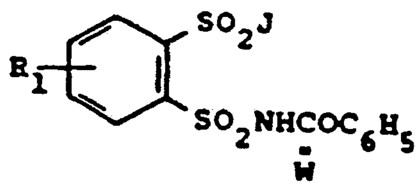

(VI)

avec une amine hétérocyclique de formule

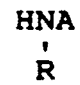

(III) ; ou

(b) faire réagir un sulfonamide de formule

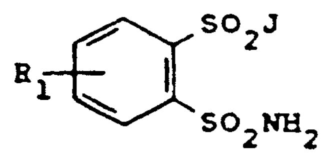

(IV)

avec un carbamate de phényle ou thiocarbamate de phényle hétérocyclique de formule

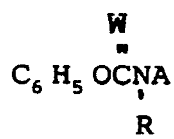

(VII)

où J, R, $R_1$, A et W sont tels que définis dans la revendication 1 ; ou
(c) oxyder un composé de Formule (I) où $R_2$, $R_3$ ou $R_4$ est un groupe alkyle en $C_1$ à $C_6$ substitué par $SR_7$ et $R_1$ est autre chose qu'un groupe alkylthio en $C_1$ à $C_3$, alkylsulfinyle en $C_1$ à $C_3$, halogénalkylthio en $C_1$ à $C_3$, alkylthioalkyle en $C_2$ ou $C_3$ ou halogénalkylthioalkyle en $C_2$ ou $C_3$, pour oxyder ledit substituant $SR_7$ en $SOR_7$ ou $SO_2R_7$ ; ou
(d) transformer un composé de Formule (I) en un autre composé de Formule (I) par des méthodes connues en soi.

**22.** Composés des formules (II), (IV) et (VI) telles que définies dans la revendication 21.

## Ansprüche

**1.** Verbindung der Formel

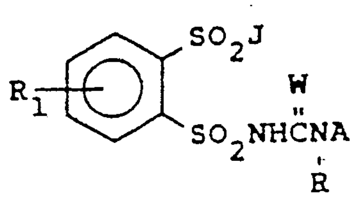

(I)

sowie landwirtschaftlich geeignete Salze davon, dadurch gekennzeichnet, daß
J $NHR_2$, $NR_3R_4$ oder $NR_2'$ $NR_3'$ $R_4'$ ist;
w O oder S ist;
R H oder $CH_3$ ist;
$R_1$ H, $C_1$-$C_3$-Alkyl, $C_1$-$C_3$-Halogenalkyl, Halogen, Nitro, $C_1$-$C_3$-Alkoxy, $SO_2NR_aR_b$, $C_1$-$C_3$-Alkylthio, $C_1$-$C_3$-Alkylsulfinyl, $C_1$-$C_3$-Alkylsulfonyl, CN, $CO_2R_c$, $C_1$-$C_3$-Halogenalkoxy, $C_1$-$C_3$-Halogenalkylthio, $C_2$-$C_3$-

Alkoxyalkyl, $C_2$-$C_3$-Halogenalkoxyalkyl, $C_2$-$C_3$-Alkylthioalkyl, $C_2$-$C_3$-Halogenalkylthioalkyl, $C_2$-$C_3$-Cyanoalkyl oder $NR_dR_e$ ist;
$R_a$ H, $C_1$-$C_4$-Alkyl, $C_2$-$C_3$-Cyanoalkyl, Methoxy oder Ethoxy ist; $R_b$ H, $C_1$-$C_4$-Alkyl oder $C_3$-$C_4$-Alkenyl ist; oder
$R_a$ und $R_b$ als $-(CH_2)_3-$, $-(CH_2)_4-$, $-(CH_2)_5-$ oder $-CH_2CH_2OCH_2CH_2-$ zusammengenommen werden können;
$R_c$ $C_1$-$C_4$-Alkyl, $C_3$-$C_4$-Alkenyl, $C_3$-$C_4$-Alkinyl, $C_2$-$C_4$-Halogenalkyl, $C_2$-$C_3$-Cyanalkyl, $C_5$-$C_6$-Cycloalkyl, $C_4$-$C_7$-Cycloalkylalkyl oder $C_2$-$C_4$-Alkoxyalkyl ist;
$R_d$ und $R_e$ unabhängig H oder $C_1$-$C_2$-Alkyl sind;
$R_2$ $C_5$-$C_6$-Alkyl, $C_5$-$C_6$-Alkenyl, $C_2$-$C_6$-Halogenalkenyl, $C_3$-$C_6$-Alkinyl, $C_3$-$C_6$-Cycloalkyl, gegebenenfalls mit 1 oder 2 $CH_3$-Gruppen substituiertes $C_3$-$C_6$-Cycloalkyl, $C_4$-$C_7$-Cycloalkylalkyl, $C_5$-$C_6$-Cycloalkenyl, $C_3$-$C_6$-Epoxyalkyl, $C_2$-$C_6$-Halogenalkyl, $CH_2CH_2(OR_5)_2$,

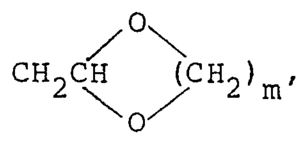

$(CH_2)_3OCH_3$, Phenyl, das gegebenenfalls mit $R_7$ substituiert sein kann,

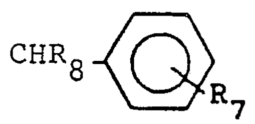 ,

$CH_2C(O)CH_3$, CN, $OR_6$, mit $OR_9$, $S(O)_nR_{10}$ oder $NR_{11}R_{12}$ substituiertes $C_1$-$C_6$-Alkyl, Q, $CHR_8Q$ oder $CH_2CH_2Q$ ist;
$R_3$ $C_1$-$C_6$-Alkyl, $C_3$-$C_6$-Alkenyl, $C_3$-$C_6$-Alkinyl, $C_3$-$C_6$-Cycloalkyl, das gegebenenfalls mit 1 oder 2 $CH_3$-Gruppen substituiert sein kann, $C_4$-$C_7$-Cycloalkylalkyl, $C_5$-$C_6$-Cycloalkenyl, $C_1$-$C_6$-Halogenalkyl, $C_1$-$C_5$-Alkoxy, $C_3$-$C_6$-Epoxyalkyl oder mit $OR_9$, $S(O)_nR_{10}$, $NR_{11}R_{12}$ oder $P(O)(OR_5)_2$ substituiertes $C_1$-$C_6$-Alkyl ist;
$R_4$ mit 1 bis 3 F-, Cl- oder Br-Atomen substituiertes $C_1$-$C_3$-Alkyl, $C_3$-$C_4$-Alkinyl, $CH_2CH_2(OR_5)_2$,

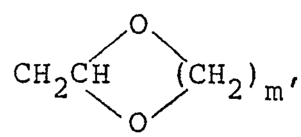

$C_2$-$C_6$-Halogenalkyl, $C_3$-$C_6$-Epoxyalkyl, $CH_2C(O)CH_3$, CN, mit $OR_9$, $S(O)_nR_{10}$ oder $NR_{11}R_{12}$ substituiertes $C_1$-$C_6$-Alkyl, Q, $CHR_8Q$ oder $CH_2CH_2Q$ ist;
$R_3$ und $R_4$ mit dem Sulfonamidstickstoff unter Bildung eines gesättigten 5- oder 6-gliedrigen Ringes, der durch eine oder mehrere aus L ausgewählte Gruppen substituiert ist, oder eines teilweise gesättigten 5- oder 6-gliedrigen Ringes, der gegebenenfalls durch eine oder mehrere aus L ausgewählte Gruppen substituiert ist, zusammengenommen werden können;
$R_5$ $C_1$-$C_3$-Alkyl ist;
$R_6$ $C_1$-$C_5$-Alkyl ist;
$R_7$ H, $C_1$-$C_3$-Alkyl, Halogen, $NO_2$, $CF_3$, CN oder $C_1$-$C_3$-Alkoxy ist;
$R_8$ H oder $CH_3$ ist;
$R_9$ H, $SO_2R_5$, $C(O)R_5$, $CO_2R_5$, $C(O)NR_{11}R_{12}$ oder $P(O)(OR_5)_2$ ist;
$R_{10}$ $C_1$-$C_3$-Alkyl ist;
$R_{11}$ H oder $C_1$-$C_3$-Alkyl ist;
$R_{12}$ H oder $C_1$-$C_3$-Alkyl ist;
m 2 oder 3 ist;
n 0, 1 oder 2 ist;
$R_2'$ $R_3'$ und $R_4'$ unabhängig H, $C_1$-$C_4$-Alkyl, $C_3$-$C_4$-Alkenyl, $C_3$-$C_4$-Alkinyl, $C_1$-$C_4$-Halogenalkyl, $C(O)R_5'$, $CO_2R_6'$, $C(O)NR_7'R_8'$, $C(S)NR_7'R_8'$, Q, CHRQ, $CH_2CH_2Q$, mit $OR_9'$ substituiertes $C_2$-$C_3$-Alkyl, Phenyl, das gegebenenfalls mit $R_{10}'$ und $R_{11}'$ substituiert sein kann, oder

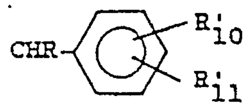 sind;

$R'_3$ und $R'_4$ unter Bildung von $-(CH_2)_4-$, $-(CH_2)_5-$, $CH_2CH_2OCH_2CH_2$, $CH=CHCH=CH$, $CH=N-N=CH$ oder

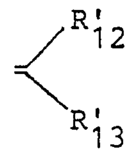 zu-

sammengenommen werden können;
$R'_5$ $C_1$-$C_3$-Alkyl oder Phenyl, das gegebenenfalls mit $R'_{10}$ und $R'_{11}$ substituiert sein kann, ist;
$R'_6$ $C_1$-$C_3$-Alkyl ist;
$R'_7$ und $R'_8$ unabhängig H oder $C_1$-$C_3$-Alkyl sind;
$R'_9$ H, $SO_2R'_6$, $C(O)R'_6$, $CO_2R'_6$, $C(O)NR'_7R'_8$, $C_1$-$C_3$-Alkyl, $C_1$-$C_3$-Halogenalkyl oder $P(O)(OR'_6)_2$ ist;
$R'_{10}$ und $R'_{11}$ unabhängig H, $C_1$-$C_3$-Alkyl, Cl, F, Br, $NO_2$, $CF_3$, CN oder $C_1$-$C_3$-Alkoxy sind;
$R'_{12}$ und $R'_{13}$ unabhängig H, $C_1$-$C_3$-Alkyl, Phenyl, das gegebenenfalls mit $R'_{10}$ und $R'_{11}$ substituiert sein kann,

oder 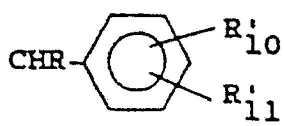 sind;

$R'_{12}$ und $R'_{13}$ unter Bildung von $-(CH_2)_4-$ oder $-(CH_2)_5-$zusammengenommen werden können;
Q ein gesättigter 5- oder 6-gliedriger Ring, der durch ein Kohlenstoffatom gebunden ist und ein aus Sauerstoff, Schwefel oder Stickstoff ausgewähltes Heteroatom enthält, oder ein ungesättigter oder teilweise ungesättigter 5- oder 6-gliedriger Ring ist, der über ein Kohlenstoffatom gebunden ist und 1 bis 3 aus 1 Schwefel, 1 Sauerstoff oder 1 bis 3 Stickstoff ausgewählte Heteroatome enthält, wobei Q gegebenenfalls durch eine oder mehrere aus L ausgewählte Gruppen substituiert sein kann;
L $C_1$-$C_4$-Alkyl, $C_1$-$C_3$-Halogenalkyl, Halogen, $C_1$-$C_3$-Alkoxy, $C_1$-$C_3$-Alkylthio, $C_3$-$C_4$-Alkenyloxy, $C_3$-$C_4$-Alkenylthio, $C_1$-$C_2$-Halogenalkoxy oder $C_1$-$C_2$-Halogenalkylthio ist;
A

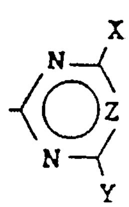 ist;

X H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino oder Di($C_1$-$C_3$-alkyl)-amino ist;
Y H, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$C_4$-Alkinyloxy, $C_2$-$C_5$-Alkylthioalkyl, $C_1$-$C_4$-Halogenalkyl, $C_3$-$C_5$-Cycloalkyl, $C_2$-$C_4$-Alkinyl oder $N(OCH_3)CH_3$ ist;
Z CH oder N ist;
und weiterhin dadurch gekennzeichnet, daß

a) wenn x Cl, F, Br oder I ist, dann Z CH ist und Y $OCH_3$, $OC_2H_5$, $N(OCH_3)CH_3$, $NHCH_3$ oder $N(CH_3)_2$ ist;
b) die Gesamtzahl der Kohlenstoffatome in $R'_2$, $R'_3$ und $R'_4$ 10 nicht übersteigt;
c) $R_2$ nicht $CH_2CF_3$ ist;
d) wenn $R_3$ oder $R_4$ $CH_2CF_3$ ist, dann das andere $CHF_2$, $CH_2CH_2F$, $CH_2CH_2Cl$, $CH_2CH_2Br$ oder

$CH_2CF_3$ ist;
e) wenn $R_4$ $CF_2H$ ist, dann $R_3$ von $C_1$ -$C_3$-Alkyl verschieden ist;
f) wenn $R_4$ $C_3$-$C_4$-Alkinyl ist, dann $R_3$ $CHF_2$, $CH_2CH_2F$, $CH_2CH_2Cl$, $CH_2CH_2Br$ oder $C_3$-$C_4$-Alkinyl ist;
g) wenn $R_2$ $OR_6$ ist und Z CH ist, dann eines oder beide aus X und Y von $C_1$-$C_4$-Alkyl verschieden sind; und
h) keines von X und Y Halogenmethoxy ist.

**2.** Verbindung nach Anspruch 1, worin
W O ist;
J $NHR_2$ ist; und
$R_2$ $C_5$-$C_6$-Alkyl, $C_5$-$C_6$-Alkenyl, $C_2$-$C_6$-Halogenalkenyl, $C_3$-$C_6$-Alkinyl, $C_3$-$C_6$-Cycloalkyl, gegebenenfalls mit 1 oder 2 $CH_3$-Gruppen substituiertes $C_3$-$C_6$-Cyclo-alkyl, $C_4$-$C_7$-Cycloalkylalkyl, $C_5$-$C_6$-Cycloalkenyl, $C_3$-$C_6$-Epoxyalkyl, $C_2$-$C_6$-Halogenalkyl, $CH_2CH_2(OR_5)_2$, $(CH_2)_3OCH_3$, $OR_6$, Phenyl, das gegebenenfalls mit $R_7$ substituiert sein kann,

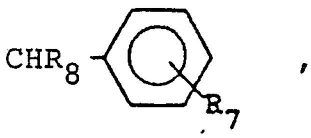

$CH_2C(O)CH_3$, CN oder mit $OR_9$, $S(O)_nR_{10}$ oder $NR_{11}R_{12}$ substituiertes $C_1$-$C_6$-Alkyl ist.

**3.** Verbindung nach Anspruch 1, worin
W O ist;
J $NHR_2$ ist; und
$R_2$ Q, $CHR_8Q$ oder $CH_2CH_2Q$ ist.

**4.** Verbindung nach Anspruch 1, worin
W O ist; und
J $NR_3R_4$ ist.

**5.** Verbindung nach Anspruch 1, worin
W O ist;
J $NR_2'$ $NR_3'$ $R_4'$ ist;
R H ist;
$R_1$ H, $C_1$-$C_2$-Alkyl, F, Cl, Br, $NO_2$, $C_1$-$C_2$-Alkoxy, $C_1$-$C_2$-Alkylthio, $C_1$-$C_2$-Halogenalkoxy, $CH_2OCH_3$, $CH_2SCH_3$ oder $CH_2CN$ ist und nicht para zur Sulfonylharnstoffbrücke steht;
X $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ oder $CH_2Br$ ist;
Y H, $C_1$-$C_3$-Alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C≡CH$, $CH_2OCH_2CH_3$, $OCH_2CH_2OCH_3$, $CH_2SCH_3$, $SCF_2H$, Cyclopropyl, C≡CH oder $C≡CCH_3$ ist.

**6.** Verbindung nach Anspruch 2, worin
R H ist;
$R_1$ H, $C_1$-$C_2$-Alkyl, F, Cl, Br, $NO_2$, $C_1$-$C_2$-Alkoxy, $C_1$-$C_2$-Alkylthio, $C_1$-$C_2$-Halogenalkoxy, $CH_2OCH_3$, $CH_2SCH_3$ oder $CH_2CN$ ist und nicht para zur Sulfonylharnstoffbrücke steht;
X $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ oder $CH_2Br$ ist; und
Y H, $C_1$-$C_3$-Alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2c≡CH$, $CH_2OCH_2CH_3$, $OCH_2CH_2OCH_3$, $CH_2SCH_3$, $SCF_2H$, Cyclopropyl, C≡CH oder $C≡CCH_3$ ist.

**7.** Verbindung nach Anspruch 3, worin
R H ist;
$R_1$ H, $C_1$-$C_2$-Alkyl, F, Cl, Br, $NO_2$, $C_1$-$C_2$-Alkoxy, $C_1$-$C_2$-Alkylthio, $C_1$-$C_2$-Halogenalkoxy, $CH_2OCH_3$, $CH_2SCH_3$ oder $CH_2CN$ ist und nicht para zur Sulfonylharnstoffbrücke steht;

X $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ oder $CH_2Br$ ist; und
Y H, $C_1$-$C_3$-Alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C=CH$, $CH_2OCH_2CH_3$, $OCH_2CH_2OCH_3$, $CH_2SCH_3$, $SCF_2H$, Cyclopropyl, C≡CH oder $C≡CCH_3$ ist.

**8.** Verbindung nach Anspruch 4, worin
R H ist;
$R_1$ H, $C_1$-$C_2$-Alkyl, F, Cl, Br, $NO_2$, $C_1$-$C_2$-Alkoxy, $C_1$-$C_2$-Alkylthio, $C_1$-$C_2$-Halogenalkoxy, $CH_2OCH_3$, $CH_2SCH_3$ oder $CH_2CN$ ist und nicht para zur Sulfonylharnstoffbrücke steht;
X $CH_3$, $OCH_3$, $OCH_2CH_3$, Cl, F, Br, I, $CH_2F$, $OCH_2CH_2F$, $OCH_2CHF_2$, $OCH_2CF_3$, $CF_3$, $CH_2Cl$ oder $CH_2Br$ ist; und
Y H, $C_1$-$C_3$-Alkyl, $OCH_3$, $OC_2H_5$, $CH_2OCH_3$, $NHCH_3$, $N(OCH_3)CH_3$, $N(CH_3)_2$, $CF_3$, $SCH_3$, $OCH_2CH=CH_2$, $OCH_2C=CH$, $CH_2OCH_2CH_3$, $OCH_2CH_2OCH_3$, $CH_2SCH_3$, $SCF_2H$, Cyclopropyl, C≡CH oder $C≡CCH_3$ ist.

**9.** Verbindung nach Anspruch 5, worin
$R_2'$, $R_3'$ und $R_4'$ unabhängig H, $C_1$-$C_3$-Alkyl oder Phenyl sind, mit der Maßgabe, daß eines aus $R_2'$, $R_3'$ und $R_4'$ H ist.

**10.** Verbindung nach Anspruch 6, worin
$R_1$ H, Cl, $OCH_3$, $OCF_2H$, $CH_2OCH_3$ oder $CH_2CN$ ist;
$R_2$ $C_3$-$C_4$-Alkinyl, $C_3$-$C_6$-Cycloalkyl, $C_2$-$C_4$-Halogenalkenyl, $CH_2CH_2F$, $CH_2CH_2Cl$ oder $CH_2CH_2Br$ ist;
X $CH_3$, $OCH_3$, $OCH_2CH_3$, Cl oder $OCH_2CF_3$ ist; und
Y $CH_3$, $OCH_3$, $CH_2CH_3$, $CH_2OCH_3$ oder $NHCH_3$ ist.

**11.** Verbindung nach Anspruch 7, worin
$R_1$ H, Cl, $OCH_3$, $OCF_2H$, $CH_2OCH_3$ oder $CH_2CN$ ist;
$R_2$ Q oder $CH_2Q$ ist;
X $CH_3$, $OCH_3$, $OCH_2CH_3$, Cl oder $OCH_2CF_3$ ist; und
Y $CH_3$, $OCH_3$, $CH_2CH_3$, $CH_2OCH_3$ oder $NHCH_3$ ist.

**12.** Verbindung nach Anspruch 8, worin
$R_1$ H, Cl, $OCH_3$, $OCF_2H$, $CH_2OCH_3$ oder $CH_2CN$ ist;
$R_3$ $C_1$-$C_3$-Alkyl, $C_3$-$C_4$-Alkenyl, $C_3$-$C_4$-Alkinyl, $C_3$-$C_6$-Cycloalkyl oder $C_1$-$C_2$-Halogenalkyl ist;
$R_4$ $C_2$-$C_4$-Halogenalkenyl oder $C_3$-$C_4$-Epoxyalkyl ist;
X $CH_3$, $OCH_3$, $OCH_2CH_3$, Cl oder $OCH_2CF_3$ ist; und
Y $CH_3$, $OCH_3$, $CH_2CH_3$, $CH_2OCH_3$ oder $NHCH_3$ ist.

**13.** Verbindung nach Anspruch 9, worin
$R_1$ H, Cl, $OCH_3$, $OCF_2H$, $CH_2OCH_3$ oder $CH_2CN$ ist;
X $CH_3$, $OCH_3$, $OCH_2CH_3$, Cl oder $OCH_2CF_3$ ist; und
Y $CH_3$, $OCH_3$, $CH_2CH_3$, $CH_2OCH_3$ oder $NHCH_3$ ist.

**14.** Verbindung nach Anspruch 2, N-Cyclopropyl-N'-[(4,6-dimethoxypyrimidin-2-yl)aminocarbonyl]-1,2-benzoldisulfonamid.

**15.** Verbindung nach Anspruch 2, N-Cyclopropyl-N'-[4-methoxy-6-methyl-1,3,5-triazin-2-yl)aminocarbonyl]-1,2-benzoldisulfonamid.

**16.** Verbindung nach Anspruch 5, 2-(2,2-Dimethylhydrazinsulfonyl)-N-[(4,6-dimethoxypyrimidin-2-yl)-aminocarbonyl]benzolsulfonamid.

**17.** Verbindung nach Anspruch 5, 2-(2,2-Dimethylhydrazinsulfonyl)-N-[(4-methoxy-6-methyl-1,3,5-triazin-2-yl)aminocarbonyl]benzolsulfonamid.

**18.** Zusammensetzung zur Bekämpfung unerwünschter Vegetation oder zur Verwendung als Pflanzenwachstumsregulans, welche eine Verbindung nach einem der Ansprüche 1 bis 17 und wenigstens eines

aus (i) einem oberflächenaktiven Mittel und (ii) einem festen oder flüssigen Verdünnungsmittel umfaßt.

**19.** Verfahren zur Bekämpfung unerwünschter Vegetation durch Anwenden einer herbizid wirksamen Menge einer Verbindung nach einem der Ansprüche 1 bis 17 auf den Ort solcher Vegetation.

**20.** Verfahren zur Steuerung des Wachstums von Pflanzen durch Anwenden einer wirksamen, jedoch im wesentlichen nicht phytotoxischen Menge eines Pflanzenwachstumsregulans, ausgewählt aus Verbindungen nach einem der Ansprüche 1 bis 17, auf den Ort solcher Pflanzen.

**21.** Verfahren zur Herstellung einer Verbindung nach Anspruch 1 durch
(a) Umsetzung eines Isocyanats oder Isothiocyanats der Formel

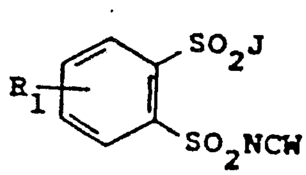

(II)

oder eines Phenylcarbamats oder Phenylthiocarbamats der Formel

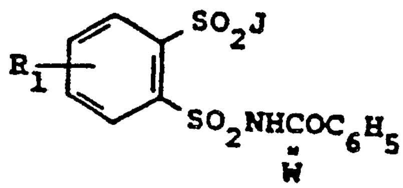

(VI)

mit einem heterocyclischen Amin der Formel

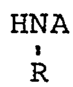

(III); oder

(b) Umsetzung eines Sulfonamids der Formel

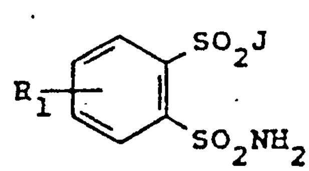

(IV)

mit einem heterocyclischen Phenylcarbamat oder Phenylthiocarbamat der Formel

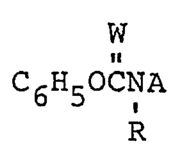

(VII)

worin J, R, $R_1$, A und W wie in Anspruch 1 definiert sind; oder
(c) Oxidieren einer Verbindung der Formel (I), worin $R_2$, $R_3$ oder $R_4$ mit $SR_7$ substituiertes $C_1$-$C_6$-Alkyl ist und $R_1$ von $C_1$-$C_3$-Alkylthio, $C_1$-$C_3$-Alkylsulfinyl, $C_1$-$C_3$-Halogenalkylthio, $C_2$-$C_3$-Alkylthioalkyl oder $C_2$-$C_3$-Halogenalkylthioalkyl verschieden ist, unter Oxidation des Substituenten $SR_7$ zu $SOR_7$ oder $SO_2R_7$; oder

(d) Umwandeln einer Verbindung der Formel (I) in eine andere Verbindung der Formel (I) nach per se bekannten Verfahren.

**22.** Verbindungen der Formeln (II), (IV) und (VI), wie in Anspruch 21 definiert.